(19) Europäisches Patentamt
European Patent Office
Office européen des brevets

(11) EP 2 220 940 A2

(12) **EUROPÄISCHE PATENTANMELDUNG**

(43) Veröffentlichungstag:
**25.08.2010 Patentblatt 2010/34**

(51) Int Cl.:
***A01P 3/00*** *(2006.01)* ***A01N 43/56*** *(2006.01)*
***A01N 43/84*** *(2006.01)*

(21) Anmeldenummer: **10163321.2**

(22) Anmeldetag: **28.09.2004**

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**LT LV**

(30) Priorität: **10.10.2003 DE 10347090**

(62) Dokumentnummer(n) der früheren Anmeldung(en) nach Art. 76 EPÜ:
**04765648.3 / 1 675 461**

(71) Anmelder: **Bayer CropScience AG**
**40789 Monheim (DE)**

(72) Erfinder: **Die Erfindernennung liegt noch nicht vor**

Bemerkungen:
Diese Anmeldung ist am 19-05-2010 als Teilanmeldung zu der unter INID-Code 62 erwähnten Anmeldung eingereicht worden.

(54) **Synergistische fungizide Wirkstoffkombinationen**

(57) Die vorliegende Erfindung betrifft neue Wirkstoffkombinationen, die aus bekannten Carboxamiden einerseits und Fenpropimoiph andererseits bestehen und sehr gut zur Bekämpfung von unerwünschten phytopathogenen Pilzen geeignet sind.



Printed by Jouve, 75001 PARIS (FR)

## Beschreibung

Die vorliegende Erfindung betrifft neue Wirkstoffkombinationen, die aus bekannten Carboxamiden einerseits und Fenpropimorph andererseits bestehen und sehr gut zur Bekämpfung von unerwünschten phytopathogenen Pilzen geeignet sind.

Es ist bereits bekannt, dass bestimmte Carboxamide fungizide Eigenschaften besitzen. So sind z.B. *N*-(3',4'-Dichlor-5-fluor-1,1'-biphenyl-2-yl)-3-(difluormethyl)-1-methyl-1H-pyrazol-4-carboxamid aus DE-A 102 15 292, 3-(Trifluormethyl)-*N*-{3'-fluor-4'-[(*E*)-(methoxyimino)methyl]-1,1'-biphenyl-2-yl}-1-methyl-1*H*-pyrazol-4-carboxamid aus WO 02/08197, sowie *N*-(3',4'-Dichlor-1,1'-biphenyl-2-yl)-5-fluor-1,3-dimethyl-1*H*-pyrazol-4-carboxamid aus WO 00/14701 bekannt. Die Wirksamkeit dieser Stoffe ist gut, lässt aber bei niedrigen Aufwandmengen in manchen Fällen zu wünschen übrig. Ferner ist schon bekannt, dass zahlreiche Triazol-Derivate, Anilin-Derivate, Dicarboximide und andere Heterocyclen zur Bekämpfung von Pilzen eingesetzt werden können (vgl. EP-A 0 040 345, DE-A 22 01 063, DE-A 23 24 010, Pesticide Manual, 9th. Edition (1991), Seiten 249 und 827, EP-A 0 382 375 und EP-A 0 515 901). Auch die Wirkung dieser Stoffe ist aber bei niedrigen Aufwandmengen nicht immer ausreichend. Ferner ist bereits bekannt, dass 1-(3,5-Dimethyl-isoxazol-4-sulfonyl)-2-chlor-6,6-difluor-[1,3]-dioxolo-[4,5f]-benzimidazol fungizide Eigenschaften besitzt (vgl. WO 97/06171). Schließlich ist auch bekannt, dass substituierte Halogenpyrimidine fungizide Eigenschaften besitzen (vgl. DE-A1-196 46 407, EP-B-712 396).

Es wurden nun neue Wirkstoffkombinationen mit sehr guten fungiziden Eigenschaften gefunden, enthaltend ein Carboxamid der allgemeinen Formel (I) (Gruppe 1)

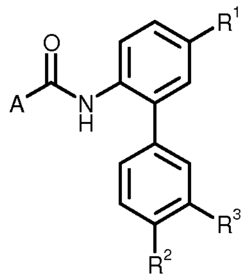

(I)

in welcher

$R^1$ für Wasserstoff oder Fluor steht,

$R^2$ für Halogen, $C_1$-$C_3$-Alkyl, $C_1$-$C_3$-Halogenalkyl mit 1 bis 7 Fluor-, Chlor- und/ oder Bromato- men, $C_1$-$C_3$-Alkoxy, $C_1$-$C_3$-Halogenalkoxy mit 1 bis 7 Fluor-, Chlor- und/ oder Bromatomen oder für -C($R^4$)=N-O$R^5$ steht,

$R^3$ für Wasserstoff, Halogen, $C_1$-$C_3$-Alkyl oder $C_1$-$C_3$-Halogenalkyl mit 1 bis 7 Fluor-, Chlor- und/oder Bromatomen steht,

$R^4$ für Wasserstoff oder Methyl steht,

$R^5$ für $C_1$-$C_5$-Alkyl, $C_1$-$C_5$-Alkenyl oder $C_1$-$C_5$-Alkinyl steht,

A für einen der folgenden Reste A1 bis A7 steht:

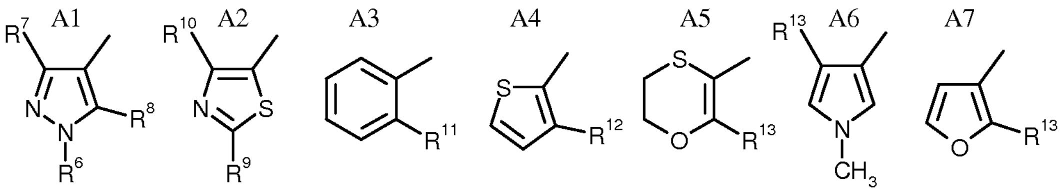

A1 A2 A3 A4 A5 A6 A7

$R^6$ für $C_1$-$C_3$-Alkyl steht,

$R^7$ für Wasserstoff, Halogen, $C_1$-$C_3$-Alkyl oder $C_1$-$C_3$-Halogenalkyl mit 1 bis 7 Fluor-, Chlor- und/oder Bromatomen steht,

$R^8$ für Wasserstoff, Halogen oder $C_1$-$C_3$-Alkyl steht,

$R^9$ für Wasserstoff, Halogen, $C_1$-$C_3$-Alkyl, Amino, Mono- oder Di($C_1$-$C_3$-alkyl)amino steht,

$R^{10}$ für Wasserstoff, Halogen, $C_1$-$C_3$-Alkyl oder $C_1$-$C_3$-Halogenalkyl mit 1 bis 7 Fluor-, Chlor- und/oder Bromatomen steht,

$R^{11}$ für Halogen, $C_1$-$C_3$-Alkyl oder $C_1$-$C_3$-Halogenalkyl mit 1 bis 7 Fluor-, Chlor- und/oder Bromatomen steht,

$R^{12}$ für Halogen, $C_1$-$C_3$-Alkyl oder $C_1$-$C_3$-Halogenalkyl mit 1 bis 7 Fluor-, Chlor- und/oder Bromatomen steht,

$R^{13}$ für Wasserstoff, Halogen, $C_1$-$C_3$-Alkyl oder $C_1$-$C_3$-Halogenalkyl mit 1 bis 7 Fluor-, Chlor- und/oder Bromatomen steht,

und mindestens einen Wirkstoff, der aus den folgenden Gruppen (2) bis (23) ausgewählt ist:

Gruppe (2) Strobilurine der allgemeinen Formel (II)

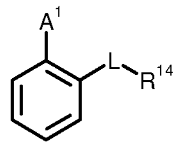 (II)

in welcher

$A^1$ für eine der Gruppen

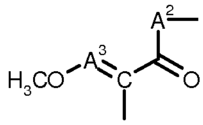
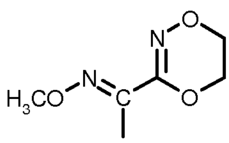
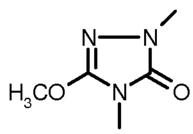
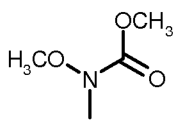

steht,

$A^2$ für NH oder O steht,

$A^3$ für N oder CH steht,

L für eine der Gruppen

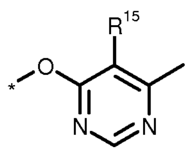
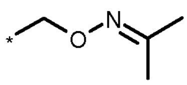
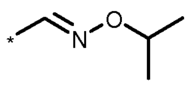
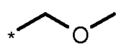
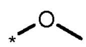

steht, wobei die Bindung, die mit einem Stern (*) markiert ist an den Phenylring gebunden ist,

$R^{14}$ für jeweils gegebenenfalls einfach oder zweifach, gleich oder verschieden durch Chlor, Cyano, Methyl oder Trifluormethyl substituiertes Phenyl, Phenoxy oder Pyridinyl, oder für 1- (4-Chlorphenyl)-pyrazol-3-yl oder für 1,2-Propandion-bis(O-methyloxim)-1-yl steht,

$R^{15}$ für Wasserstoff oder Fluor steht;

Gruppe (3) Triazole der allgemeinen Formel (III)

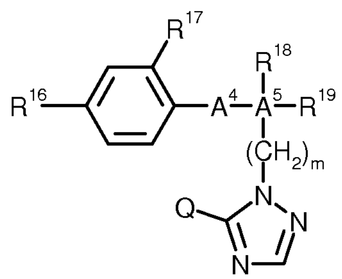

(III)

in welcher

Q für Wasserstoff oder SH steht,

m für 0 oder 1 steht,

$R^{16}$ für Wasserstoff, Fluor, Chlor, Phenyl oder 4-Chlor-phenoxy steht,

$R^{17}$ für Wasserstoff oder Chlor steht,

$A^4$ für eine direkte Bindung, $-CH_2-$, $-(CH_2)_2-$ oder -O- steht,

$A^4$ außerdem für $*-CH_2-CHR^{20}-$ oder $*-CH=CR^{20}-$ steht, wobei die mit * markierte Bindung mit dem Phenylring verknüpft ist, und $R^{18}$ und $R^{20}$ dann zusammen für $-CH_2-CH_2-CH[CH(CH_3)_2]-$ oder $-CH_2-CH_2-C(CH_3)_2-$ stehen,

$A^5$ für C oder Si (Silizium) steht,

$A^4$ außerdem für $-N(R^{20})-$ steht und $A^5$ außerdem zusammen mit $R^{18}$ und $R^{19}$ für die Gruppe $C=N-R^{21}$ steht, wobei $R^{20}$ und $R^{21}$ dann zusammen für die Gruppe

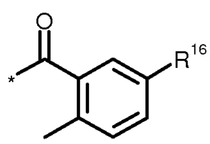

stehen, wobei die mit * markierte Bindung mit $R^{20}$ verbunden ist,

$R^{18}$ für Wasserstoff, Hydroxy oder Cyano steht,

$R^{19}$ für 1-Cyclopropylethyl, 1-Chlorcyclopropyl, $C_1$-$C_4$-Alkyl, $C_1$-$C_6$-Hydroxyalkyl, $C_1$-$C_4$-Alkyl- carbonyl, $C_1$-$C_2$-Halogenalkoxy-$C_1$-$C_2$-alkyl, Trimethylsilyl-$C_1$-$C_2$-alkyl, Monofluorphenyl, oder Phenyl steht,

$R^{18}$ und $R^{19}$ außerdem zusammen für $-O-CH_2-CH(R^{21})-O-$, $-O-CH_2-CH(R^{21})-CH_2-$, oder -O-CH(2- Chlorphenyl)- ste-

hen,

$R^{21}$ für Wasserstoff, $C_1$-$C_4$-Alkyl oder Brom steht;

Gruppe (4) Sulfenamide der allgemeinen Formel (IV)

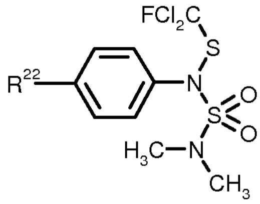

(IV)

in welcher $R^{22}$ für Wasserstoff oder Methyl steht;

Gruppe (5) Valinamide ausgewählt aus

(5-1) Iprovalicarb
(5-2) *N*[1]-[2-(4-{[3-(4-chlorophenyl)-2-propynyl]oxy}-3-methoxyphenyl)ethyl]-*N*[2]-(methylsulfonyl)-D-valinamid;
(5-3) Benthiavalicarb

Gruppe (6) Carboxamide der allgemeinen Formel (V)

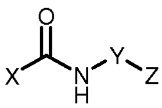

(V)

in welcher

X für 2-Chlor-3-pyridinyl, für 1-Methylpyrazol-4-yl, welches in 3-Position durch Methyl oder Trifluormethyl und in 5-Position durch Wasserstoff oder Chlor substituiert ist, für 4-Ethyl-2- ethylamino-1,3-thiazol-5-yl, für 1-Methyl-cyclohexyl, für 2,2-Dichlor-1-ethyl-3-methyl- cyclopropyl, für 2-Fluor-2-propyl, oder für Phenyl steht, welches einfach bis dreifach, gleich oder verschieden durch Chlor oder Methyl substituiert ist, steht,

X außerdem für 3,4-Dichlor-isothiazol-5-yl, 5,6-Dihydro-2-methyl-1,4-oxathiin-3-yl, 4-Methyl- 1,2,3-thiadiazol-5-yl, 4,5-Dimethyl-2-trimethylsilyl-thiophen-3-yl, 1-Methylpyrrol-3-yl, wel- ches in 4-Position durch Methyl oder Trifluormethyl und in 5-Position durch Wasserstoff oder Chlor substituiert ist, steht,

Y für eine direkte Bindung, gegebenenfalls durch Chlor, Cyano oder Oxo substituiertes $C_1$-$C_6$- Alkandiyl (Alkylen) oder Thiophendiyl steht,

Y außerdem für $C_2$-$C_6$-Alkendiyl (Alkenylen) steht,

Z für Wasserstoff oder die Gruppe

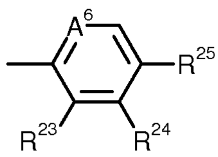

steht,

Z außerdem für $C_1$-$C_6$-Alkyl steht,

$A^6$ für CH oder N steht,

$R^{23}$ für Wasserstoff, Chlor, durch gegebenenfalls einfach oder zweifach, gleich oder verschieden durch Chlor oder Di($C_1$-$C_3$-alkyl)aminocarbonyl substituiertes Phenyl steht,

$R^{23}$ außerdem für Cyano oder $C_1$-$C_6$-Alkyl steht,

$R^{24}$ für Wasserstoff oder Chlor steht,

$R^{25}$ für Wasserstoff, Chlor, Hydroxy, Methyl oder Trifluormethyl steht,

$R^{25}$ außerdem für Di($C_1$-$C_3$-alkyl)aminocarbonyl steht,

$R^{23}$ und $R^{24}$ außerdem gemeinsam für *-$CH(CH_3)$-$CH_2$-$C(CH_3)_2$- oder *-$CH(CH_3)$-O-$C(CH_3)_2$- steht, wobei die mit * markierte Bindung mit $R^{23}$ verknüpft ist;

Gruppe (7) Dithiocarbamate ausgewählt aus

(7-1) Mancozeb

(7-2) Maneb

(7-3) Metiram

(7-4) Propineb

(7-5) Thiram

(7-6) Zineb

(7-7) Ziram

Gruppe (8) Acylalanine der allgemeinen Formel (VI)

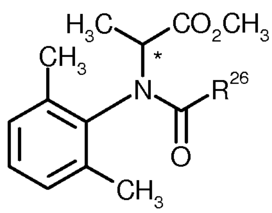

(VI)

in welcher

| | |
|---|---|
| * | ein Kohlenstoffatom in der R- oder der S-Konfiguration, bevorzugt in der S-Konfiguration, kennzeichnet, |
| $R^{26}$ | für Benzyl, Furyl oder Methoxymethyl steht; |

Gruppe (9): Anilino-pyrinmidine der allgemeinen Formel (VII)

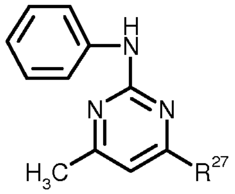

(VII)

in welcher

| | |
|---|---|
| $R^{27}$ | für Methyl, Cyclopropyl oder 1-Propinyl steht; |

Gruppe (10): Benzimidazole der allgemeinen Formel (VIII)

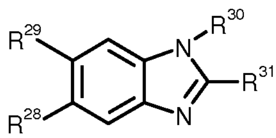

(VIII)

in welcher

| | |
|---|---|
| $R^{28}$ und $R^{29}$ | jeweils für Wasserstoff oder zusammen für $-O-CF_2-O-$ stehen, |
| $R^{30}$ | für Wasserstoff, $C_1$-$C_4$-Alkylaminocarbonyl oder für 3,5-Dimethylisoxazol-4-ylsulfonyl steht, |
| $R^{31}$ | für Chlor, Methoxycarbonylamino, Chlorphenyl, Furyl oder Thiazolyl steht; |

Gruppe (11): Carbamate der allgemeinen Formel (IX)

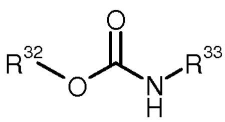

(IX)

in welcher

| | |
|---|---|
| $R^{32}$ | für n- oder iso-Propyl steht, |
| $R^{33}$ | für Di($C_1$-$C_2$-alkyl)amino-$C_2$-$C_4$-alkyl oder Diethoxyphenyl steht, |

wobei auch Salz dieser Verbindungen eingeschlossen sind;

Gruppe (12): Dicarboximide ausgewählt aus

(12-1) Captafol

(12-2) Captan

(12-3) Folpet

(12-4) Iprodione

(12-5) Procymidone

(12-6) Vinclozolin

Gruppe (13): Guanidine ausgewählt aus

(13-1) Dodine

(13-2) Guazatine

(13-3) Iminoctadine triacetate

(13-4) Iminoctadine tris(albesilate)

Gruppe (14): Imidazole ausgewählt aus

(14-1) Cyazofamid

(14-2) Prochloraz

(14-3) Triazoxide

(14-4) Pefurazoate

Gruppe (15): Morpholine der allgemeinen Formel (X)

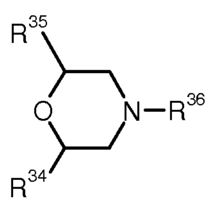

(X)

in welcher

$R^{34}$ und $R^{35}$ unabhängig voneinander für Wasserstoff oder Methyl stehen,

$R^{36}$ für $C_1$-$C_{14}$-Alkyl (bevorzugt $C_{12}$-$C_{14}$-Alkyl), $C_5$-$C_{12}$-Cycloalkyl (bevorzugt $C_{10}$-$C_{12}$-Cyclo- alkyl), Phenyl-$C_1$-$C_4$-alkyl, welches im Phenylteil durch Halogen oder $C_1$-$C_4$-Alkyl substitu- iert sein kann, oder für Acrylyl, welches durch Chlorphenyl und Dimethoxyphenyl substitu- iert ist, steht;

(16): Pyrrole der allgemeinen Formel (XI)

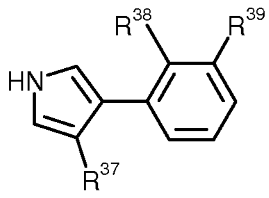

(XI)

in welcher

$R^{37}$ für Chlor oder Cyano steht,

$R^{38}$ für Chlor oder Nitro steht,

$R^{39}$ für Chlor steht

$R^{38}$ und $R^{39}$ außerdem gemeinsam für -O-$CF_2$-O- stehen;

Gruppe (17): Phosphonate ausgewählt aus

(17-1) Fosetyl-Al

(17-2) Phosphonsäure;

Gruppe (18): Phenylethanamide der allgemeinen Formel (XII)

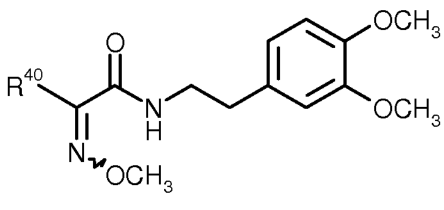

(XII)

in welcher

$R^{40}$ für unsubstituiertes oder durch Fluor, Chlor, Brom, Methyl oder Ethyl substituiertes Phenyl, 2-Naphthyl, 1,2,3,4-Tetrahydronaphthyl oder Indanyl steht;

Gruppe (19): Fungizide ausgewählt aus

(19-1) Acibenzolar-S-methyl

(19-2) Chlorothalonil
(19-3) Cymoxanil
(19-4) Edifenphos
(19-5) Famoxadone
(19-6) Fluazinam
(19-7) Kupferoxychlorid
(19-8) Kupferhydroxid
(19-9) Oxadixyl
(19-10) Spiroxamine
(19-11) Dithianon
(19-12) Metrafenone
(19-13) Fenamidone
(19-14) 2,3-Dibutyl-6-chlor-thieno[2,3-d]pyrimidin-4(3H)on
(19-15) Probenazole
(19-16) Isoprothiolane
(19-17) Kasugamycin
(19-18) Phthalide
(19-19) Ferimzone
(19-20) Tricyclazole
(19-21) N-({4-[(Cyclopropylamino)carbonyl]phenyl}sulfonyl)-2-methoxybenzamid
(19-22) 2-(4-Chlorphenyl)-N-{2-[3-methoxy-4-(prop-2-in-1-yloxy)phenyl]ethyl}-2-(prop-2-in-1-yloxy)acetamid

Gruppe (20): (Thio)Harnstoff-Derivate ausgewählt aus

(20-1) Pencycuron

(20-2) Thiophanate-methyl

(20-3) Thiophanate-ethyl

Gruppe (21): Amide der allgemeinen Formel (XIII)

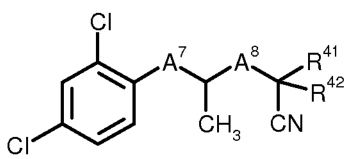

(XIII)

in welcher

$A^7$ für eine direkte Bindung oder -O- steht,

$A^8$ für -C(=O)NH- oder -NHC(=O)- steht,

$R^{41}$ für Wasserstoff oder $C_1$-$C_4$-Alkyl steht,

$R^{42}$ für $C_1$-$C_6$-Alkyl steht;

Gruppe (22): Triazolopyrimidine der allgemeinen Formel (XIV)

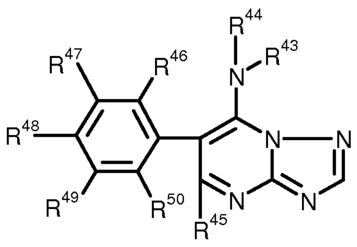

(XIV)

in welcher

$R^{43}$ für $C_1$-$C_6$-Alkyl oder $C_2$-$C_6$-Alkenyl steht,

$R^{44}$ für $C_1$-$C_6$-Alkyl steht,

$R^{43}$ und $R^{44}$ außerdem gemeinsam für $C_4$-$C_5$-Alkandiyl (Alkylen) stehen, welches einfach oder zweifach durch $C_1$-$C_6$-Alkyl substituiert ist,

$R^{45}$ für Brom oder Chlor steht,

$R^{46}$ und $R^{50}$ unabhängig voneinander für Wasserstoff, Fluor, Chlor oder Methyl stehen,

$R^{47}$ und $R^{49}$ unabhängig voneinander für Wasserstoff oder Fluor stehen,

$R^{48}$ für Wasserstoff, Fluor oder Methyl steht,

Gruppe (23): Iodochromone der allgemeinen Formel (XV)

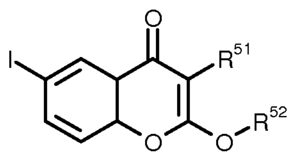

(XV)

in welcher

$R^{51}$ für $C_1$-$C_6$-Alkyl steht,

$R^{52}$ für $C_1$-$C_6$-Alkyl, $C_2$-$C_6$-Alkenyl oder $C_2$-$C_6$-Alkinyl steht.

Überraschenderweise ist die fungizide Wirkung der erfindungsgemäßen Wirkstoffkombinationen wesentlich höher als die Summe der Wirkungen der einzelnen Wirkstoffe. Es liegt also ein nicht vorhersehbarer, echter synergistischer Effekt vor und nicht nur eine Wirkungsergänzung.

Die Verbindungen der Gruppe (1) sind durch die Formel (I) allgemein definiert.

Bevorzugt sind Carboxamide der Formel (I), in welcher

$R^1$ für Wasserstoff oder Fluor steht,

$R^2$ für Fluor, Chlor, Brom, Iod, Methyl, Trifluormethyl, Trifluormethoxy oder für $-C(R^4)=N-OR^5$ steht,

$R^3$ für Wasserstoff, Fluor, Chlor, Brom, Methyl oder Trifluormethyl steht,

$R^4$ für Wasserstoff oder Methyl steht,

$R^5$ für $C_1$-$C_5$-Alkyl steht,

A für einen der folgenden Reste A1 bis A7 steht:

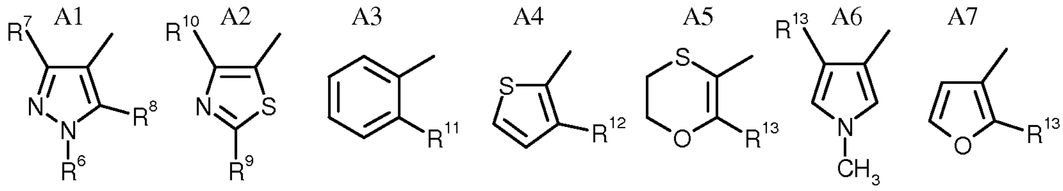

A1 A2 A3 A4 A5 A6 A7

$R^6$ für Methyl steht,

$R^7$ für Iod, Methyl, Difluormethyl oder Trifluormethyl steht,

$R^8$ für Wasserstoff, Fluor, Chlor oder Methyl steht,

$R^9$ für Wasserstoff, Chlor, Methyl, Amino oder Dimethylamino steht,

$R^{10}$ für Methyl, Difluormethyl oder Trifluormethyl steht,

$R^{11}$ für Chlor, Brom, Iod, Methyl, Difluormethyl oder Trifluormethyl steht,

$R^{12}$ für Brom oder Methyl steht,

$R^{13}$ für Methyl oder Trifluormethyl steht.

Besonders bevorzugt sind Carboxamide der Formel (I), in welcher

$R^1$ für Wasserstoff oder Fluor steht,

$R^2$ für Fluor, Chlor, Brom, Trifluormethyl oder für -CH=N-$OCH_3$ steht,

$R^3$ für Wasserstoff, Fluor oder Chlor steht,

A für einen der folgenden Reste A1 bis A5 steht:

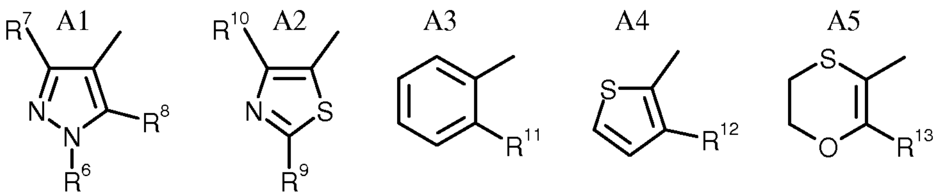

A1 A2 A3 A4 A5

$R^6$ für Methyl steht,

$R^7$ für Methyl, Difluormethyl oder Trifluormethyl steht,

$R^8$ für Wasserstoff oder Fluor steht,

$R^9$ für Methyl steht,

$R^{10}$ für Methyl, Difluormethyl oder Trifluormethyl steht,

$R^{11}$ für Iod, Difluormethyl oder Trifluormethyl steht,

$R^{12}$ für Methyl steht,

$R^{13}$ für Methyl oder Trifluormethyl steht.

Ganz besonders bevorzugt sind Carboxamide der Formel (I), in welcher

$R^1$ für Wasserstoff oder Fluor steht,

$R^2$ für Fluor, Chlor, Brom, Trifluormethyl oder für $-CH{=}N{-}OCH_3$ steht,

$R^3$ für Wasserstoff, Fluor oder Chlor steht,

A für einen der folgenden Reste A1 oder A2 steht:

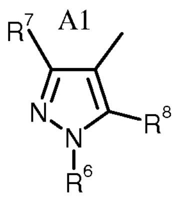
A1

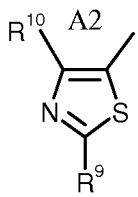
A2

$R^6$ für Methyl steht,

$R^7$ für Methyl, Difluormethyl oder Trifluormethyl steht,

$R^8$ für Wasserstoff oder Fluor steht,

$R^9$ für Methyl steht,

$R^{10}$ für Methyl, Difluormethyl oder Trifluormethyl steht.

Ganz besonders bevorzugt werden Verbindungen der Formel (Ia) in Mischungen eingesetzt,

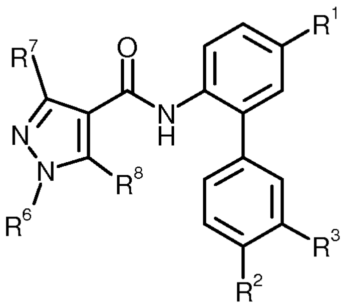

(Ia)

in welcher $R^1$, $R^2$, $R^3$, $R^6$, $R^7$ und $R^8$ die oben angegebenen Bedeutungen haben.

Ganz besonders bevorzugt werden Verbindungen der Formel (Ib) in Mischungen eingesetzt,

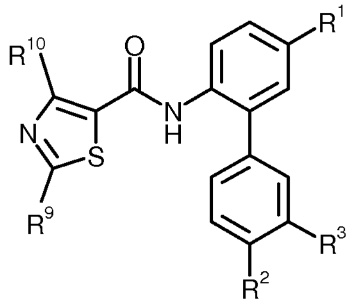

(Ib)

in welcher $R^1$, $R^2$, $R^3$, $R^9$ und $R^{10}$ die oben angegebenen Bedeutungen haben.

Die Formel (I) umfasst insbesondere die folgenden bevorzugten Mischungspartner der Gruppe (1):

(1-1) *N*-(3',4'-Dichlor-5-fluor-1,1'-biphenyl-2-yl)-3-(difluormethyl)-1-methyl-1H-pyrazol-4-carboxamid (bekannt aus WO 03/070705)

(1-2) 3-(Difluormethyl)-*N*-{3'-fluor-4'-[(*E*)-(methoxyimino)methyl]-1,1'-biphenyl-2-yl}-1-methyl-1*H*-pyrazol-4-carboxamid (bekannt aus WO 02/08197)

(1-3) 3-(Trifluormethyl)-*N*-{3'-fluor-4'-[(*E*)-(methoxyimino)methyl]-1,1'-biphenyl-2-yl}-1-methyl-1*H*-pyrazol-4-carboxamid (bekannt aus WO 02/08197)

(1-4) *N*-(3',4'-Dichlor-1,1'-biphenyl-2-yl)-5-fluor-1,3-dimethyl-1*H*-pyrazol-4-carboxamid (bekannt aus WO 00/14701)

(1-5) *N*-(4'-Chlor-3'-fluor-1,1'-biphenyl-2-yl)-2-methyl-4-(trifluormethyl)-1,3-thiazol-5-carboxamid (bekannt aus WO 03/066609)

(1-6) *N*-(4'-Chlor-1,1'-biphenyl-2-yl)-4-(difluormethyl)-2-methyl-1,3-thiazol-5-carboxamid (bekannt aus WO 03/066610)

(1-7) *N*-(4'-Brom-1,1'-biphenyl-2-yl)-4-(difluormethyl)-2-methyl-1,3-thiazol-5-carboxamid (bekannt aus WO 03/066610)

(1-8) 4-(Difluormethyl)-2-methyl-*N*-[4'-(trifluormethyl)-1,1'-biphenyl-2-yl]-1,3-thiazol-5-carboxamid (bekannt aus WO 03/066610)

(1-9) *N*-(4'-Chlor-3'-fluor-1,1'-biphenyl-2-yl)-4-(difluormethyl)-2-methyl-1,3-thiazol-5-carboxamid (bekannt aus WO 03/066610)

Hervorgehoben sind erfindungsgemäße Wirkstoffkombinationen, die neben dem Carboxamid (1-1) *N*-(3',4'-Dichlor-5-fluor-1,1'-biphenyl-2-yl)-3-(difluormethyl)-1-methyl-1*H*-pyrazol-4-carboxamid (Gruppe 1) einen oder mehrere, bevorzugt einen, Mischungspartner der Gruppen (2) bis (23) enthält.

Hervorgehoben sind erfindungsgemäße Wirkstoffkombinationen, die neben dem Carboxamid (1-7) *N*-(4'-Brom-1,1'-biphenyl-2-yl)-4-(difluormethyl)-2-methyl-1,3-thiazol-5-carboxamid (Gruppe 1) einen oder mehrere, bevorzugt einen, Mischungspartner der Gruppen (2) bis (23) enthält.

Hervorgehoben sind erfindungsgemäße Wirkstoffkombinationen, die neben dem Carboxamid (1-8) 4-(Difluormethyl)-2-methyl-*N*-[4'-(trifluormethyl)-1,1'-biphenyl-2-yl]-1,3-thiazol-5-carboxamid (Gruppe 1) einen oder mehrere, bevorzugt einen, Mischungspartner der Gruppen (2) bis (23) enthält.

Hervorgehoben sind erfindungsgemäße Wirkstoffkombinationen, die neben dem Carboxamid (1-9) *N*-(4'-Chlor-3'-fluor-1,1'-biphenyl-2-yl)-4-(difluormethyl)-2-methyl-1,3-thiazol-5-carboxamid (Gruppe 1) einen oder mehrere, bevorzugt einen, Mischungspartner der Gruppen (2) bis (23) enthält.

Die Formel (II) umfasst folgende bevorzugte Mischungspartner der Gruppe (2):

(2-1) Azoxystrobin (bekannt aus EP-A 0 382 375) der Formel

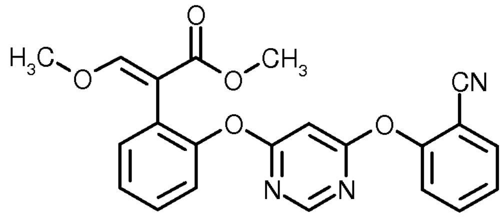

(2-2) Fluoxastrobin (bekannt aus DE-A 196 02 095) der Formel

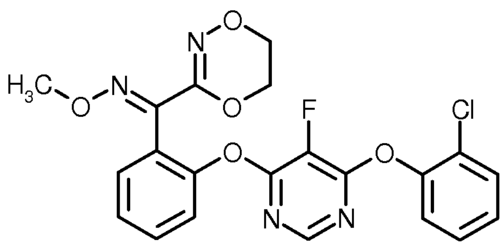

(2-3) (2*E*)-2-(2-{[6-(3-Chlor-2-methylphenoxy)-5-fluor-4-pyrimidinyl]oxy}phenyl)-2-(methoxyimino)-*N*-methylethanamid (bekannt aus DE-A 196 46 407, EP-B 0 712 396) der Formel

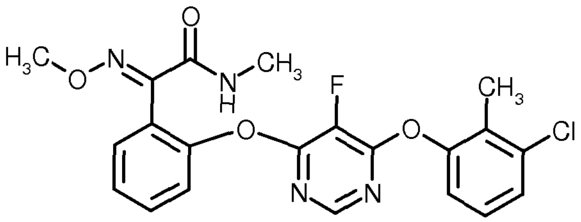

(2-4) Trifloxystrobin (bekannt aus EP-A 0 460 575) der Formel

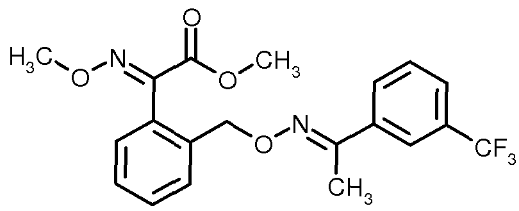

(2-5) (2*E*)-2-(Methoxyimino)-*N*-methyl-2-(2-{[({(1*E*)-1-[3-(trifluormethyl)phenyl]ethyliden}-amino)oxy]methyl}phenyl)ethanamid (bekannt aus EP-A 0 569 384) der Formel

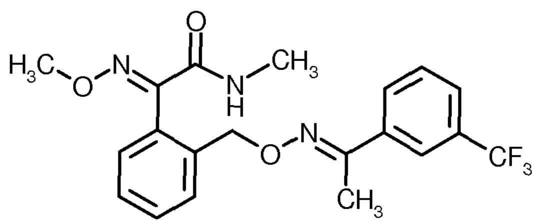

(2-6) (2*E*)-2-(Methoxyimino)-*N*-methyl-2-{2-[(*E*)-({1-[3-(trifluormethyl)phenyl]ethoxy}imino)-methyl]phenyl}ethanamid (bekannt aus EP-A 0 596 254) der Formel

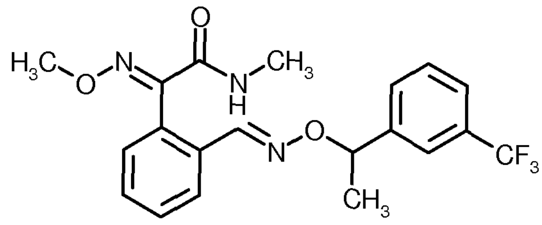

(2-7) Orysastrobin (bekannt aus DE-A 195 39 324) der Formel

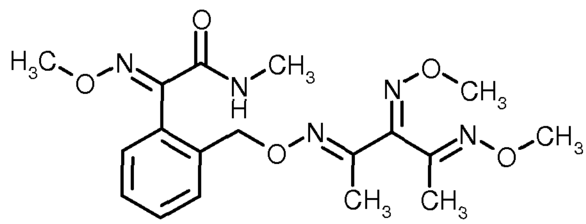

(2-8) 5-Methoxy-2-methyl-4-(2-{[({(1*E*)-1-[3-(trifluormethyl)phenyl]ethyliden}amino)oxy]-methyl}phenyl)-2,4-dihydro-3*H*-1,2,4-triazol-3-on (bekannt aus WO 98/23155) der Formel

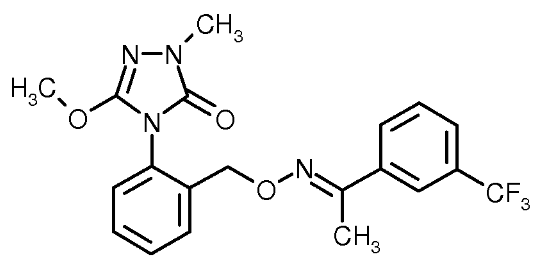

(2-9) Kresoxim-methyl (bekannt aus EP-A 0 253 213) der Formel

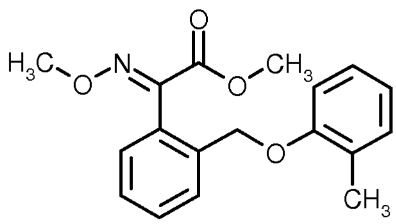

(2-10) Dimoxystrobin (bekannt aus EP-A 0 398 692) der Formel

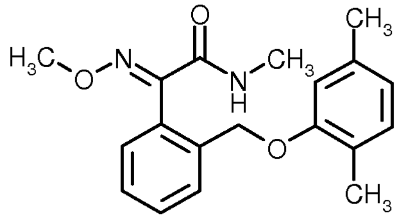

(2-11) Picoxystrobin (bekannt aus EP-A 0 278 595) der Formel

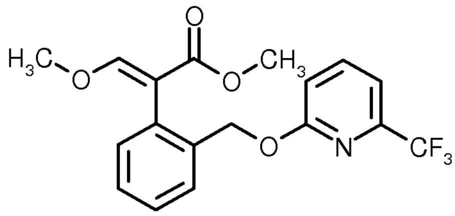

(2-12) Pyraclostrobin (bekannt aus DE-A 44 23 612) der Formel

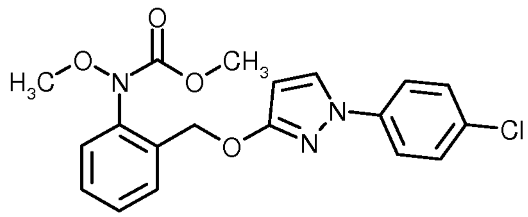

(2-13) Metominostrobin (bekannt aus EP-A 0 398 692) der Formel

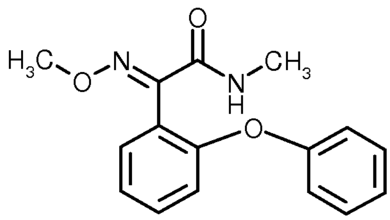

Die Formel (III) umfasst folgende bevorzugte Mischungspartner der Gruppe (3):

(3-1) Azaconazole (bekannt aus DE-A 25 51 560) der Formel

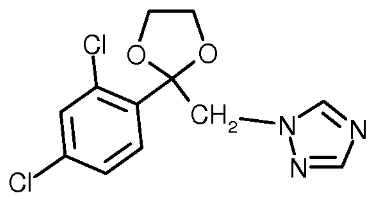

(3-2) Etaconazole (bekannt aus DE-A 25 51 560) der Formel

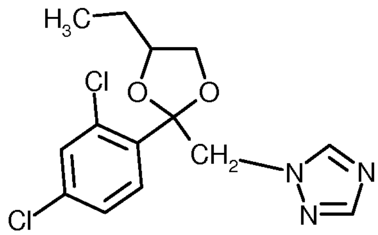

(3-3) Propiconazole (bekannt aus DE-A 25 51 560) der Formel

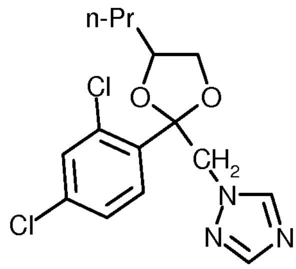

(3-4) Difenoconazole (bekannt aus EP-A 0 112 284) der Formel

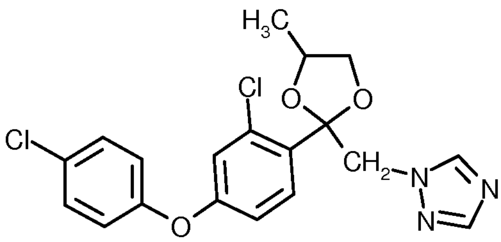

(3-5) Bromuconazole (bekannt aus EP-A 0 258 161) der Formel

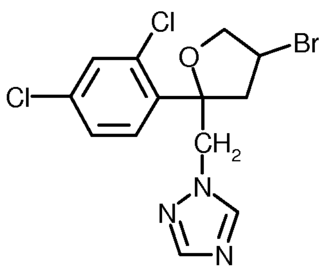

(3-6) Cyproconazole (bekannt aus DE-A 34 06 993) der Formel

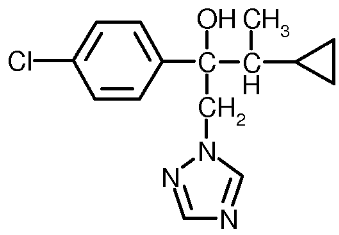

(3-7) Hexaconazole (bekannt aus DE-A 30 42 303) der Formel

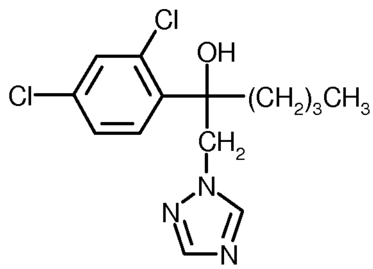

(3-8) Penconazole (bekannt aus DE-A 27 35 872 ) der Formel

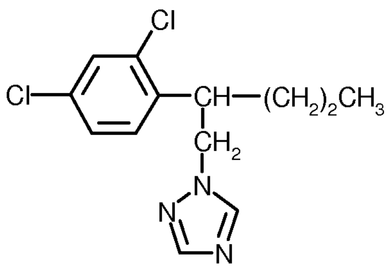

(3-9) Myclobutanil (bekannt aus EP-A 0 145 294) der Formel

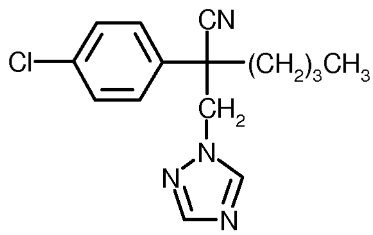

(3-10) Tetraconazole (bekannt aus EP-A 0 234 242) der Formel

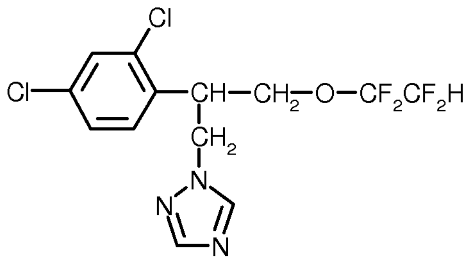

(3-11) Flutriafol (bekannt aus EP-A 0015 756) der Formel

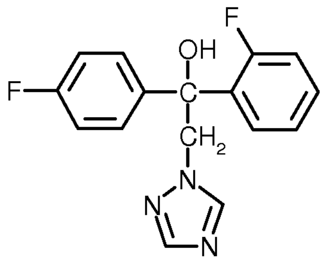

(3-12) Epoxiconazole (bekannt aus EP-A 0 196 038) der Formel

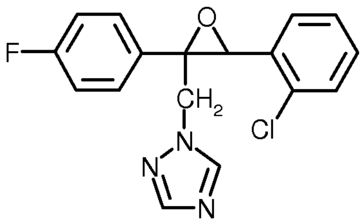

(3-13) Flusilazole (bekannt aus EP-A 0 068 813) der Formel

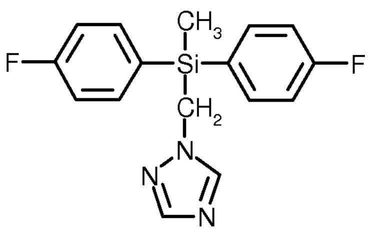

(3-14) Simeconazole (bekannt aus EP-A 0 537 957) der Formel

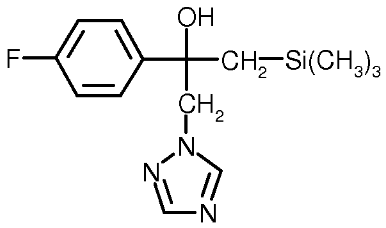

(3-15) Prothioconazole (bekannt aus WO 96/16048) der Formel

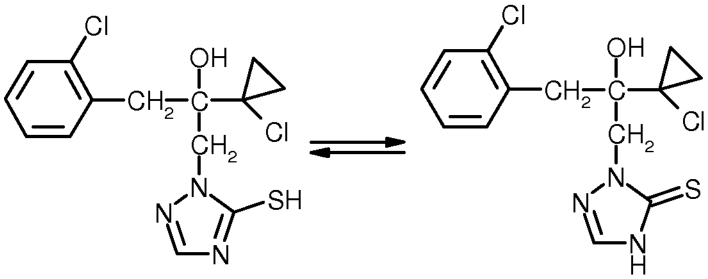

(3-16) Fenbuconazole (bekannt aus DE-A 37 21 786) der Formel

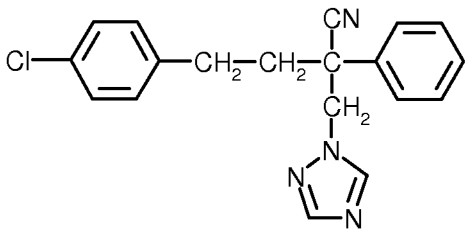

(3-17) Tebuconazole (bekannt aus EP-A 0 040 345) der Formel

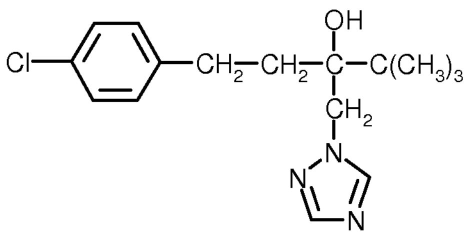

(3-18) Ipconazole (bekannt aus EP-A 0 329 397) der Formel

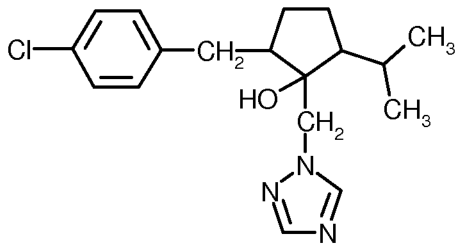

(3-19) Metconazole (bekannt aus EP-A 0 329 397) der Formel

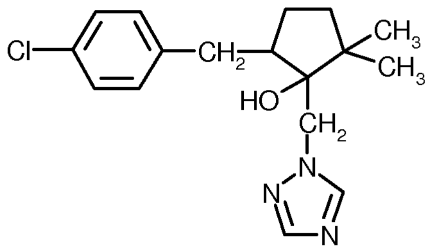

(3-20) Triticonazole (bekannt aus EP-A 0 378 953) der Formel

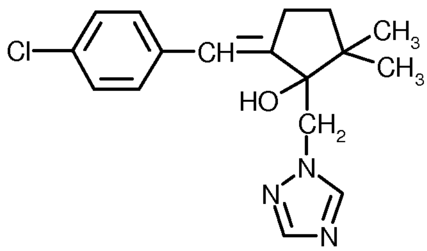

(3-21) Bitertanol (bekannt aus DE-A 23 24 010) der Formel

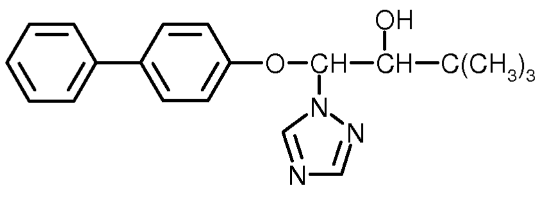

(3-22) Triadimenol (bekannt aus DE-A 23 24 010) der Formel

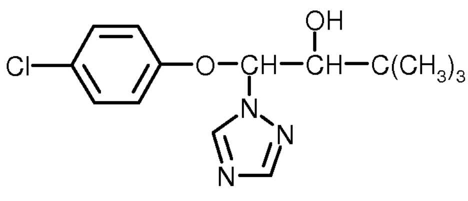

(3-23) Triadimefon (bekannt aus DE-A 22 01 063) der Formel

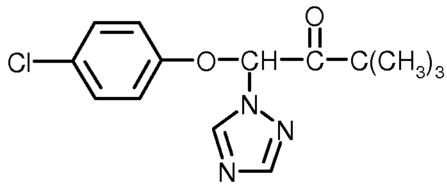

(3-24) Fluquinconazole (bekannt aus EP-A 0 183 458) der Formel

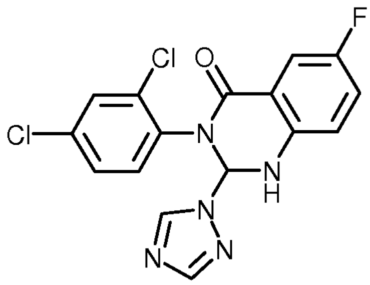

(3-25) Quinconazole (bekannt aus EP-A 0 183 458) der Formel

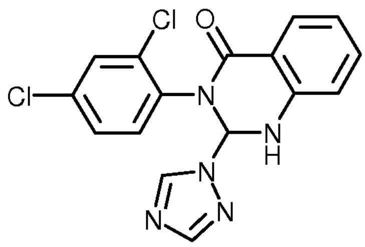

Die Formel (IV) umfasst folgende bevorzugte Mischungspartner der Gruppe (4):

(4-1) Dichlofluanid (bekannt aus DE-A 11 93 498) der Formel

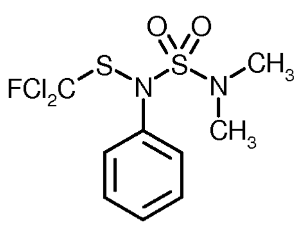

(4-2) Tolylfluanid (bekannt aus DE-A 11 93 498) der Formel

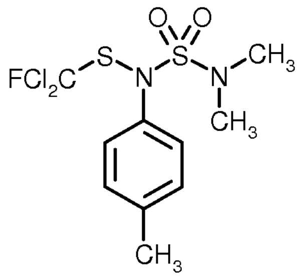

Bevorzugte Mischungspartner der Gruppe (5) sind

(5-1) Iprovalicarb (bekannt aus DE-A 40 26 966) der Formel

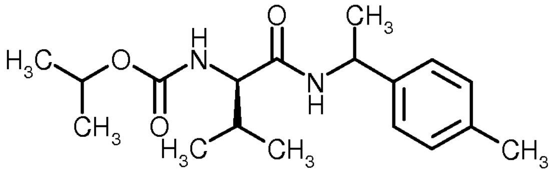

(5-3) Benthiavalicarb (bekannt aus WO 96/04252) der Formel

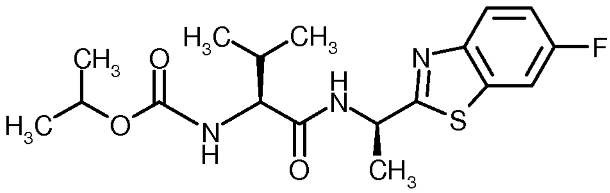

Die Formel (V) umfasst folgende bevorzugte Mischungspartner der Gruppe (6):

(6-1) 2-Chloro-N-(1,1,3-trimethyl-indan-4-yl)-nicotinamid (bekannt aus EP-A 0256503) der Formel

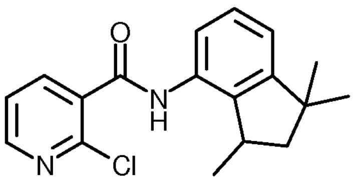

(6-2) Boscalid (bekannt aus DE-A 195 31 813) der Formel

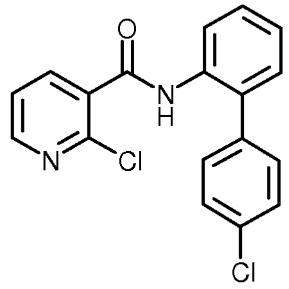

(6-3) Furametpyr (bekannt aus EP-A 0 315 502) der Formel

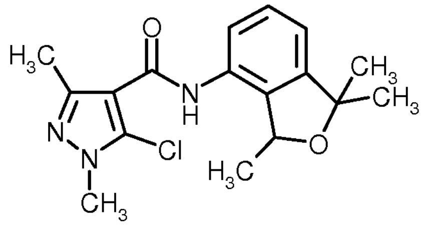

(6-4) 1-Methyl-3-trifluormethyl-1H-pyrazol-4-carbonsäure-(3-p-tolyl-thiophen-2-yl)-amid (bekannt aus EP-A 0 737 682) der Formel

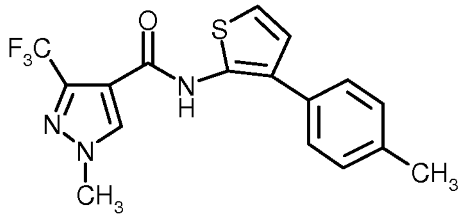

(6-5) Ethaboxam (bekannt aus EP-A 0 639 574) der Formel

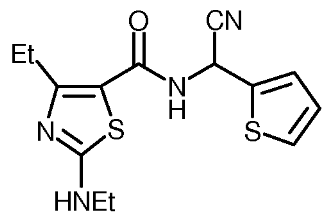

(6-6) Fenhexamid (bekannt aus EP-A 0 339 418) der Formel

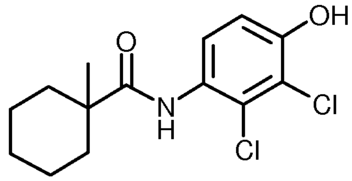

(6-7) Carpropamid (bekannt aus EP-A 0 341 475) der Formel

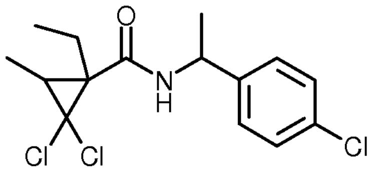

(6-8) 2-Chlor-4-(2-fluor-2-methyl-propionylamino)-N,N-dimethyl-benzamid (bekannt aus EP-A 0 600 629) der Formel

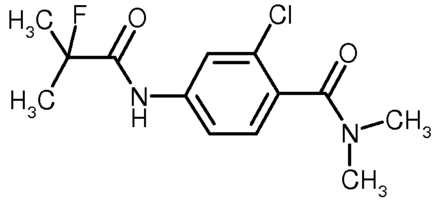

(6-9) Picobenzamid (bekannt aus WO 99/42447) der Formel

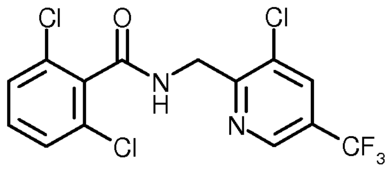

(6-10) Zoxamide (bekannt aus EP-A 0 604 019) der Formel

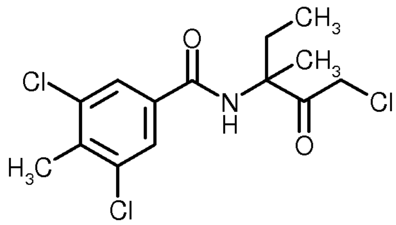

(6-11) 3,4-Dichlor-N-(2-cyanophenyl)isothiazol-5-carboxamid (bekannt aus WO 99/24413) der Formel

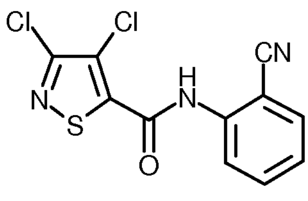

(6-12) Carboxin (bekannt aus US 3,249,499) der Formel

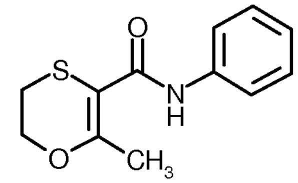

(6-13) Tiadinil (bekannt aus US 6,616,054) der Formel

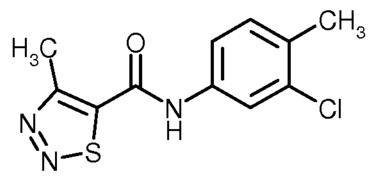

(6-14) Penthiopyrad (bekannt aus EP-A 0 737 682) der Formel

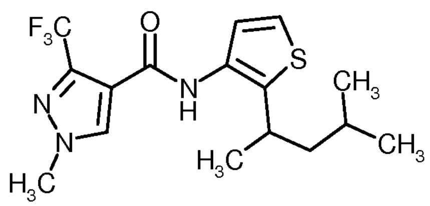

(6-15) Silthiofam (bekannt aus WO 96/18631) der Formel

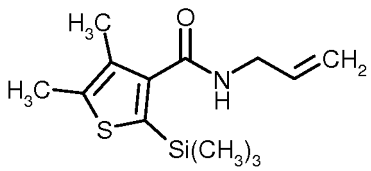

(6-16) *N*-[2-(1,3-Dimethylbutyl)phenyl]-1-methyl-4-(trifluormethyl)-1*H*-pyrrol-3-carboxamid (bekannt aus WO 02/38542) der Formel

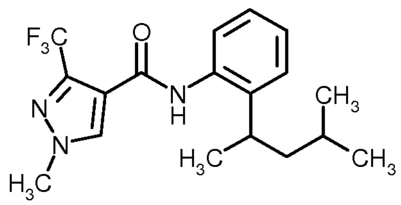

Bevorzugte Mischungspartner der Gruppe (7) sind

(7-1) Mancozeb (bekannt aus DE-A 12 34 704) mit dem IUPAC-Namen Manganese ethylenebis(dithiocarbamate) (polymeric) complex with zinc salt

(7-2) Maneb (bekannt aus US 2,504,404) der Formel

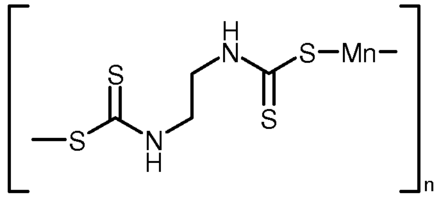

(7-3) Metiram (bekannt aus DE-A 10 76 434) mit dem IUPAC-Namen Zinc ammoniate ethylenebis(dithiocarbamate) - poly(ethylenethiuram disulfide)

(7-4) Propineb (bekannt aus GB 935 981) der Formel

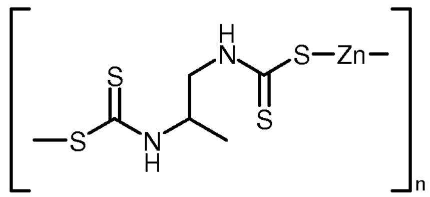

(7-5) Thiram (bekannt aus US 1,972,961) der Formel

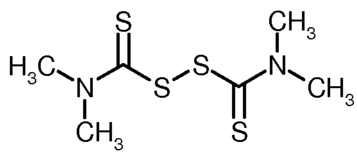

(7-6) Zineb (bekannt aus DE-A 10 81 446) der Formel

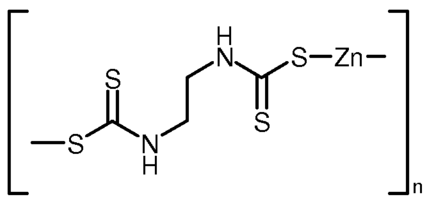

(7-7) Ziram (bekannt aus US 2,588,428) der Formel

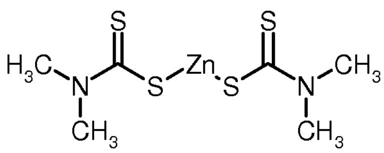

Die Formel (VI) umfasst folgende bevorzugte Mischungspartner der Gruppe (8):

(8-1) Benalaxyl (bekannt aus DE-A 29 03 612) der Formel

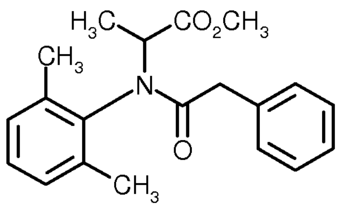

(8-2) Furalaxyl (bekannt aus DE-A 25 13 732) der Formel

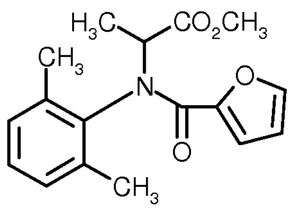

(8-3) Metalaxyl (bekannt aus DE-A 25 15 091) der Formel

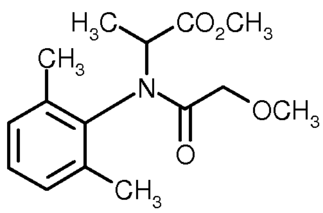

(8-4) Metalaxyl-M (bekannt aus WO 96/01559) der Formel

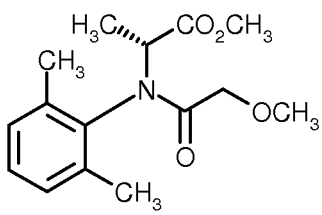

(8-5) Benalaxyl-M der Formel

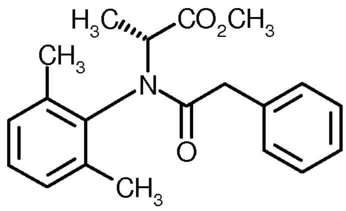

Die Formel (VII) umfasst folgende bevorzugte Mischungspartner der Gruppe (9):

(9-1) Cyprodinil (bekannt aus EP-A 0 310 550) der Formel

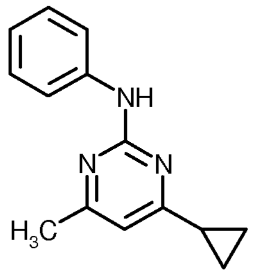

(9-2) Mepanipyrim (bekannt aus EP-A 0 270 111) der Formel

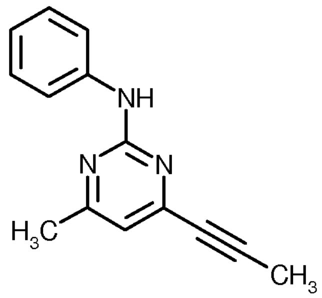

(9-3) Pyrimethanil (bekannt aus DD 151 404) der Formel

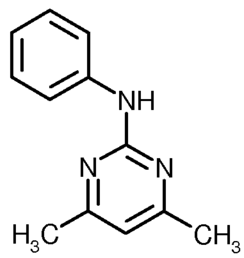

Die Formel (VIII) umfasst folgende bevorzugte Mischungspartner der Gruppe (10):

(10-1) 6-Chlor-5-[(3,5-dimethylisoxazol-4-yl)sulfonyl]-2,2-difluor-5H-[1,3]dioxolo[4,5-f]-benzimidazol (bekannt aus WO 97/06171) der Formel

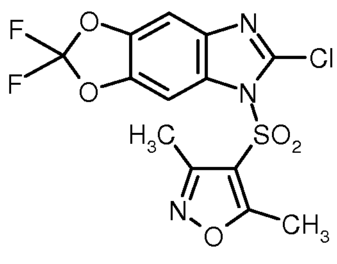

(10-2) Benomyl (bekannt aus US 3,631,176) der Formel

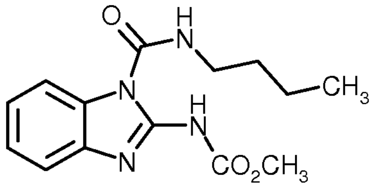

(10-3) Carbendazim (bekannt aus US 3,010,968) der Formel

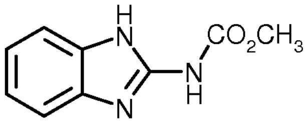

(10-4) Chlorfenazole der Formel

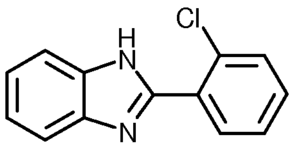

(10-5) Fuberidazole (bekannt aus DE-A 12 09 799) der Formel

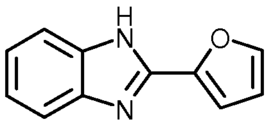

(10-6) Thiabendazole (bekannt aus US 3,206,468) der Formel

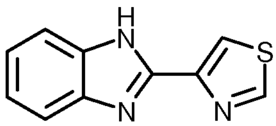

Die Formel (IX) umfasst folgende bevorzugte Mischungspartner der Gruppe (11):

(11-1) Diethofencarb (bekannt aus EP-A 0 078 663) der Formel

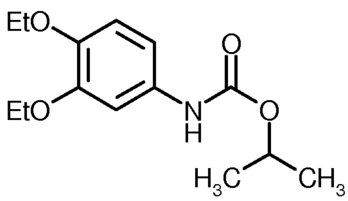

(11-2) Propamocarb (bekannt aus US 3,513,241) der Formel

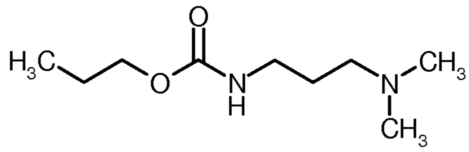

(11-3) Propamocarb-hydrochloride (bekannt aus US 3,513,241) der Formel

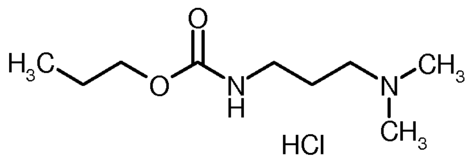

(11-4) Propamocarb-Fosetyl der Formel

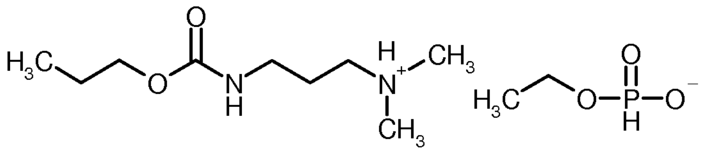

Bevorzugte Mischungspartner der Gruppe (12) sind

(12-1) Captafol (bekannt aus US 3,178,447) der Formel

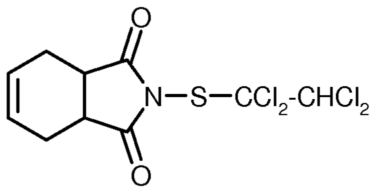

(12-2) Captan (bekannt aus US 2,553,770) der Formel

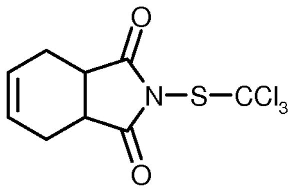

(12-3) Folpet (bekannt aus US 2,553,770) der Formel

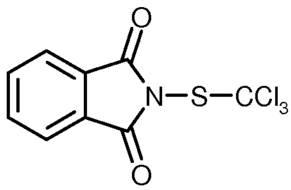

(12-4) Iprodione (bekannt aus DE-A 21 49 923) der Formel

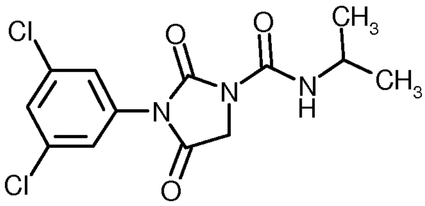

(12-5) Procymidone (bekannt aus DE-A 20 12 656) der Formel

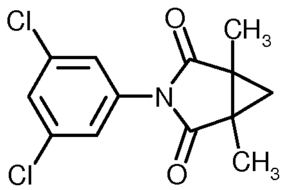

(12-6) Vinclozolin (bekannt aus DE-A 22 07 576) der Formel

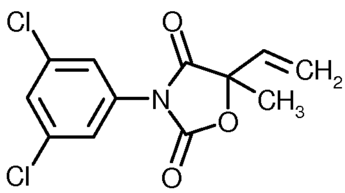

Bevorzugte Mischungspartner der Gruppe (13) sind

(13-1) Dodine (bekannt aus GB 11 03 989) der Formel

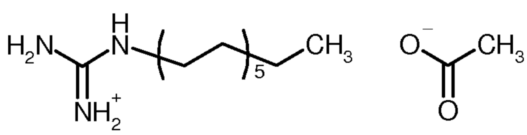

(13-2) Guazatine (bekannt aus GB 11 14 155)

(13-3) Iminoctadine triacetate (bekannt aus EP-A 0 155 509) der Formel

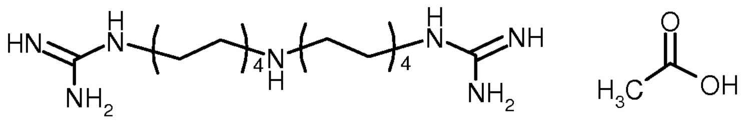

Bevorzugter Mischungspartner der Gruppe (14) ist

(14-1) Cyazofamid (bekannt aus EP-A 0 298 196) der Formel

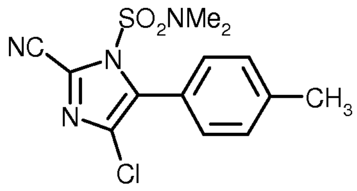

(14-2) Prochloraz (bekannt aus DE-A 24 29 523) der Formel

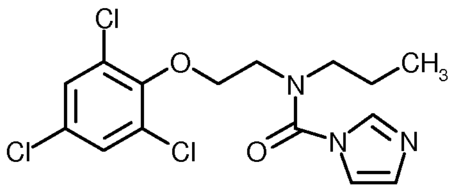

(14-3) Triazoxide (bekannt aus DE-A 28 02 488) der Formel

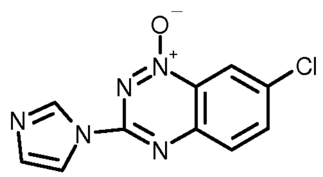

(14-4) Pefurazoate (bekannt aus EP-A 0 248 086) der Formel

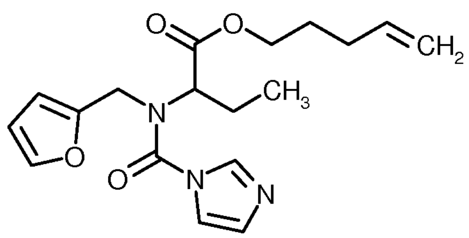

Die Formel (X) umfasst folgende bevorzugte Mischungspartner der Gruppe (15):

(15-1) Aldimorph (bekannt aus DD 140 041) der Formel

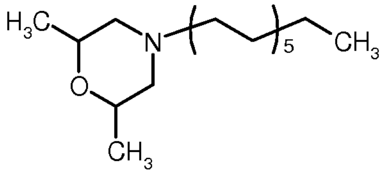

(15-2) Tridemorph (bekannt aus GB 988 630) der Formel

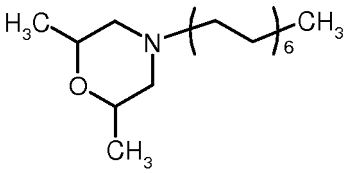

(15-3) Dodemorph (bekannt aus DE-A 25 432 79) der Formel

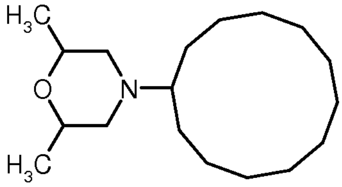

(15-4) Fenpropimorph (bekannt aus DE-A 26 56 747) der Formel

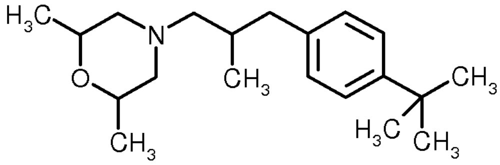

(15-5) Dimethomorph (bekannt aus EP-A 0 219 756) der Formel

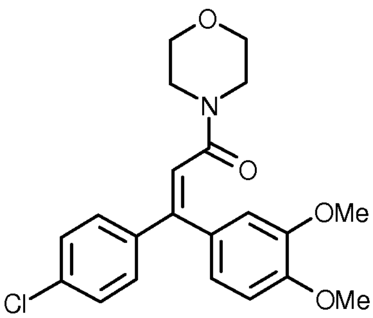

Die Formel (XI) umfasst folgende bevorzugte Mischungspartner der Gruppe (16):

(16-1) Fenpiclonil (bekannt aus EP-A 0 236 272) der Formel

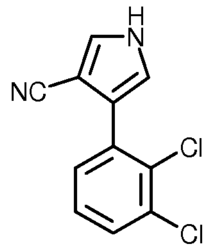

(16-2) Fludioxonil (bekannt aus EP-A 0 206 999) der Formel

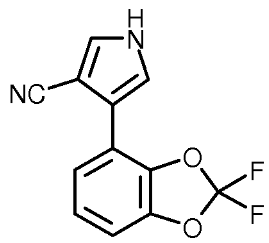

(16-3) Pyrrolnitrine (bekannt aus JP 65-25876) der Formel

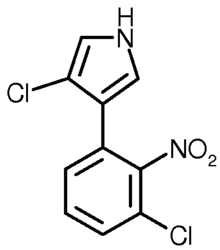

Bevorzugte Mischungspartner der Gruppe (17) sind

(17-1) Fosetyl-Al (bekannt aus DE-A 24 56 627) der Formel

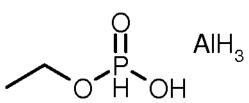

(17-2) Phosphonic acid (bekannte Chemikalie) der Formel

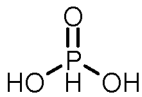

Die Formel (XII) umfasst folgende bevorzugte Mischungspartner der Gruppe (18), welche aus WO 96/23793 bekannt sind und jeweils als E- oder Z-Isomere vorliegen können. Verbindungen der Formel (XII) können daher als

Gemisch von verschiedenen Isomeren oder auch in Form eines einzigen Isomeren vorliegen. Bevorzugt sind Verbindungen der Formel (XII) in Form ihres E-Isomers.

(18-1) 2-(2,3-Dihydro-1H-inden-5-yl)-N-[2-(3,4-dimethoxyphenyl)ethyl]-2-(methoxyimino)acetamid der Formel

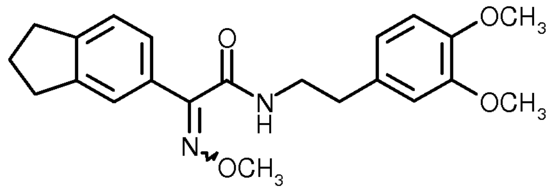

(18-2) N-[2-(3,4-Dimethoxyphenyl)ethyl]-2-(methoxyimino)-2-(5,6,7,8-tetrahydronaphthalen-2-yl)acetamid der Formel

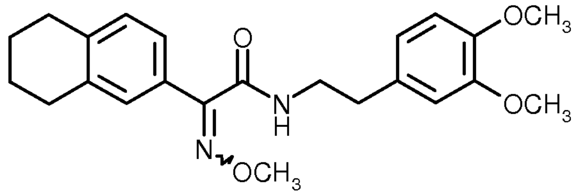

(18-3) 2-(4-Chlorphenyl)-N-[2-(3,4-dimethoxyphenyl)ethyl]-2-(methoxyimino)acetamid der Formel

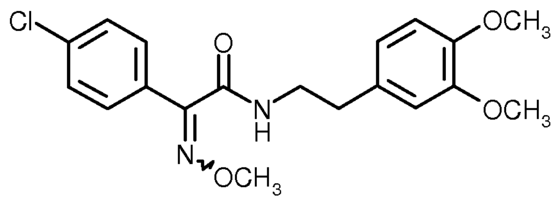

(18-4) 2-(4-Bromphenyl)-N-[2-(3,4-dimethoxyphenyl)ethyl]-2-(methoxyimino)acetamid der Formel

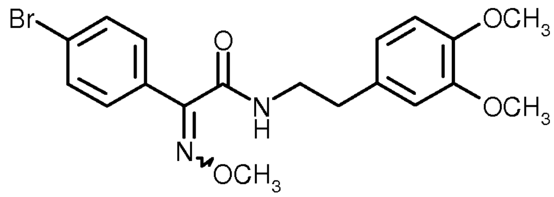

(18-5) 2-(4-Methylphenyl)-N-[2-(3,4-dimethoxyphenyl)ethyl]-2-(methoxyimino)acetamid der Formel

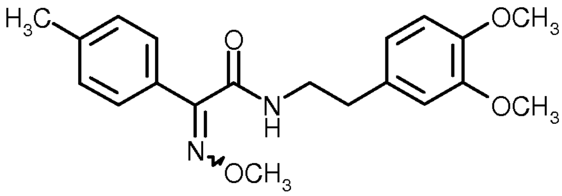

(18-6) 2-(4-Ethylphenyl)-N-[2-(3,4-dimethoxyphenyl)ethyl]-2-(methoxyimino)acetamid der Formel

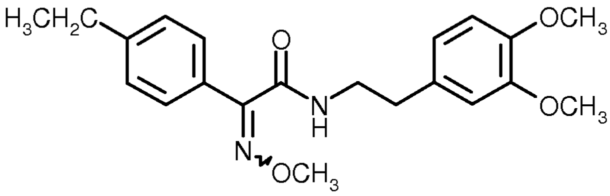

Bevorzugte Mischungspartner der Gruppe (19) sind

(19-1) Acibenzolar-S-methyl (bekannt aus EP-A 0 313 512) der Formel

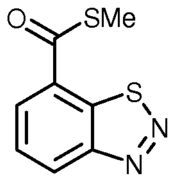

(19-2) Chlorothalonil (bekannt aus US 3,290,353) der Formel

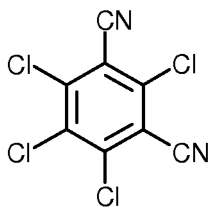

(19-3) Cymoxanil (bekannt aus DE-A 23 12 956) der Formel

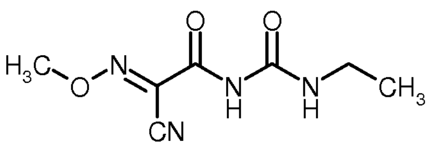

(19-4) Edifenphos (bekannt aus DE-A 14 93 736) der Formel

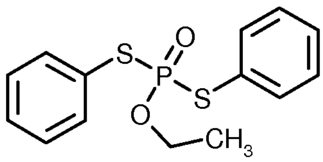

(19-5) Famoxadone (bekannt aus EP-A 0 393 911) der Formel

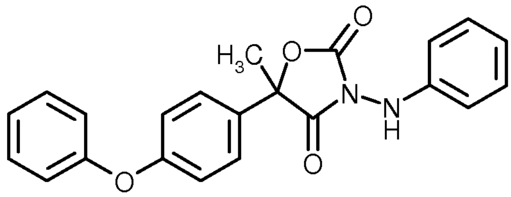

(19-6) Fluazinam (bekannt aus EP-A 0 031 257) der Formel

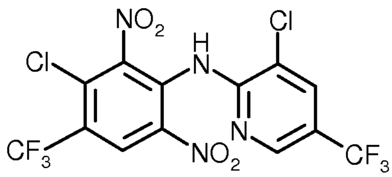

(19-7) Kupferoxychlorid
(19-9) Oxadixyl (bekannt aus DE-A 30 30 026) der Formel

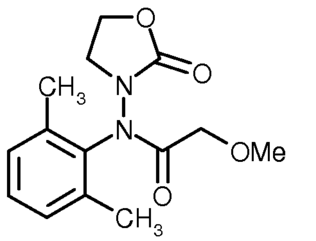

(19-10) Spiroxamine (bekannt aus DE-A 37 35 555) der Formel

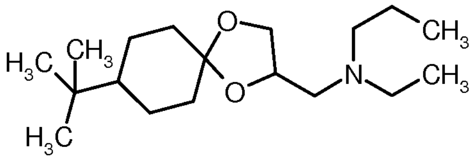

(19-11) Dithianon (bekannt aus JP-A 44-29464) der Formel

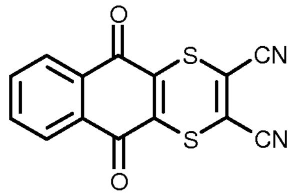

(19-12) Metrafenone (bekannt aus EP-A 0 897 904) der Formel

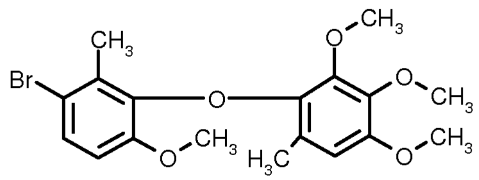

(19-13) Fenamidone (bekannt aus EP-A 0 629 616) der Formel

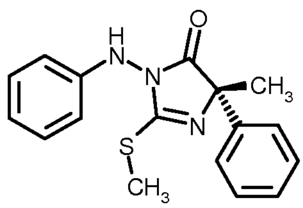

(19-14) 2,3-Dibutyl-6-chlor-thieno[2,3-d]pyrimidin-4(3H)on (bekannt aus WO 99/14202) der Formel

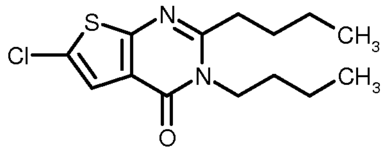

(19-15) Probenazole (bekannt aus US 3,629,428) der Formel

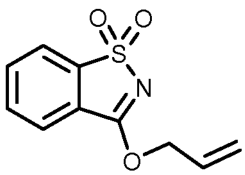

(19-16) Isoprothiolane (bekannt aus US 3,856,814) der Formel

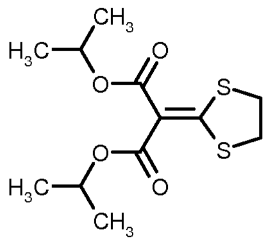

(19-17) Kasugamycin (bekannt aus GB 1 094 567) der Formel

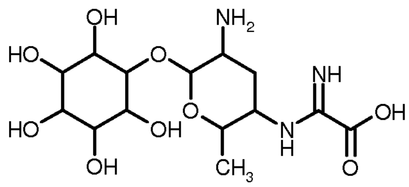

(19-18) Phthalide (bekannt aus JP-A 57-55844) der Formel

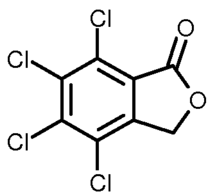

(19-19) Ferimzone (bekannt aus EP-A 0 019 450) der Formel

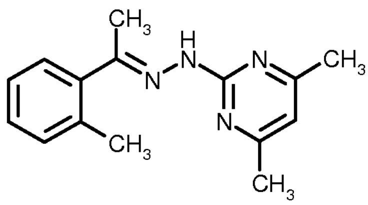

(19-20) Tricyclazole (bekannt aus DE-A 22 50 077) der Formel

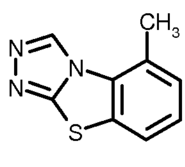

(19-21) N-({4-[(Cyclopropylamino)carbonyl]phenyl}sulfonyl)-2-methoxybenzamid der Formel

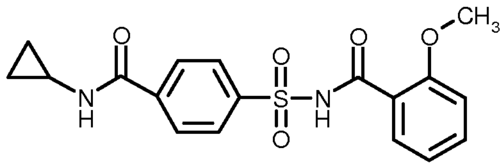

(19-22) 2-(4-Chlorphenyl)-N-{2-[3-methoxy-4-(prop-2-in-1-yloxy)phenyl]ethyl}-2-(prop-2-in-1-yloxy)acetamid (bekannt aus WO 01/87822) der Formel

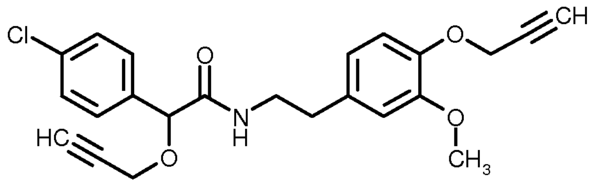

Bevorzugte Mischungspartner der Gruppe (20) sind

(20-1) Pencycuron (bekannt aus DE-A 27 32 257) der Formel

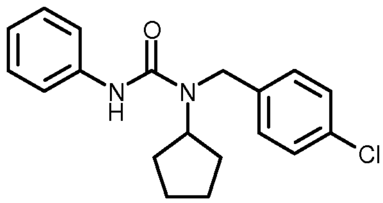

(20-2) Thiophanate-methyl (bekannt aus DE-A 18 06 123) der Formel

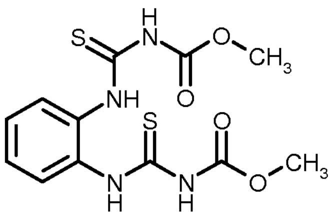

(20-3) Thiophanate-ethyl (bekannt aus DE-A 18 06 123) der Formel

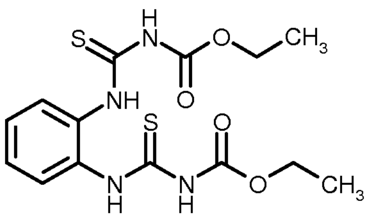

Bevorzugte Mischungspartner der Gruppe (21) sind

(21-1) Fenoxanil (bekannt aus EP-A 0 262 393) der Formel

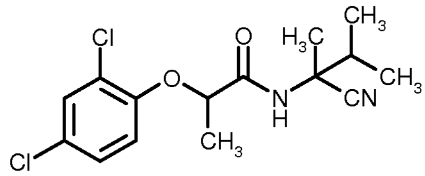

(21-2) Diclocymet (bekannt aus JP-A 7-206608) der Formel

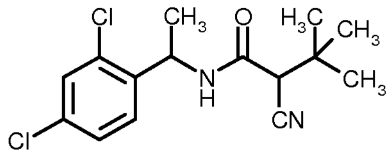

Bevorzugte Mischungspartner der Gruppe (22) sind

(22-1) 5-Chlor-*N*-[(*1S*)-2,2,2-trifluor-1-methylethyl]-6-(2,4,6-trifluorphenyl)[1,2,4]triazolo[1,5-a]-pyrimidin-7-amin (bekannt aus US 5,986,135) der Formel

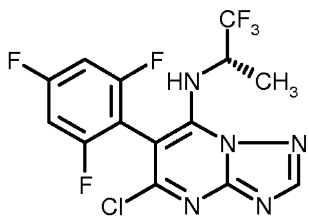

(22-2) 5-Chlor-*N*-[(*1R*)-1,2-dimethylpropyl]-6-(2,4,6-trifluorphenyl)[1,2,4]triazolo[1,5-a]pyrimidin-7-amin (bekannt aus WO 02/38565) der Formel

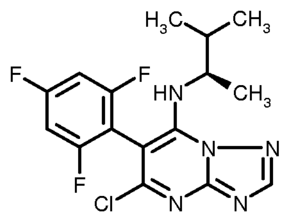

(22-3) 5-Chlor-6-(2-chlor-6-fluorphenyl)-7-(4-methylpiperidin-1-yl)[1,2,4]triazolo[1,5-a]pyrimidin (bekannt aus US 5,593,996) der Formel

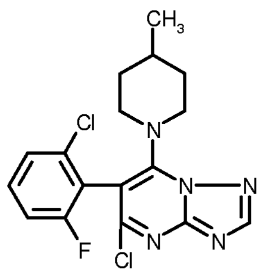

(22-4) 5-Chlor-6-(2,4,6-trifluorphenyl)-7-(4-methylpiperidin-1-yl)[1,2,4]triazolo[1,5-a]pyrimidin (bekannt aus DE-A 101 24 208) der Formel

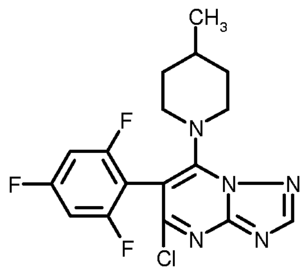

Bevorzugte Mischungspartner der Gruppe (23) sind

(23-1) 2-Butoxy-6-iod-3-propyl-benzopyran-4-on (bekannt aus WO 03/014103) der Formel

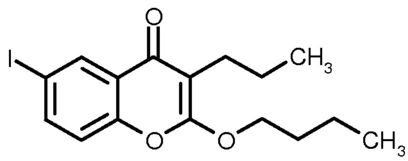

(23-2) 2-Ethoxy-6-iod-3-propyl-benzopyran-4-on (bekannt aus WO 03/014103) der Formel

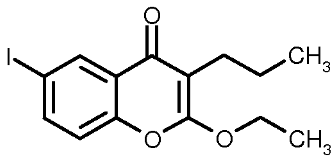

(23-3) 6-Iod-2-propoxy-3-propyl-benzopyran-4-on (bekannt aus WO 03/014103) der Formel

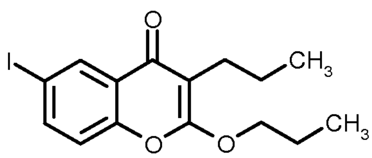

(23-4) 2-But-2-inyloxy-6-iod-3-propyl-benzopyran-4-on (bekannt aus WO 03/014103) der Formel

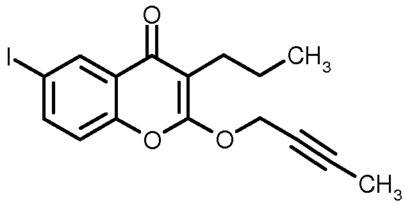

(23-5) 6-Iod-2-(1-methyl-butoxy)-3-propyl-benzopyran-4-on (bekannt aus WO 03/014103) der Formel

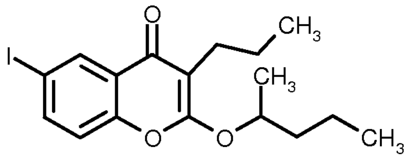

(23-6) 2-But-3-enyloxy-6-iod-benzopyran-4-on (bekannt aus WO 03/014103) der Formel

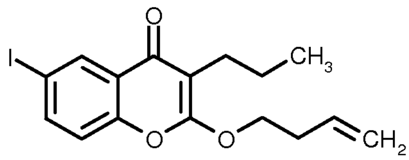

(23-7) 3-Butyl-6-iod-2-isopropoxy-benzopyran-4-on (bekannt aus WO 03/014103) der Formel

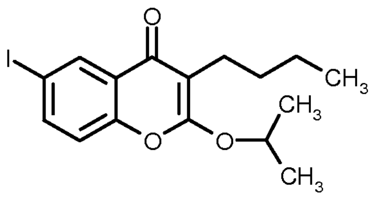

Die Verbindung (6-7) Carpropamid besitzt drei asymmetrische substituierte Kohlenstoffatome. Die Verbindung (6-7) kann daher als Gemisch von verschiedenen Isomeren oder auch in Form einer einzigen Komponente vorliegen. Besonders bevorzugt sind die Verbindungen

(1*S*,3*R*)-2,2-Dichlor-*N*-[(1*R*)-1-(4-chlorphenyl)ethyl]-1-ethyl-3-methylcyclopropancarboxamid der Formel

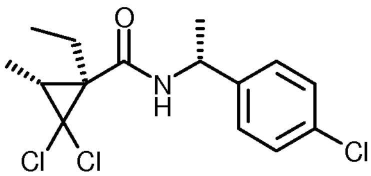

und

(1*R*,3*S*)-2,2-Dichlor-*N*-[(1*R*)-1-(4-chlorphenyl)ethyl]-1-ethyl-3-methylcyclopropancarboxamid der Formel

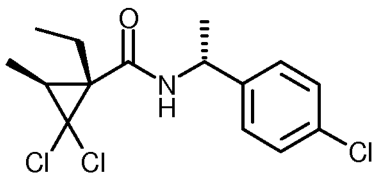

Als Mischungspartner sind die folgenden Wirkstoffe besonders bevorzugt:

(2-1) Azoxystrobin

(2-2) Fluoxastrobin

(2-3) (2*E*)-2-(2-{[6-(3-Chlor-2-methylphenoxy)-5-fluor-4-pyrimidinyl]oxy}phenyl)-2-(methoxyimino)-*N*-methylethanamid

(2-4) Trifloxystrobin

(2-5) (2*E*)-2-(Methoxyimino)-*N*-methyl-2-(2-{[({(1*E*)-1-[3-(trifluormethyl)phenyl]ethyliden}-amino)oxy]methyl}phenyl)ethanamid

(2-6) (2*E*)-2-(Methoxyimino)-*N*-methyl-2-{2-[(*E*)-({1-[3-(trifluormethyl)phenyl]ethoxy}imino)-methyl]phenyl}ethanamid

(2-8) 5-Methoxy-2-methyl-4-(2-{[({(1*E*)-1-[3-(trifluormethyl)phenyl]ethyliden}amino)oxy]-methyl}phenyl)-2,4-dihydro-3*H*-1,2,4-triazol-3-on

(2-11) Picoxystrobin

(2-9) Kresoxim-methyl

(2-10) Dimoxystrobin

(2-12) Pyraclostrobin

(2-13) Metominostrobin

(3-3) Propiconazole

(3-4) Difenoconazole

(3-6) Cyproconazole

(3-7) Hexaconazole

(3-8) Penconazole

(3-9) Myclobutanil

(3-10) Tetraconazole

(3-13) Flusilazole

(3-15) Prothioconazole

(3-16) Fenbuconazole

(3-17) Tebuconazole

(3-21) Bitertanol

(3-22) Triadimenol

(3-23) Triadimefon

(3-12) Epoxiconazole

(3-19) Metconazole

(3-24) Fluquinconazole

(4-1) Dichlofluanid

(4-2) Tolylfluanid

(5-1) Iprovalicarb

(5-3) Benthiavalicarb

(6-2) Boscalid

(6-5) Ethaboxam

(6-6) Fenhexamid

(6-7) Carpropamid

(6-8) 2-Chloro-4-[(2-fluor-2-methylpropanoyl)amino]-*N*,*N*-dimethylbenzamid

(6-9) Picobenzamid

(6-10) Zoxamide

(6-11) 3,4-Dichlor-N-(2-cyanophenyl)isothiazol-5-carboxamid

(6-14) Penthiopyrad

(6-16) *N*-[2-(1,3-Dimethylbutyl)phenyl]-1-methyl-4-(trifluormethyl)-1*H*-pyrrol-3-carboxamid

(7-1) Mancozeb

(7-2) Maneb

(7-4) Propineb

(7-5) Thiram

(7-6) Zineb

(8-1) Benalaxyl

(8-2) Furalaxyl

(8-3) Metalaxyl

(8-4) Metalaxyl-M

(8-5) Benalaxyl-M

(9-1) Cyprodinil

(9-2) Mepanipyrim

(9-3) Pyrimethanil

(10-1) 6-Chlor-5-[(3,5-dimethylisoxazol-4-yl)sulfonyl]-2,2-difluor-5*H*-[1,3]dioxolo[4,5-*f*]benzimidazol

(10-3) Carbendazim

(11-1) Diethofencarb

(11-2) Propamocarb

(11-3) Propamocarb-hydrochloride

(11-4) Propamocarb-Fosetyl

(12-2) Captan

(12-3) Folpet

(12-4) Iprodione

(12-5) Procymidone

(13-1) Dodine

(13-2) Guazatine

(13-3) Iminoctadine triacetate

(14-1) Cyazofamid

(14-2) Prochloraz

(14-3) Triazoxide

(15-5) Dimethomorph

(15-4) Fenpropimorph

(16-2) Fludioxonil

(17-1) Fosetyl-Al

(17-2) Phosphonic acid

(19-1) Acibenzolar-S-methyl

(19-2) Chlorothalonil

(19-3) Cymoxanil

(19-5) Famoxadone

(19-6) Fluazinam

(19-9) Oxadixyl

(19-10) Spiroxamine

(19-7) Kupferoxychlorid

(19-13) Fenamidone

(19-22) 2-(4-Chlorphenyl)-N-{2-[3-methoxy-4-(prop-2-in-1-yloxy)phenyl]ethyl}-2-(prop-2-in-1-yloxy)acetamid

(20-1) Pencycuron

(20-2) Thiophanate-methyl

(22-1) 5-Chlor-*N*-[(*1S*)-2,2,2-trifluor-1-methylethyl]-6-(2,4,6-trifluorphenyl)[1,2,4]triazolo[1,5-a]-pyrimidin-7-amin

(22-2) 5-Chlor-*N*-[(*1R*)-1,2-dimethylpropyl]-6-(2,4,6-trifluorphenyl)[1,2,4]triazolo[1,5-a]pyrimidin-7-amin

(22-4) 5-Chlor-6-(2,4,6-trifluorphenyl)-7-(4-methylpiperidin-1-yl)[1,2,4]triazolo[1,5-a]pyrimidin (23-1) 2-Butoxy-6-iod-3-propyl-benzopyran-4-on

(23-2) 2-Ethoxy-6-iod-3-propyl-benzopyran-4-on

(23-3) 6-Iod-2-propoxy-3-propyl-benzopyran-4-on

Als Mischungspartner sind die folgenden Wirkstoffe ganz besonders bevorzugt:

(2-2) Fluoxastrobin

(2-4) Trifloxystrobin

(2-3) (2*E*)-2-(2-{[6-(3-Chlor-2-methylphenoxy)-5-fluor-4-pyrimidinyl]oxy}phenyl)-2-(methoxyimino)-*N*-methylethanamid

(3-15) Prothioconazole

(3-17) Tebuconazole

(3-21) Bitertanol

(3-22) Triadimenol

(3-24) Fluquinconazole

(4-1) Dichlofluanid

(4-2) Tolylfluanid

(5-1) Iprovalicarb

(6-6) Fenhexamid

(6-9) Picobenzamid

(6-7) Carpropamid

(6-14) Penthiopyrad

(7-4) Propineb

(8-4) Metalaxyl-M

(8-5) Benalaxyl-M

(9-3) Pyrimethanil

(10-3) Carbendazim

(11-4) Propamocarb-Fosetyl

(12-4) Iprodione

(14-2) Prochloraz

(14-3) Triazoxide

(16-2) Fludioxonil

(19-10) Spiroxamine

(19-22) 2-(4-Chlorphenyl)-N-{2-[3-methoxy-4-(prop-2-in-1-yloxy)phenyl]ethyl}-2-(prop-2-in-1-yloxy)acetamid

(22-4) 5-Chlor-6-(2,4,6-trifluorphenyl)-7-(4-methylpiperidin-1-yl)[1,2,4]triazolo[1,5-a]pyrimidin

Im Folgenden werden bevorzugte Wirkstoffkombinationen beschrieben, die aus zwei Gruppen von Wirkstoffen bestehen und jeweils wenigstens ein Carboxamid der Formel (I) (Gruppe 1) und wenigstens einen Wirkstoff der angegebenen Gruppe (2) bis (23) enthalten. Diese Kombinationen sind die Wirkstoffkombinationen A bis T.

Innerhalb der bevorzugten Wirkstoffkombinationen A bis T sind solche hervorzuheben, die ein Carboxamid der Formel (I) (Gruppe 1)

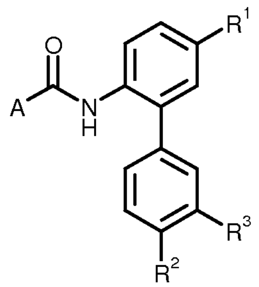

(I)

in welcher $R^1$, $R^2$, $R^3$ und A die oben angegebenen Bedeutungen haben, enthalten.

Besonders bevorzugt sind Wirkstoffkombinationen A bis T, enthaltend ein Carboxamid der Formel (I) (Gruppe 1)

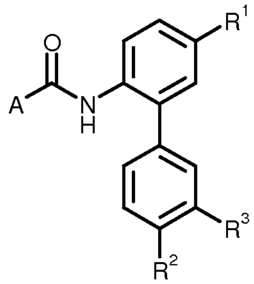

(I)

in welcher

$R^1$ für Wasserstoff oder Fluor steht,

$R^2$ für Fluor, Chlor, Brom, Trifluormethyl oder für $-CH=N-OCH_3$ steht,

$R^3$ für Wasserstoff, Fluor oder Chlor steht,

A für einen der folgenden Reste A1 oder A2 steht:

A1

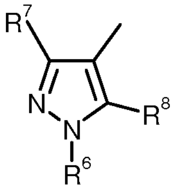

A2

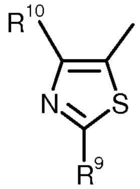

$R^6$ für Methyl steht,

$R^7$ für Methyl, Difluormethyl oder Trifluormethyl steht,

$R^8$ für Wasserstoff oder Fluor steht,

$R^9$ für Methyl steht,

$R^{10}$ für Methyl, Difluormethyl oder Trifluormethyl steht.

Ganz besonders bevorzugt sind Wirkstoffkombinationen A bis T, worin das Carboxamid der Formel (I) (Gruppe 1) aus der folgenden Liste ausgewählt ist:

(1-1) *N*-(3',4'-Dichlor-5-fluor-1,1'-biphenyl-2-yl)-3-(difluormethyl)-1-methyl-1*H*-pyrazol-4-carboxamid

(1-2) 3-(Difluormethyl)-*N*-{3'-fluor-4'-[(*E*)-(methoxyimino)methyl]-1,1'-biphenyl-2-yl}-1-methyl-1*H*-pyrazol-4-carboxamid

(1-3) 3-(Trifluormethyl)-*N*-{3'-fluor-4'-[(*E*)-(methoxyimino)methyl]-1,1'-biphenyl-2-yl}-1-methyl-1*H*-pyrazol-4-carboxamid

(1-4) *N*-(3',4'-Dichlor-1,1'-biphenyl-2-yl)-5-fluor-1,3-dimethyl-1H-pyrazol-4-carboxamid

(1-5) *N*-(4'-Chlor-3'-fluor-1,1'-biphenyl-2-yl)-2-methyl-4-(trifluormethyl)-1,3-thiazol-5-carboxamid

(1-6) *N*-(4'-Chlor-1,1'-biphenyl-2-yl)-4-(difluormethyl)-2-methyl-1,3-thiazol-5-carboxamid

(1-7) *N*-(4'-Brom-1,1'-biphenyl-2-yl)-4-(difluormethyl)-2-methyl-1,3-thiazol-5-carboxamid

(1-8) 4-(Difluormethyl)-2-methyl-*N*-[4'-(trifluormethyl)-1,1'-biphenyl-2-yl]-1,3-thiazol-5-carboxamid

(1-9) *N*-(4'-Chlor-3'-fluor-1,1'-biphenyl-2-yl)-4-(difluormethyl)-2-methyl-1,3-thiazol-5-carboxamid

Insbesondere bevorzugt sind Wirkstoffkombinationen A bis T, worin das Carboxamid der Formel (I) (Gruppe 1) aus der folgenden Liste ausgewählt ist:

(1-1) *N*-(3',4'-Dichlor-5-fluor-1,1'-biphenyl-2-yl)-3-(difluormethyl)-1-methyl-1H-pyrazol-4-carboxamid

(1-7) *N*-(4'-Brom-1,1'-biphenyl-2-yl)-4-(difluormethyl)-2-methyl-1,3-thiazol-5-carboxamid

(1-8) 4-(Difluormethyl)-2-methyl-*N*-[4'-(trifluormethyl)-1,1'-biphenyl-2-yl]-1,3-thiazol-5-carboxamid

(1-9) *N*-(4'-Chlor-3'-fluor-1,1'-biphenyl-2-yl)-4-(difluormethyl)-2-methyl-1,3-thiazol-5-carboxamid

Die Wirkstoffkombinationen A enthalten neben einem Carboxamid der Formel (I) (Gruppe 1) auch ein Strobilurin der Formel (II) (Gruppe 2)

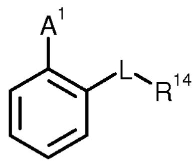

(II)

in welcher $A^1$, L und $R^{14}$ die oben angegebenen Bedeutungen haben.

Besonders bevorzugt sind Wirkstoffkombinationen A, worin das Strobilurin der Formel (II) (Gruppe 2) aus der folgenden Liste ausgewählt ist:

(2-1) Azoxystrobin

(2-2) Fluoxastrobin

(2-3) (2*E*)-2-(2-{[6-(3-Chlor-2-methylphenoxy)-5-fluor-4-pyrimidinyl]oxy}phenyl)-2-(methoxyimino)-*N*-methylethanamid

(2-4) Trifloxystrobin

(2-5) (2*E*)-2-(Methoxyimino)-*N*-methyl-2-(2-{[({(1*E*)-1-[3-(trifluormethyl)phenyl]ethyliden}-amino)oxy]methyl}phenyl)ethanamid

(2-6) (2*E*)-2-(Methoxyimino)-*N*-methyl-2-{2-[(*E*)-({1-[3-(trifluormethyl)phenyl]ethoxy}imino)-methyl]phenyl}ethanamid

(2-7) Orysastrobin

(2-8) 5-Methoxy-2-methyl-4-(2-{[({(1*E*)-1-[3-(trifluormethyl)phenyl]ethyliden}amino)oxy]-methyl}phenyl)-2,4-dihydro-3*H*-1,2,4-triazol-3-on

(2-9) Kresoxim-methyl

(2-10) Dimoxystrobin

(2-11) Picoxystrobin

(2-12) Pyraclostrobin

(2-13) Metominostrobin

Ganz besonders bevorzugt sind Wirkstoffkombinationen A, worin das Strobilurin der Formel (II) (Gruppe 2) aus der folgenden Liste ausgewählt ist:

(2-1) Azoxystrobin

(2-2) Fluoxastrobin

(2-3) (2*E*)-2-(2-{[6-(3-Chlor-2-methylphenoxy)-5-fluor-4-pyrimidinyl]oxy}phenyl)-2-(methoxyimino)-*N*-methylethanamid

(2-4) Trifloxystrobin

(2-12) Pyraclostrobin

(2-9) Kresoxim-methyl

(2-10) Dimoxystrobin

(2-11) Picoxystrobin

(2-13) Metominostrobin

Hervorgehoben sind die in der folgenden Tabelle 1 angeführten Wirkstoffkombinationen A:

**Tabelle 1: Wirkstoffkombinationen A**

| Nr. | Carboxamid der Formel (I) | Strobilurin der Formel (II) |
|---|---|---|
| A-1 | (1-1) N-(3',4'-Dichlor-5-fluor-1,1'-biphenyl-2-yl)-3-(difluormethyl)-1-methyl-1H-pyrazol-4-carboxamid | (2-1) Azoxystrobin |
| A-2 | (1-1) N-(3',4'-Dichlor-5-fluor-1,1'-biphenyl-2-yl)-3-(difluormethyl)-1-methyl-1H-pyrazol-4-carboxamid | (2-2) Fluoxastrobin |
| A-3 | (1-1) N-(3',4'-Dichlor-5-fluor-1,1'-biphenyl-2-yl)-3-(difluormethyl)-1-methyl-1H-pyrazol-4-carboxamid | (2-3) (2*E*)-2-(2-{[6-(3-Chlor-2-methylphenoxy)-5-fluor-4-pyri-midinyl]oxy}phenyl)-2-(meth-oxyimino)-*N*-methylethanamid |
| A-4 | (1-1) N-(3',4'-Dichlor-5-fluor-1,1'-biphenyl-2-yl)-3-(difluormethyl)-1-methyl-1H-pyrazol-4-carboxamid | (2-4) Trifloxystrobin |
| A-5 | (1-1) N-(3',4'-Dichlor-5-fluor-1,1'-biphenyl-2-yl)-3-(difluormethyl)-1-methyl-1H-pyrazol-4-carboxamid | (2-12) Pyraclostrobin |
| A-6 | (1-2) 3-(Difluormethyl)-N-{3'-fluor-4'-[(*E*)-(meth-oxyimino)methyl]-1,1'-biphenyl-2-yl}-1-methyl-1H-pyrazol-4-carboxamid | (2-1) Azoxystrobin |
| A-7 | (1-2) 3-(Difluormethyl)-N-{3'-fluor-4'-[(E)-(meth-oxyimino)methyl]-1,1'-biphenyl-2-yl}-1-methyl-1H-pyrazol-4-carboxamid | (2-2) Fluoxastrobin |

(fortgesetzt)

| Nr. | Carboxamid der Formel (I) | Strobilurin der Formel (II) |
|---|---|---|
| A-8 | (1-2) 3-(Difluormethyl)-N-{3'-fluor-4'-[(E)-(meth-oxyimino)methyl]-1,1'-biphenyl-2-yl}-1-methyl-1H-pyrazol-4-carboxamid | (2-3) (2*E*)-2-(2-{[6-(3-Chlor-2-methylphenoxy)-5-fluor-4-pyri-midmyl]oxy}phenyl)-2-(meth-oxyimmo)-*N*-methylethanamid |
| A-9 | (1-2) 3-(Difluormethyl)-N-{3'-fluor-4'-[(E)-(meth-oxyimino)methyl]-1,1'-biphenyl-2-yl}-1-methyl-1H-pyrazol-4-carboxamid | (2-4) Trifloxystrobin |
| A-10 | (1-2) 3-(Difluormethyl)-N-{3'-fluor-4'-[(E)-(meth-oxyimino)methyl]-1,1'-biphenyl-2-yl}-1-methyl-1H-pyrazol-4-carboxamid | (2-12) Pyraclostrobin |
| A-11 | (1-3) 3-(Trifluormethyl)-N-{3'-fluor-4'-[(E)-(meth-oxyimino)methyl]-1,1'-biphenyl-2-yl}-1-methyl-1H-pyrazol-4-carboxamid | (2-1) Azoxystrobin |
| A-12 | (1-3) 3-(Trifluormethyl)-N-{3'-fluor-4'-[(E)-(meth-oxyimino)methyl]-1,1'-biphenyl-2-yl}-1-methyl-1H-pyrazol-4-carboxamid | (2-2) Fluoxastrobin |
| A-13 | (1-3) 3-(Trifluormethyl)-N-{3'-fluor-4'-[(E)-(meth-oxyimino)methyl]-1,1-biphenyl-2-yl}-1-methyl-1H-pyrazol-4-carboxamid | (2-3) (2*E*)-2-(2-{[6-(3-Chlor-2-methylphenoxy)-5-fluor-4-pyri-midinyl]oxy}phenyl)-2-(meth-oxyimino)-*N*-methylethanamid |
| A-14 | (1-3) 3-(Trifluormethyl)-N-{3'-fluor-4'-[(E)-(meth-oxyimino)methyl]-1,1'-biphenyl-2-yl}-1-methyl-1H-pyrazol-4-carboxamid | (2-4) Trifloxystrobin |
| A-15 | (1-3) 3-(Trifluormethyl)-N-{3'-fluor-4'-[(E)-(meth-oxyimino)methyl]-1,1'-biphenyl-2-yl}-1-methyl-1H-pyrazol-4-carboxamid | (2-12) Pyraclostrobin |
| A-16 | (1-4) N-(3',4'-Dichlor-1,1'-biphenyl-2-yl)-5-fluor-1,3dimethyl-1H-pyrazol-4-carboxamid | (2-1) Azoxystrobin |
| A-17 | (1-4) N-(3',4'-Dichlor-1,1'-biphenyl-2-yl)-5-fluor-1,3-dimethyl-1H-pyrazol-4-carboxamid | (2-2) Fluoxastrobin |
| A-18 | (1-4) N-(3',4'-Dichlor-1,1'-biphenyl-2-yl)-5-fluor-1,3-dimethyl-1H-pyrazol-4-carboxamid | 2-3) (2*E*)-2-(2-{[6-(3-Chlor-2-methylphenoxy)-5-fluor-4-pyri-midinyl]oxy}phenyl)-2-(meth-oxyimino)-*N*-methylethanamid |
| A-19 | (1-4) N-(3',4'-Dichlor-1,1'-biphenyl-2-yl)-5-fluor-1,3-dimethyl-1H-pyrazol-4-carboxamid | (2-4) Trifloxystrobin |
| A-20 | (1-4) N-(3',4'-Dichlor-1,1'-biphenyl-2-yl)-5-fluor-1,3-dimethyl-1H-pyrazol-4-carboxamid | (2-12) Pyraclostrobin |
| A-21 | (1-5) N-(4'-Chlor-3'-fluor-1,1'-biphenyl-2-yl)-2-methyl-4-(trifluormethyl)-1,3-thiazol-5-carboxamid | (2-1) Azoxystrobin |
| A-22 | (1-5) N-(4'-Chlor-3'-fluor-1,1'-biphenyl-2-yl)-2-methyl-4-(trifluormethyl)-1,3-thiazol-5-carboxamid | (2-2) Fluoxastrobin |
| A-23 | (1-5) N-(4'-Chlor-3'-fluor-1,1'-biphenyl-2-yl)-2-methyl-4-(trifluormethyl)-1,3-thiazol-5-carboxamid | (2-3) (2*E*)-2-(2-{[6-(3-Chlor-2-methylphenoxy)-5-fluor-4-pyri-midinyl]oxy}phenyl)-2-(meth-oxyimino)-*N*-methylethanamid |
| A-24 | (1-5) N-(4'-Chlor-3'-fluor-1,1'-biphenyl-2-yl)-2-methyl-4-(trifluormethyl)-1,3-thiazol-5-carboxamid | (2-4) Trifloxystrobin |

(fortgesetzt)

| Nr. | Carboxamid der Formel (I) | Strobilurin der Formel (II) |
|---|---|---|
| A-25 | (1-5) N-(4'-Chlor-3'-fluor-1,1'-biphenyl-2-yl)-2-methyl-4-(trifluormethyl)-1,3-thiazol-5-carboxamid | (2-12) Pyraclostrobin |
| A-26 | (1-6) N-(4'-Chlor-1,1'-biphenyl-2-yl)-4-(difluor-(2-1) Azoxystrobin methyl)-2-methyl-1,3-thiazol-5-carboxamid | |
| A-27 | (1-6) N-(4'-Chlor-1,1'-biphenyl-2-yl)-4-(difluor-(2-2) methyl)-2-methyl-1,3-thiazol-5-carboxamid | Fluoxastrobin |
| A-28 | (1-6) N-(4'-Chlor-1,1'-biphenyl-2-yl)-4-(difluor-methyl)-2-methyl-1,3-thiazol-5-carboxamid | (2-3) (2*E*)-2-(2-{[6-(3-Chlor-2-methylphenoxy)-5-fluor-4-pyri-midinyl]oxy}phenyl)-2-(meth-oxyimino)-*N*-methylethanamid |
| A-29 | (1-6) N-(4'-Chlor-1,1'-biphenyl-2-yl)-4-(difluor-(2-4) Trifloxystrobin methyl)-2-methyl-1,3-thiazol-5-carboxamid | |
| A-30 | (1-6) N-(4'-Chlor-1,1'-biphenyl-2-yl)-4-(difluor-(2-12) Pyraclostrobin methyl)-2-methyl-1,3-thiazol-5-carboxamid | |
| A-31 | (1-7) N-(4'-Brom-1,1'-biphenyl-2-yl)-4-(difluor-(2-1) Azoxystrobin methyl)-2-methyl-1,3-thiazol-5-carboxamid | |
| A-32 | (1-7) N-(4'-Brom-1,1'-biphenyl-2-yl)-4-(difluor-(2-2) methyl)-2-methyl-1,3-thiazol-5-carboxamid | Fluoxastrobin |
| A-33 | (1-7) N-(4'-Brom-1,1'-biphenyl-2-yl)-4-(difluor-methyl)-2-methyl-1,3-thiazol-5-carboxamid | (2-3) (2*E*)-2-(2-{[6-(3-Chlor-2-methylphenoxy)-5-fluor-4-pyri-midinyl]oxy}phenyl)-2-(meth-oxyimino)-*N*-methylethanamid |
| A-34 | (1-7) N-(4'-Brom-1,1'-biphenyl-2-yl)-4-(difluor-(2-4) Trifloxystrobin methyl)-2-methyl-1,3-thiazol-5-carboxamid | |
| A-35 | (1-7) N-(4'-Brom-1,1'-biphenyl-2-yl)-4-(difluor-(2-12) Pyraclostrobin methyl)-2-methyl-1,3-thiazol-5-carboxamid | |
| A-36 | (1-8) 4-(Difluormethyl)-2-methyl-N-[4'-(trifluorme-thyl)-1,1'-biphenyl-2-yl]-1,3-thiazol-5-carboxamid | (2-1) Azoxystrobin |
| A-37 | (1-8) 4-(Difluormethyl)-2-methyl-N-[4'-(trifluorme-thyl)-1,1'-biphenyl-2-yl]-1,3-thiazol-5-carboxamid | (2-2) Fluoxastrobin |
| A-38 | (1-8) 4-(Difluormethyl)-2-methyl-N-[4'-(trifluorme-thyl)-1,1'-biphenyl-2-yl]-1,3-thiazol-5-carboxamid | (2-3) (2*E*)-2-(2-{[6-(3-Chlor-2-methylphenoxy)-5-fluor-4-pyri-midinyl]oxy}phenyl)-2-(meth-oxyimino)-*N*-methylethanamid |
| A-39 | (1-8) 4-(Difluormethyl)-2-methyl-N-[4'-(trifluorme-thyl)-1,1-biphenyl-2-yl]-1,3-thiazol-5-carboxamid | (2-4) Trifloxystrobin |
| A-40 | (1-8) 4-(Difluormethyl)-2-methyl-N-[4'-(trifluorme-thyl)-1,1'-biphenyl-2-yl]-1,3-thiazol-5-carboxamid | (2-12) Pyraclostrobin |
| A-41 | (1-1) N-(3',4'-Dichlor-5-fluor-1,1'-biphenyl-2-yl)-3- (difluormethyl)-1-methyl-1H-pyrazol-4-carboxamid | (2-9) Kresoxim-methyl |

(fortgesetzt)

| Nr. | Carboxamid der Formel (I) | Strobilurin der Formel (II) |
|---|---|---|
| A-42 | (1-1) N-(3',4'-Dichlor-5-fluor-1,1'-biphenyl-2-yl)-3-(difluormethyl)-1-methyl-1H-pyrazol-4-carboxamid | (2-10) Dimoxystrobin |
| A-43 | (1-1) N-(3',4'-Dichlor-5-fluor-1,1'-biphenyl-2-yl)-3-(difluormethyl)-1-methyl-1H-pyrazol-4-carboxamid | (2-11) Picoxystrobin |
| A-44 | (1-1) N-(3',4'-Dichlor-5-fluor-1,1'-biphenyl-2-yl)-3-(difluormethyl)-1-methyl-1H-pyrazol-4-carboxamid | (2-13) Metominostrobin |
| A-45 | (1-7) N-(4'-Brom-1,1'-biphenyl-2-yl)-4-(difluormethyl)-2-methyl-1,3-thiazol-5-carboxamid | (2-9) Kresoxim-methyl |
| A-46 | (1-7) N-(4'-Brom-1,1'-biphenyl-2-yl)-4-(difluormethyl)-2-methyl-1,3-thiazol-5-carboxamid | (2-10) Dimoxystrobin |
| A-47 | (1-7)N-(4'-Brom-1,1'-biphenyl-2-yl)-4-(difluormethyl)-2-methyl-1,3-thiazol-5-carboxamid | (2-11) Picoxystrobin |
| A-48 | (1-7) N-(4'-Brom-1,1'-biphenyl-2-yl)-4-(difluormethyl)-2-methyl-1,3-thiazol-5-carboxamid | (2-13) Metominostrobin |
| A-49 | (1-8) 4-(Difluormethyl)-2-methyl-N-[4'-(trifluormethyl)-1,1'-biphenyl-2-yl]-1,3-thiazol-5-carboxamid | (2-9) Kesoxim-methyl |
| A-50 | (1-8) 4-(Difluormethyl)-2-methyl-N-[4'-(trifluormethyl)-1,1'-biphenyl-2-yl]-1,3-thiazol-5-carboxamid | (2-10) Dimoxystrobin |
| A-51 | (1-8) 4-(Difluormethyl)-2-methyl-N-[4'-(trifluormethyl)-1,1'-biphenyl-2-yl]-1,3-thiazol-5-carboxamid | (2-11) Picoxystrobin |
| A-52 | (1-8) 4-(Difluormethyl)-2-methyl-N-[4'-(trifluormethyl)-1,1'-biphenyl-2-yl]-1,3-thiazol-5-carboxamid | (2-13) Metominostrobin |
| A-53 | (1-9) *N*-(4'-Chlor-3'-fluor-1,1'-biphenyl-2-yl)-4-(difluormethyl)-2-methyl-1,3-thiazol-5-carboxamid | (2-9) Kresoxim-methyl |
| A-54 | (1-9) *N*-(4'-Chlor-3'-fluor-1,1'-biphenyl-2-yl)-4-(difluormethyl)-2-methyl-1,3-thiazol-5-carboxamid | (2-10) Dimoxystrobin |
| A-55 | (1-9) *N*-(4'-Chlor-3'-fluor-1,1'-biphenyl-2-yl)-4-(difluormethyl)-2-methyl-1,3-thiazol-5-carboxamid | (2-11) Picoxystrobin |
| A-56 | (1-9) *N*-(4'-Chlor-3'-fluor-1,1'-biphenyl-2-yl)-4-(difluormethyl)-2-methyl-1,3-thiazol-5-carboxamid | (2-13) Metominostrobin |
| A-57 | (1-9) *N*-(4'-Chlor-3'-fluor-1,1'-biphenyl-2-yl)-4-(difluormethyl)-2-methyl-1,3-thiazol-5-carboxamid | (2-1) Azoxystrobin |
| A-58 | (1-9) *N*-(4'-Chlor-3'-fluor-1,1'-biphenyl-2-yl)-4-(difluormethyl)-2-methyl-1,3-thiazol-5-carboxamid | (2-2) Fluoxastrobin |
| A-59 | (1-9) *N*-(4'-Chlor-3'-fluor-1,1'-biphenyl-2-yl)-4-(difluormethyl)-2-methyl-1,3-thiazol-5-carboxamid | (2-3) (2*E*)-2-(2-{[6-(3-Chlor-2-methylphenoxy)-5-fluor-4-pyri-midinyl]oxy}phenyl)-2-(methoxyimino)-*N*-methylethanamid |
| A-60 | (1-9) *N*-(4'-Chlor-3'-fluor-1,1'-biphenyl-2-yl)-4-(difluormethyl)-2-methyl-1,3-thiazol-5-carboxamid | (2-4) Trifloxystrobin |
| A-61 | (1-9) *N*-(4'-Chlor-3'-fluor-1,1'-biphenyl-2-yl)-4-(difluormethyl)-2-methyl-1,3-thiazol-5-carboxamid | (2-12) Pyraclostrobin |

Die Wirkstoffkombinationen B enthalten neben einem Carboxamid der Formel (I) (Gruppe 1) auch ein Triazol der Formel (III) (Gruppe 3)

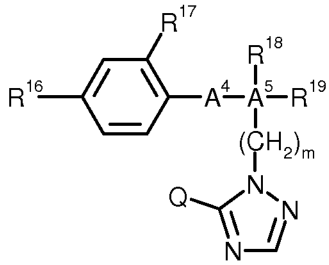

(III)

in welcher Q, m, $R^{16}$, $R^{17}$, $A^4$, $A^5$, $R^{18}$ und $R^{19}$ die oben angegebenen Bedeutungen haben.

Bevorzugt sind Wirkstoffkombinationen B, worin das Triazol der Formel (III) (Gruppe 3) aus der folgenden Liste ausgewählt ist:

(3-1) Azaconazole
(3-2) Etaconazole
(3-3) Propiconazole
(3-4) Difenoconazole
(3-5) Bromuconazole
(3-6) Cyproconazole
(3-7) Hexaconazole
(3-8) Penconazole
(3-9) Myclobutanil
(3-10) Tetraconazole
(3-11) Flutriafol
(3-12) Epoxiconazole
(3-13) Flusilazole
(3-14) Simeconazole
(3-15) Prothioconazole
(3-16) Fenbuconazole
(3-17) Tebuconazole
(3-18) Ipconazole
(3-19) Metconazole
(3-20) Triticonazole
(3-21) Bitertanol
(3-22) Triadimenol
(3-23) Triadimefon
(3-24) Fluquinconazole
(3-25) Quinconazole

Besonders bevorzugt sind Wirkstoffkombinationen B, worin das Triazol der Formel (III) (Gruppe 3) aus der folgenden Liste ausgewählt ist:

(3-3) Propiconazole

(3-6) Cyproconazole

(3-15) Prothioconazole

(3-17) Tebuconazole

(3-21) Bitertanol

(3-4) Difenoconazole

(3-7) Hexaconazole

(3-19) Metconazole

(3-22) Triadimenol

(3-24) Fluquinconazole

Hervorgehoben sind die in der folgenden Tabelle 2 angeführten Wirkstoffkombinationen B:

**Tabelle 2: Wirkstoffkombinationen B**

| Nr. | Carboxamid der Formel (I) | Azol der Formel (III) |
|---|---|---|
| B-1 | (1-1) N-(3',4'-Dichlor-5-fluor-1,1'-biphenyl-2-yl)-3-(difluormethyl)-1-methyl-1H-pyrazol-4-carboxamid | (3-3) Propiconazole |
| B-2 | (1-1) N-(3',4'-Dichlor-5-fluor-1,1'-biphenyl-2-yl)-3 (difluormethyl)-1-methyl-1H-pyrazol-4-carboxamid | (3-6) Ciprocomazole |
| B-3 | (1-1) N-(3',4'-Dichlor-5-fluor-1,1'-biphenyl-2-yl)-3-(difluormethyl)-1-methyl-1H-pyrazol-4-carboxamid | (3-15) Prothioconazole |
| B-4 | (1-1) N-(3',4'-Dichlor-5-fluor-1,1'-biphenyl-2-yl)-3-(difluormethyl)-1-methyl-1H-pyrazol-4-carboxamid | (3-17) Tebuconazole |
| B-5 | (1-1) N-(3',4'-Dichlor-5-fluor-1,1'-biphenyl-2-yl)-3-(difluormethyl)-1-methyl-1H-pyrazol-4-carboxamid | (3-21) Bitertanol |
| B-6 | (1-2) 3-(Difluormethyl)-N-{3'-fluor-4'-[(E)-(methoxyimino)me-thyl]-1,1'-biphenyl-2-yl}-1-methyl-1H-pyrazol-4-carboxamid | (3-3) Propiconazole |
| B-7 | (1-2) 3-(Difluormethyl)-N-{3'-fluor-4'-[(E)-(methoxyimino)-methyl]-1,1'-biphenyl-2-yl}-1-methyl-1H-pyrazol-4-carboxamid | (3-6) Cyproconazol |
| B-8 | (1-2) 3-(Difluormethyl)-N-{3'-fluor-4'-[(E)-(methoxyimino)-methyl]-1,1'-biphenyl-2-yl}-1-methyl-1H-pyrazol-4-carboxamid | (3-15) Prothioconazole |
| B-9 | (1-2) 3-(Difluormethyl)-N-{3'-fluor-4'-[(E)-(methoxyimino)-methyl]-1,1'-biphenyl-2-yl}-1-methyl-1H-pyrazol-4-carboxamid | (3-17) Tebuconazole |
| B-10 | (1-2) 3-(Difluormethyl)-N-{3'-fluor-4'-[(E)-(methoxyimino)-methyl]-1,1'-biphenyl-2-yl}-1-methyl-1H-pyrazol-4-carboxamid | (3-21) Bitertanol |
| B-11 | (1-3) 3-(Trifluormethyl)-N-{3'-fluor-4'-[(E)-(methoxyimino)-methyl]-1,1'-biphenyl-2-yl}-1-methyl-1H-pyrazol-4-carboxamid | (3-3) Propiconazole |
| B-12 | (1-3) 3-(Trifluormethyl)-N-{3'-fluor-4'-[(E)-(methoxyimino)-methyl]-1,1'-biphenyl-2-yl}-1-methyl-1H-pyrazol-4-carboxamid | (3-6) Cyproconazole |
| B-13 | (1-3) 3-(Trifluormethyl)-N-{3'-fuor-4'-[(E)-(methoxyimino)-methyl]-1,1'-biphenyl-2-yl}-1-methyl-1H-pyrazol-4-carboxamid | (3-15) Prothioconazole |
| B-14 | (1-3) 3-(Trifluormethyl)-N-{3'-fluor-4'-[(E)-(methoxyimino)-methyl]-1,1'-biphenyl-2-yl}-1-methyl-1H-pyrazol-4-carboxamid | (3-17) Tebuconazol |
| B-15 | (1-3) 3-(Trifluormethyl)-N-{3'-fluor-4'-[(E)-(methoxyimino)-methyl]-1,1'-biphenyl-2-yl}-1-methyl-1H-pyrazol-4-carboxamid | (3-21) Bitertanol |
| B-16 | (1-4) N-(3',4'-Dichlor-1,1'-biphenyl-2-yl)-5-fluor-1,3-dimethyl-1H-pyrazol-4-carboxamid | (3-3) Propiconazole |
| B-17 | (1-4) N-(3',4'-Dichlor-1,1'-biphenyl-2-yl)-5-fluor-1,3-dimethyl-1H-pyrazol-4-carboxamid | (3-6) Cyproconazole |
| B-18 | (1-4) N-(3',4'-Dichlor-1,1'-biphenyl-2-yl)-5-fluor-1,3-dimethyl-1H-pyrazol-4-carboxamid | (3-15) Prothioconazole |

(fortgesetzt)

| Nr. | Carboxamid der Formel (I) | Azol der Formel (III) |
|---|---|---|
| B-19 | (1-4) N-(3',4'-Dichlor-1,1'-biphenyl-2-yl)-5-fluor-1,3-dimethyl-1H-pyrazol-4-carboxamid | (3-17) Tebuconazole |
| B-20 | (1-4) N-(3',4'-Dichlor-1,1'-biphenyl-2-yl)-5-fluor-1,3-dimethyl-1H-pyrazol-4-carboxamid | (3-21) Bitertanol |
| B-21 | (1-5) N-(4'-Chlor-3'-fluor-1,1'-biphenyl-2-yl)-2-methyl-4-(trifluormethyl)-1,3-thiazol-5-carboxamid | (3-3) Propiconazole |
| B-22 | (1-5) N-(4'-Chlor-3'-fluor-1,1'-biphenyl-2-yl)-2-methyl-4-(trifluormethyl)-1,3-thiazol-5-carboxamid | (3-6) Cyproconazole |
| B-23 | (1-5) N-(4'-Chlor-3'-fluor-1,1'-biphenyl-2-yl)-2-methyl-4-(trifluormethyl)-1,3-thiazol-5-carboxamid | (3-15) Prothioconazole |
| B-24 | (1-5) N-(4'-Chlor-3'-fluor-1,1'-biphenyl-2-yl)-2-methyl-4-(trifluormethyl)-1,3-thiazol-5-carboxamid | (3-17) Tebuconazole |
| B-25 | (1-5) N-(4'-Chlor-3'-fluor-1,1'-biphenyl-2-yl)-2-methyl-4-(trifluormethyl)-1,3-thiazol-5-carboxamid | (3-21) Bitertanol |
| B-26 | (1-6) N-(4'-Chlor-1,1'-biphenyl-2-yl)-4-(difluormethyl)-2-methyl-1,3-thiazol-5-carboxamid | (3-3) Propiconazole |
| B-27 | (1-6) N-(4'-Chlor-1,1'-biphenyl-2-yl)-4-(difluormethyl)-2-methyl-1,3-thiazol-5-carboxamid | (3-6) Cyproconazole |
| B-28 | (1-6) N-(4'-Chlor-1,1'-biphenyl-2-yl)-4-(difluormethyl)-2-methyl-1,3-thiazol-5-carboxamid | (3-15) Prothioconazole |
| B-29 | (1-6) N-(4'-Chlor-1,1'-biphenyl-2-yl)-4-(difluormethyl)-2-methyl-1,3-thiazol-5-carboxamid | (3-17) Tebuconazole |
| B-30 | (1-6) N-(4'-Chlor-1,1'-biphenyl-2-yl)-4-(difluormethyl)-2-methyl-1,3-thiazol-5-carboxamid | (3-21) Bitertanol |
| B-31 | (1-7) N-(4'-Brom-1,1'-biphenyl-2-yl)-4-(difluormethyl)-2-methyl-1,3-thiazol-5-carboxamid | (3-3) Propiconazole |
| B-32 | (1-7) N-(4'-Brom-1,1'-biphenyl-2-yl)-4-(difluormethyl)-2-methyl-1,3-thiazol-5-carboxamid | (3-6) Cyproconazole |
| B-33 | (1-7) N-(4'-Brom-1,1'-biphenyl-2-yl)-4-(difluormethyl)-2-methyl-1,3-thiazol-5-carboxamid | (3-15) Prothioconazole |
| B-34 | (1-7) N-(4'-Brom-1,1'-biphenyl-2-yl)-4-(difluormethyl)-2-methyl-1,3-thiazol-5-carboxamid | (3-17) Tebuconazole |
| B-35 | (1-7) N-(4'-Brom-1,1'-biphenyl-2-yl)-4-(difluormethyl)-2-methyl-1,3-thiazol-5-carboxamid | (3-21) Bitertanol |
| B-36 | (1-8) 4-(Difluormethyl)-2-methyl-N-[4'-(trifluormethyl)-1,1'-biphenyl-2-yl]-1,3-thiazol-5-carboxamid | (3-3) Propiconazole |
| B-37 | (1-8) 4-(Difluormethyl)-2-methyl-N-[4'-(trifluormethyl)-1,1'-biphenyl-2-yl]-1,3-thiazol-5-carboxamid | (3-6) Cyproconazole |
| B-38 | (1-8) 4-(Difluormethyl)-2-methyl-N-[4'-(trifluormethyl)-1,1'-biphenyl-2-yl]-1,3-thiazol-5-carboxamid | (3-15) Prothioconazole |
| B-39 | (1-8) 4-(Difluormethyl)-2-methyl-N-[4'-(tnfluormethyl)-1,1-biphenyl-2-yl]-1,3-thiazol-5-carboxamid | (3-17) Tebuconazole |
| B-40 | (1-8) 4-(Difluormethyl)-2-methyl-N-[4'-(trifluormethyl)-1,1'-biphenyl-2-yl]-1,3-thiazol-5-carboxamid | (3-21) Bitertanol |

(fortgesetzt)

| Nr. | Carboxamid der Formel (I) | Azol der Formel (III) |
|---|---|---|
| B-41 | (1-1) N-(3',4'-Dichlor-5-fluor-1,1'-biphenyl-2-yl)-3-(difluormethyl)-1-methyl-1H-pyrazol-4-carboxamid | (3-4) Difenoconazole |
| B-42 | (1-1) N-(3',4'-Dichlor-5-fluor-1,1'-biphenyl-2-yl)-3-(difluormethyl)-1-methyl-1H-pyrazol-4-carboxamid | (3-7) Hexaconazole |
| B-43 | (1-1) N-(3',4'-Dichlor-5-fluor-1,1'-biphenyl-2-yl)-3-(difluormethyl)-1-methyl-1H-pyrazol-4-carboxamid | (3-19) Metconazole |
| B-44 | (1-1) N-(3',4'-Dichlor-5-fluor-1,1'-biphenyl-2-yl)-3-(difluormethyl)-1-methyl-1H-pyrazol-4-carboxamid | (3-22) Triadimenol |
| B-45 | (1-1) N-(3',4'-Dichlor-5-fluor-1,1'-biphenyl-2-yl)-3-(difluormethyl)-1-methyl-1H-pyrazol-4-carboxamid | (3-24) Fluquinconazole |
| B-46 | (1-7) N-(4'-Brom-1,1'-biphenyl-2-yl)-4-(difluormethyl)-2-methyl-1,3-thiazol-5-carboxamid | (3-4) Difenoconazole |
| B-47 | (1-7) N-(4'-Brom-1,1'-biphenyl-2-yl)-4-(difluormethyl)-2-methyl-1,3-thiazol-5-carboxamid | (3-7) Hexaconazole |
| B-48 | (1-7) N-(4'-Brom-1,1'-biphenyl-2-yl)-4-(difluormethyl)-2-methyl-1,3-thiazol-5-carboxamid | (3-19) Metconazole |
| B-49 | (1-7) N-(4'-Brom-1,1'-biphenyl-2-yl)-4-(difluormethyl)-2-methyl-1,3-thiazol-5-carboxamid | (3-22) Triadimenol |
| B-50 | (1-7) N-(4'-Brom-1,1'-biphenyl-2-yl)-4-(difluormethyl)-2-methyl-1,3-thiazol-5-carboxamid | (3-24) Fluquinconazole |
| B-51 | (1-8) 4-(Difluormethyl)-2-methyl-N-[4'-(trifluormethyl)-1,1'-biphenyl-2-yl]-1,3-thiazol-5-carboxamid | (3-4) Difenoconazole |
| B-52 | (1-8) 4-(Difluormethyl)-2-methyl-N-[4'-(trifluormethyl)-1,1'- biphenyl-2-yl]-1,3-thiazol-5-carboxamid | (3-7) Hexaconazole |
| B-53 | (1-8) 4-(Difluormethyl)-2-methyl-N-[4'-(trifluormethyl)-1,1'-biphenyl-2-yl]-1,3-thiazol-5-carboxamid | (3-19) Metconazole |
| B-54 | (1-8) 4-(Difluormethyl)-2-methyl-N-[4'-(trifluormethyl)-1,1'-biphenyl-2-yl]-1,3-thiazol-5-carboxamid | (3-22) Triadimenol |
| B-55 | (1-8) 4-(Difluormethyl)-2-methyl-N-[4'-(trifluormethyl)-1,1'-biphenyl-2-yl]-1,3-thiazol-5-carboxamid | (3-24) Fluquinconazole |
| B-56 | (1-9) *N*-(4'-Chlor-3'-fluor-1,1'-biphenyl-2-yl)-4-(difluormethyl)-2-methyl-1,3-thiazol-5-carboxamid | (3-4) Difenoconazole |
| B-57 | (1-9) *N*-(4'-Chlor-3'-fluor-1,1'-biphenyl-2-yl)-4-(difluor-methyl)-2-methyl-1,3-thiazol-5-carboxamid | (3-7) Hexaconazole |
| B-58 | (1-9) *N*-(4'-Chlor-3'-fluor-1,1'-biphenyl-2-yl)-4-(difluor-methyl)-2-methyl-1,3-thiazol-5-carboxamid | (3-19) Metconazole |
| B-59 | (1-9) *N*-(4'-Chlor-3'-fluor-1,1'-biphenyl-2-yl)-4-(difluor-methyl)-2-methyl-1,3-thiazol-5-carboxamid | (3-22) Triadimenol |
| B-60 | (1-9) *N*-(4'-Chlor-3'-fluor-1,1'-biphenyl-2-yl)-4-(difluor-methyl)-2-methyl-1,3-thiazol-5-carboxamid | (3-24) Fluquinconazole |
| B-61 | (1-9) *N*-(4'-Chlor-3'-fluor-1,1'-biphenyl-2-yl)-4-(difluor-methyl)-2-methyl-1,3-thiazol-5-carboxamid | (3-3) Propiconazole |
| B-62 | (1-9) *N*-(4'-Chlor-3'-fluor-1,1'-biphenyl-2-yl)-4-(difluor-methyl)-2-methyl-1,3-thiazol-5-carboxamid | (3-6) Cyproconazole |

(fortgesetzt)

| Nr. | Carboxamid der Formel (I) | Azol der Formel (III) |
|---|---|---|
| B-63 | (1-9) *N*-(4'-Chlor-3'-fluor-1,1'-biphenyl-2-yl)-4-(difluor-methyl)-2-methyl-1,3-thiazol-5-carboxamid | (3-15) Prothioconazole |
| B-64 | (1-9) *N*-(4'-Chlor-3'-fluor-1,1'-biphenyl-2-yl)-4-(difluor-methyl)-2-methyl-1,3-thiazol-5-carboxamid | (3-17) Tebuconazole |
| B-65 | (1-9) *N*-(4'-Chlor-3'-fluor-1,1'-biphenyl-2-yl)-4-(difluor-methyl)-2-methyl-1,3-thiazol-5-carboxamid | (3-21) Bitertanol |

Die Wirkstoffkombinationen C enthalten neben einem Carboxamid der Formel (I) (Gruppe 1) auch ein Sulfenamid der Formel (IV) (Gruppe 4)

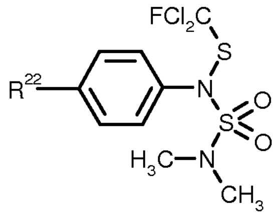

(IV)

in welcher $R^{22}$ die oben angegebenen Bedeutungen hat.

Bevorzugt sind Wirkstoffkombinationen C, worin das Sulfenamid der Formel (IV) (Gruppe 4) aus der folgenden Liste ausgewählt ist:

(4-1) Dichlofluanid

(4-2) Tolylfluanid

Hervorgehoben sind die in der folgenden Tabelle 3 angeführten Wirkstoffkombinationen C:

**Tabelle 3: Wirkstoffkombinationen C**

| Nr. | Carboxamid der Formel (I) | Sulfenamid der Formel (IV) |
|---|---|---|
| C-1 | (1-1) N-(3',4'-Dichlor-5-fluor-1,1'-biphenyl-2-yl)-3-(difluormethyl)-1-methyl-1H-pyrazol-4-carboxamid | (4-1) Dichlofluanid |
| C-2 | (1-1) N-(3',4'-Dichlor-5-fluor-1,1'-biphenyl-2-yl)-3-(difluormethyl)-1-methyl-1H-pyrazol-4-carboxamid | (4-2) Tolylfluanid |
| C-3 | (1-7) N-(4'-Brom-1,1'-biphenyl-2-yl)-4-(difluormethyl)-2-methyl-1,3-thiazol-5-carboxamid | (4-1) Dichlofluanid |
| C-4 | (1-7) N-(4'-Brom-1,1'-biphenyl-2-yl)-4-(difluormethyl)-2-methyl-1,3-thiazol-5-carboxamid | (4-2) Tolylfluanid |
| C-5 | (1-8) 4-(Difluormethyl)-2-methyl-N-[4'-(trifluormethyl)-1,1'-biphenyl-2-yl]-1,3-thiazol-5-carboxamid | (4-1) Dichlofluanid |
| C-6 | (1-8) 4-(Difluormethyl)-2-methyl-N-[4'-(trifluormethyl)-1,1'-biphenyl-2-yl]-1,3-thiazol-5-carboxamid | (4-2) Tolylfluanid |
| C-7 | (1-9) *N*-(4'-Chlor-3'-fluor-1,1'-biphenyl-2-yl)-4-(difluor-methyl)-2-methyl-1,3-thiazol-5-carboxamid | (4-1) Dichlofluanid |
| C-8 | (1-9) *N*-(4'-Chlor-3'-fluor-1,1'-biphenyl-2-yl)-4-(difluor- methyl)-2-methyl-1,3-thiazol-5-carboxamid | (4-2) Tolylfluanid |

Die Wirkstoffkombinationen D enthalten neben einem Carboxamid der Formel (I) (Gruppe 1) auch ein Valinamid

(Gruppe 5) ausgewählt aus

(5-1) Iprovalicarb

(5-2) $N^1$-[2-(4-{[3-(4-chlorophenyl)-2-propynyl]oxy}-3-methoxyphenyl)ethyl]-$N^2$-(methylsulfonyl)-D-valinamid

(5-3) Benthiavalicarb

Bevorzugt sind Wirkstoffkombinationen D, worin das Valinamid (Gruppe 5) aus der folgenden Liste ausgewählt ist:

(5-1) Iprovalicarb
(5-3) Benthiavalicarb

Hervorgehoben sind die in der folgenden Tabelle 4 angeführten Wirkstoffkombinationen D:

**Tabelle 4: Wirkstoffkombinationen D**

| Nr. | Carboxamid der Formel (I) | Valinamid |
|---|---|---|
| D-1 | (1-1) N-(3',4'-Dichlor-5-fluor-1,1'-biphenyl-2-yl)-3-(difluormethyl)-1-methyl-1H-pyrazol-4-carboxamid | (5-1) Iprovalicarb |
| D-2 | (1-1) N-(3',4'-Dichlor-5-fluor-1,1'-biphenyl-2-yl)-3-(difluormethyl)-1-methyl-1H-pyrazol-4-carboxamid | (5-2) Benthiavalicarb |
| D-3 | (1-7) N-(4'-Brom-1,1'-biphenyl-2-yl)-4-(difluormethyl)-2-methyl-1,3-thiazol-5-carboxamid | (5-1) Iprovalicarb |
| D-4 | (1-7) N-(4'-Brom-1,1'-biphenyl-2-yl)-4-(difluormethyl)-2-methyl-1,3-thiazol-5-carboxamid | (5-2) Benthiavalicarb |
| D-5 | (1-8) 4-(Difluormethyl)-2-methyl-N-[4'-(trifluormethyl)-1,1'-biphenyl-2-yl]-1,3-thiazol-5-carboxamid | (5-1) Iprovalicarb |
| D-6 | (1-8) 4-(Dmuormethyl)-2-methyl-N-[4'-(trifluormethyl)-1,1'-biphenyl-2-yl]-1,3-thiazol-5-carboxamid | (5-2) Benthiavalicarb |
| D-7 | (1-9) *N*-(4'-Chlor-3'-fluor-1,1'-biphenyl-2-yl)-4-(difluormethyl)-2-methyl-1,3-thiazol-5-carboxamid | (5-1) Iprovalicarb |
| D-8 | (1-9) *N*-(4'-Chlor-3'-fluor-1,1'-biphenyl-2-yl)-4-(difluormethyl)-2-methyl-1,3-thiazol-5-carboxamid | (5-2) Benthiavalicarb |

Die Wirkstoffkombinationen E enthalten neben einem Carboxamid der Formel (I) (Gruppe 1) auch ein Carboxamid der Formel (V) (Gruppe 6)

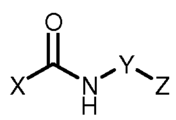

(V)

in welcher X, Y und Z die oben angegebenen Bedeutungen haben.
Bevorzugt sind Wirkstoffkombinationen E, worin das Carboxamid der Formel (V) (Gruppe 6) aus der folgenden Liste ausgewählt ist:

(6-1) 2-Chloro-N-(1,1,3-trimethyl-indan-4-yl)-nicotinamid

(6-2) Boscalid

(6-3) Furametpyr

(6-4) 1-Methyl-3-trifluormethyl-1H-pyrazol-4-carbonsäure-(3-p-tolyl-thiophen-2-yl)-amid

(6-5) Ethaboxam

(6-6) Fenhexamid

(6-7) Carpropamid

(6-8) 2-Chlor-4-(2-fluor-2-methyl-propionylamino)-N,N-dimethyl-benzamid

(6-9) Picobenzamid

(6-10) Zoxamide

(6-11) 3,4-Dichlor-N-(2-cyanophenyl)isothiazol-5-carboxamid

(6-12) Carboxin

(6-13) Tiadinil

(6-14) Penthiopyrad

(6-15) Silthiofam

(6-16) *N*-[2-(1,3-Dimethylbutyl)phenyl]-1-methyl-4-(trifluormethyl)-1*H*-pyrrol-3-carboxamid

Besonders bevorzugt sind Wirkstoffkombinationen E, worin das Carboxamid der Formel (V) (Gruppe 6) aus der folgenden Liste ausgewählt ist:

(6-2) Boscalid

(6-5) Ethaboxam

(6-6) Fenhexamid

(6-7) Carpropamid

(6-8) 2-Chlor-4-(2-fluor-2-methyl-propionylamino)-N,N-dimethyl-benzamid

(6-9) Picobenzamid

(6-10) Zoxamide

(6-11) 3,4-Dichlor-N-(2-cyanophenyl)isothiazol-5-carboxamid

(6-14) Penthiopyrad

(6-16) *N*-[2-(1,3-Dimethylbutyl)phenyl]-1-methyl-4-(trifluormethyl)-1*H*-pyrrol-3-carboxamid

Ganz besonders bevorzugt sind Wirkstoffkombinationen E, worin das Carboxamid der Formel (V) (Gruppe 6) aus der folgenden Liste ausgewählt ist:

(6-2) Boscalid

(6-6) Fenhexamid

(6-7) Carpropamid

(6-9) Picobenzamid

(6-14) Penthiopyrad

Hervorgehoben sind die in der folgenden Tabelle 5 angeführten Wirkstoffkombinationen E:

**Tabelle 5: Wirkstoffkombinationen E**

| Nr. | Carboxamid der Formel (I) | Carboxamid der Formel (V) |
|---|---|---|
| E-1 | (1-1) N-(3',4'-Dichlor-5-fluor-1,1'-biphenyl-2-yl)-3-(difluormethyl)-1-methyl-1H-pyrazol-4-carboxamid | (6-2) Boscalid |
| E-2 | (1-1) N-(3',4'-Dichlor-5-fluor-1,1'-biphenyl-2-yl)-3-(difluormethyl)-1-methyl-1H-pyrazol-4-carboxamid | (6-6) Fenhexamid |
| E-3 | (1-1) N-(3',4'-Dichlor-5-fluor-1,1'-biphenyl-2-yl)-3-(difluormethyl)-1-methyl-1H-pyrazol-4-carboxamid | (6-7) Carpropamid |
| E-4 | (1-1) N-(3',4'-Dichlor-5-fluor-1,1'-biphenyl-2-yl)-3-(difluormethyl)-1-methyl-1H-pyrazol-4-carboxamid | (6-9) Picobenzamid |
| E-5 | (1-1) N-(3',4'-Dichlor-5-fluor-1,1'-biphenyl-2-yl)-3-(difluormethyl)-1-methyl-1H-pyrazol-4-carboxamid | (6-14) Penthiopyrad |
| E-6 | (1-7) N-(4'-Brom-1,1'-biphenyl-2-yl)-4-(difluormethyl)-2-methyl-1,3-thiazol-5-carboxamid | (6-2) Boscalid |
| E-7 | (1-7) N-(4'-Brom-1,1'-biphenyl-2-yl)-4-(difluormethyl)-2-methyl-1,3-thiazol-5-carboxamid | (6-6) Fenhexamid |
| E-8 | (1-7) N-(4'-Brom-1,1'-biphenyl-2-yl)-4-(difluormethyl)-2-methyl-1,3-thiazol-5-carboxamid | (6-7) Carpropamid |
| E-9 | (1-7) N-(4'-Brom-1,1'-biphenyl-2-yl)-4-(difluormethyl)-2-methyl-1,3-thiazol-5-carboxamid | (6-9) Picobenzamid |
| E-10 | (1-7) N-(4'-Brom-1,1'-biphenyl-2-yl)-4-(difluormethyl)-2-methyl-1,3-thiazol-5-carboxamid | (6-14) Penthiopyrad |
| E-11 | (1-8) 4-(Difluormethyl)-2-methyl-N-[4'-(trifluormethyl)-1,1'-biphenyl-2-yl]-1,3-thiazol-5-carboxamid | (6-2) Boscalid |
| E-12 | (1-8) 4-(Difluormethyl)-2-methyl-N-[4'-(trifluormethyl)-1,1'-biphenyl-2-yl]-1,3-thiazol-5-carboxamid | (6-6) Fenhexamid |
| E-13 | (1-8) 4-(Difluormethyl)-2-methyl-N-[4'-(trifluormethyl)-1,1'-biphenyl-2-yl]-1,3-thiazol-5-carboxamid | (6-7) Carpropamid |
| E-14 | (1-8) 4-(Difluormethyl)-2-methyl-N-[4'-(trifluormethyl)-1,1'-biphenyl-2-yl]-1,3-thiazol-5-carboxamid | (6-9) Picobenzamid |
| E-15 | (1-8) 4-(Difluormethyl)-2-methyl-N-[4'-(trifluormethyl)-1,1'-biphenyl-2-yl]-1,3-thiazol-5-carboxamid | (6-14) Penthiopyrad |
| E-16 | (1-9) *N*-(4'-Chlor-3'-fluor-1,1'-biphenyl-2-yl)-4-(difluor-methyl)-2-methyl-1,3-thiazol-5-carboxamid | (6-2) Boscalid |
| E-17 | (1-9) *N*-(4'-Chlor-3'-fluor-1,1'-biphenyl-2-yl)-4-(difluor-methyl)-2-methyl-1,3-thiazol-5-carboxamid | (6-6) Fenhexamid |
| E-18 | (1-9) *N*-(4'-Chlor-3'-fluor-1,1'-biphenyl-2-yl)-4-(difluor-methyl)-2-methyl-1,3-thiazol-5-carboxamid | (6-7) Carpropamid |
| E-19 | (1-9) *N*-(4'-Chlor-3'-fluor-1,1'-biphenyl-2-yl)-4-(difluor-methyl)-2-methyl-1,3-thiazol-5-carboxamid | (6-9) Picobenzamid |

(fortgesetzt)

| Nr. | Carboxamid der Formel (I) | Carboxamid der Formel (V) |
|---|---|---|
| E-20 | (1-9) *N*-(4'-Chlor-3'-fluor-1,1'-biphenyl-2-yl)-4-(difluor-methyl)-2-methyl-1,3-thiazol-5-carboxamid | (6-14) Penthiopyrad |

Die Wirkstoffkombinationen F enthalten neben einem Carboxamid der Formel (I) (Gruppe 1) auch ein Dithiocarbamat (Gruppe 7) ausgewählt aus

(7-1) Mancozeb

(7-2) Maneb

(7-3) Metiram

(7-4) Propineb

(7-5) Thiram

(7-6) Zineb

(7-7) Ziram

Bevorzugt sind Wirkstoffkombinationen F, worin das Dithiocarbamat (Gruppe 7) aus der folgenden Liste ausgewählt ist:

(7-1) Mancozeb

(7-2) Maneb

(7-4) Propineb

(7-5) Thiram

(7-6) Zineb

Besonders bevorzugt sind Wirkstoffkombinationen F, worin das Dithiocarbamat (Gruppe 7) aus der folgenden Liste ausgewählt ist:

(7-1) Mancozeb

(7-4) Propineb

Hervorgehoben sind die in der folgenden Tabelle 6 angeführten Wirkstoffkombinationen F:

**Tabelle 6: Wirkstoffkombinationen F**

| Nr. | Carboxamid der Formel (I) | Dithiocarbamat |
|---|---|---|
| F-1 | (1-1) N-(3',4'-Dichlor-5-fluor-1,1'-biphenyl-2-yl)-3-(difluormethyl)-1-methyl-1H-pyrazol-4-carboxamid | (7-1) Mancozeb |
| F-2 | (1-1) N-(3',4'-Dichlor-5-fluor-1,1'-biphenyl-2-yl)-3-(difluormethyl)-1-methyl-1H-pyrazol-4-carboxamid | (7-4) Propineb |
| F-3 | (1-7) N-(4'-Brom-1,1'-biphenyl-2-yl)-4-(difluormethyl)-2-methyl-1,3-thiazol-5-carboxamid | (7-1) Mancozeb |
| F-4 | (1-7) N-(4'-Brom-1,1'-biphenyl-2-yl)-4-(difluormethyl)-2-methyl-1,3-thiazol-5-carboxamid | (7-4) Propineb |

(fortgesetzt)

| Nr. | Carboxamid der Formel (I) | Dithiocarbamat |
|---|---|---|
| F-5 | (1-8) 4-(Difluormethyl)-2-methyl-N-[4'-(trifluormethyl)-1,1'-biphenyl-2-yl]-1,3-thiazol-5-carboxamid | (7-1) Mancozeb |
| F-6 | (1-8) 4-(Difluormethyl)-2-methyl-N-[4'-(trifluormethyl)-1,1'-biphenyl-2-yl]-1,3-thiazol-5-carboxamid | (7-4) Propineb |
| F-7 | (1-9) *N*-(4'-Chlor-3'-fluor-1,1'-biphenyl-2-yl)-4-(difluormethyl)-2-methyl-1,3-thiazol-5-carboxamid | (7-1) Mancozeb |
| F-8 | (1-9) *N*-(4'-Chlor-3'-fluor-1,1'-biphenyl-2-yl)-4-(difluormethyl)-2-methyl-1,3-thiazol-5-carboxamid | (7-4) Propineb |

Die Wirkstoffkombinationen G enthalten neben einem Carboxamid der Formel (I) (Gruppe 1) auch ein Acylalanin der Formel (VI) (Gruppe 8)

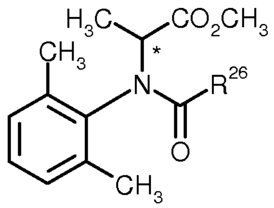

(VI)

in welcher * und $R^{26}$ die oben angegebenen Bedeutungen haben.

Bevorzugt sind Wirkstoffkombinationen G, worin das Acylalanin der Formel (VI) (Gruppe 8) aus der folgenden Liste ausgewählt ist:

(8-1) Benalaxyl

(8-2) Furalaxyl

(8-3) Metalaxyl

(8-4) Metalaxyl-M

(8-5) Benalaxyl-M

Besonders bevorzugt sind Wirkstoffkombinationen G, worin das Acylalanin der Formel (VI) (Gruppe 8) aus der folgenden Liste ausgewählt ist:

(8-3) Metalaxyl

(8-4) Metalaxyl-M

(8-5) Benalaxyl-M

Hervorgehoben sind die in der folgenden Tabelle 7 angeführten Wirkstoffkombinationen G:

**Tabelle 7: Wirkstoffkombinationen G**

| Nr. | Carboxamid der Formel (I) | Acylalanin der Formel (VI) |
|---|---|---|
| G-1 | (1-1) N-(3',4'-Dichlor-5-fluor-1,1'-biphenyl-2-yl)-3-(difluormethyl)-1-methyl-1H-pyrazol-4-carboxamid | (8-3) Metalaxyl |

(fortgesetzt)

| Nr. | Carboxamid der Formel (I) | Acylalanin der Formel (VI) |
|---|---|---|
| G-2 | (1-1) N-(3',4'-Dichlor-5-fluor-1,1'-biphenyl-2-yl)-3-(difluormethyl)-1-methyl-1H-pyrazol-4-carboxamid | (8-4) Metalaxyl-M |
| G-3 | (1-1) N-(3',4'-Dichlor-5-fluor-1,1'-biphenyl-2-yl)-3-(difluormethyl)-1-methyl-1H-pyrazol-4-carboxamid | (8-5) Benalaxyl-M |
| G-4 | (1-7) N-(4'-Brom-1,1'-biphenyl-2-yl)-4-(difluormethyl)-2-methyl-1,3-thiazol-5-carboxamid | (8-3) Metalaxyl |
| G-5 | (1-7) N-(4'-Brom-1,1'-biphenyl-2-yl)-4-(difluormethyl)-2-methyl-1,3-thiazol-5-carboxamid | (8-4) Metalaxyl-M |
| G-6 | (1-7) N-(4'-Brom-1,1'-biphenyl-2-yl)-4-(difluormethyl)-2-methyl-1,3-thiazol-5-carboxamid | (8-5) Benalaxyl-M |
| G-7 | (1-8) 4-(Difluormethyl)-2-methyl-N-[4'-(trifluormethyl)-1,1'-biphenyl-2-yl]-1,3-thiazol-5-carboxamid | (8-3) Metalaxyl |
| G-8 | (1-8) 4-(Difluormethyl)-2-methyl-N-[4'-(trifluormethyl)-1,1'-biphenyl-2-yl]-1,3-thiazol-5-carboxamid | (8-4) Metalaxyl-M |
| G-9 | (1-8) 4-(Difluormethyl)-2-methyl-N-[4'-(trifluormethyl)-1,1'-biphenyl-2-yl]-1,3-thiazol-5-carboxamid | (8-5) Benalaxyl-M |
| G-10 | (1-9) *N*-(4'-Chlor-3'-fluor-1,1'-biphenyl-2-yl)-4-(difluor-methyl)-2-methyl-1,3-thiazol-5-carboxamid | (8-3) Metalaxyl |
| G-11 | (1-9) *N*-(4'-Chlor-3'-fluor-1,1'-biphenyl-2-yl)-4-(difluor-methyl)-2-methyl-1,3-thiazol-5-carboxamid | (8-4) Metalaxyl-M |
| G-12 | (1-9) *N*-(4'-Chlor-3'-fluor-1,1'-biphenyl-2-yl)-4-(difluor-methyl)-2-methyl-1,3-thiazol-5-carboxamid | (8-5) Benalaxyl-M |

Die Wirkstoffkombinationen H enthalten neben einem Carboxamid der Formel (I) (Gruppe 1) auch ein Anilino-pyrimidin (Gruppe 9) ausgewählt aus

(9-1) Cyprodinil

(9-2) Mepanipyrim

(9-3) Pyrimethanil

Hervorgehoben sind die in der folgenden Tabelle 8 angeführten Wirkstoffkombinationen H:

**Tabelle 8: Wirkstoffkombinationen H**

| Nr. | Carboxamid der Formel (I) | Anilino-pyrimidin |
|---|---|---|
| H-1 | (1-1) N-(3',4'-Dichlor-5-fluor-1,1'-biphenyl-2-yl)-3-(difluormethyl)-1-methyl-1H-pyrazol-4-carboxamid | (9-1) Cyprodinil |
| H-2 | (1-1) N-(3',4'-Dichlor-5-fluor-1,1'-biphenyl-2-yl)-3-(difluormethyl)-1-methyl-1H-pyrazol-4-carboxamid | (9-2) Mepanipyrim |
| H-3 | (1-1) N-(3',4'-Dichlor-5-fluor-1,1'-biphenyl-2-yl)-3-(difluormethyl)-1-methyl-1H-pyrazol-4-carboxamid | (9-3) Pyrimethanil |
| H-4 | (1-7) N-(4'-Brom-1,1'-biphenyl-2-yl)-4-(difluormethyl)-2-methyl-1,3-thiazol-5-carboxamid | (9-1) Cyprodinil |
| H-5 | (1-7) N-(4'-Brom-1,1'-biphenyl-2-yl)-4-(difluormethyl)-2-methyl-1,3-thiazol-5-carboxamid | (9-2) Mepanipyrim |

(fortgesetzt)

| Nr. | Carboxamid der Formel (I) | Anilino-pyrimidin |
|---|---|---|
| H-6 | (1-7) N-(4'-Brom-1,1'-biphenyl-2-yl)-4-(difluormethyl)-2-methyl-1,3-thiazol-5-carboxamid | (9-3) Pyrimethanil |
| H-7 | (1-8) 4-(Difluormethyl)-2-methyl-N-[4'-(trifluormethyl)-1,1'-biphenyl-2-yl]-1,3-thiazol-5-carboxamid | (9-1) Cyprodinil |
| H-8 | (1-8) 4-(Difluormethyl)-2-methyl-N-[4'-(trifluormethyl)-1,1'-biphenyl-2-yl]-1,3-thiazol-5-carboxamid | (9-2) Mepanipyrim |
| H-9 | (1-8) 4-(Difluormethyl)-2-methyl-N-[4'-(trifluormethyl)-1,1'-biphenyl-2-yl]-1,3-thiazol-5-carboxamid | (9-3) Pyrimethanil |
| H-10 | (1-9) *N*-(4'-Chlor-3'-fluor-1,1'-biphenyl-2-yl)-4-(difluor-methyl-2-methyl-1,3-thiazol-5-carboxamid | (9-1) Cyprodinil |
| H-11 | (1-9) *N*-(4'-Chlor-3'-fluor-1,1'-biphenyl-2-yl)-4-(difluor-methyl)-2-methyl-1,3-thiazol-5-carboxamid | (9-2) Mepanipyrim |
| H-12 | (1-9) *N*-(4'-Chlor-3'-fluor-1,1'-biphenyl-2-yl)-4-(difluor-methyl)-2-methyl-1,3-thiazol-5-carboxamid | (9-3) Pyrimethanil |

Die Wirkstoffkombinationen I enthalten neben einem Carboxamid der Formel (I) (Gruppe 1) auch ein Benzimidazol der Formel (VIII) (Gruppe 10)

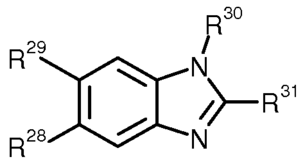

(VIII)

in welcher $R^{28}$, $R^{29}$, $R^{30}$ und $R^{31}$ die oben angegebenen Bedeutungen haben.

Bevorzugt sind Wirkstoffkombinationen I, worin das Benzimidazol der Formel (VIII) (Gruppe 10) aus der folgenden Liste ausgewählt ist:

(10-1) 6-Chlor-5-[(3,5-dimethylisoxazol-4-yl)sulfonyl]-2,2-difluor-5H-[1,3]dioxolo[4,5-f]-benzimidazol

(10-2) Benomyl

(10-3) Carbendazim

(10-4) Chlorfenazole

(10-5) Fuberidazole

(10-6) Thiabendazole

Besonders bevorzugt sind Wirkstoffkombinationen I, worin das Benzimidazol der Formel (VIII) (Gruppe 10) ist:

(10-3) Carbendazim

Hervorgehoben sind die in der folgenden Tabelle 9 angeführten Wirkstoffkombinationen I:

**Tabelle 9: Wirkstoffkombinationen I**

| Nr. | Carboxamid der Formel (I) | Benzimidazol der Formel (VIII) |
|---|---|---|
| I-1 | (1-1) N-(3',4'-Dichlor-5-fluor-1,1'-biphenyl-2-yl)-3-(difluormethyl)-1-methyl-1H-pyrazol-4-carboxamid | (10-3) Carbendazim |
| I-2 | (1-7) N-(4'-Brom-1,1'-biphenyl-2-yl)-4-(difluormethyl)-2-methyl-1,3-thiazol-5-carboxamid | (10-3) Carbendazim |
| I-3 | (1-8) 4-(Difluormethyl)-2-methyl-N-[4'-(trifluormethyl)-1,1'-biphenyl-2-yl]-1,3-thiazol-5-carboxamid | (10-3) Carbendazim |
| I-4 | (1-9) *N*-(4'-Chlor-3'-fluor-1,1'-biphenyl-2-yl)-4-(difluormethyl)-2-methyl-1,3-thiazol-5-carboxamid | (10-3) Carbendazim |

Die Wirkstoffkombinationen J enthalten neben einem Carboxamid der Formel (I) (Gruppe 1) auch ein Carbamat (Gruppe 11) der Formel (IX)

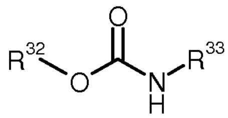

(IX)

in welcher $R^{32}$ und $R^{33}$ die oben angegebenen Bedeutungen haben.

Bevorzugt sind Wirkstoffkombinationen J, worin das Carbamat (Gruppe 11) aus der folgenden Liste ausgewählt ist:

(11-1) Diethofencarb

(11-2) Propamocarb

(11-3) Propamocarb-hydrochloride

(11-4) Propamocarb-Fosetyl

Hervorgehoben sind die in der folgenden Tabelle 10 angeführten Wirkstoffkombinationen J:

**Tabelle 10: Wirkstoffkombinationen J**

| Nr. | Carboxamid der Formel (I) | Carbamat der Formel (IX) |
|---|---|---|
| J-1 | (1-1) N-(3',4'-Dichlor-5-fluor-1,1'-biphenyl-2-yl)-3-(difluormethyl)-1-methyl-1H-pyrazol-4-carboxamid | (11-2) Propamocarb |
| J-2 | (1-1) N-(3',4'-Dichlor-5-fluor-1,1'-biphenyl-2-yl)-3-(difluormethyl)-1-methyl-1H-pyrazol-4-carboxamid | (11-3) Propamocarb-hydrochloride |
| J-3 | (1-1) N-(3',4'-Dichlor-5-fluor-1,1'-biphenyl-2-yl)-3-(difluormethyl)-1-methyl-1H-pyrazol-4-carboxamid | (11-4) Propamocarb-Fosetyl |
| J-4 | (1-7) N-(4'-Brom-1,1'-biphenyl-2-yl)-4-(difluormethyl)-2-methyl-1,3-thiazol-5-carboxamid | (11-2) Propamocarb |
| J-5 | (1-7) N-(4'-Brom-1,1'-biphenyl-2-yl)-4-(difluormethyl)-2-methyl-1,3-thiazol-5-carboxamid | (11-3) Propamocarb-hydrochloride |
| J-6 | (1-7) N-(4'-Brom-1,1'-biphenyl-2-yl)-4-(difluormethyl)-2-methyl-1,3-thiazol-5-carboxamid | (11-4) Propamocarb-Fosetyl |
| J-7 | (1-8) 4-(Difluormethyl)-2-methyl-N-[4'-(trifluormethyl)-1,1'-biphenyl-2-yl]-1,3-thiazol-5-carboxamid | (11-2) Propamocarb |

(fortgesetzt)

| Nr. | Carboxamid der Formel (I) | Carbamat der Formel (IX) |
|---|---|---|
| J-8 | (1-8) 4-(Difluormethyl)-2-methyl-N-[4'-(trifluormethyl)-1,1'-biphenyl-2-yl]-1,3-thiazol-5-carboxamid | (11-3) Propamocarb-hydrochloride |
| J-9 | (1-8) 4-(Difluormethyl)-2-methyl-N-[4'-(trifluormethyl)-1,1'-biphenyl-2-yl]-1,3-thiazol-5-carboxamid | (11-4) Propamocarb-Fosetyl |
| J-10 | (1-9) *N*-(4'-Chlor-3'-fluor-1,1'-biphenyl-2-yl)-4-(difluor-methyl)-2-methyl-1,3-thiazol-5-carboxamid | (11-2) Propamocarb |
| J-11 | (1-9) *N*-(4'-Chlor-3'-fluor-1,1'-biphenyl-2-yl)-4-(difluor-methyl)-2-methyl-1,3-thiazol-5-carboxamid | (11-3) Propamocarb-hydrochloride |
| J-12 | (1-9) *N*-(4'-Chlor-3'-fluor-1,1'-biphenyl-2-yl)-4-(difluor-methyl)-2-methyl-1,3-thiazol-5-carboxamid | (11-4) Propamocarb-Fosetyl |

Die Wirkstoffkombinationen K enthalten neben einem Carboxamid der Formel (I) (Gruppe 1) auch ein Dicarboximid (Gruppe 12) ausgewählt aus

(12-1) Captafol

(12-2) Captan

(12-3) Folpet

(12-4) Iprodione

(12-5) Procymidone

(12-6) Vinclozolin

Bevorzugt sind Wirkstoffkombinationen K, worin das Dicarboximid (Gruppe 12) aus der folgenden Liste ausgewählt ist:

(12-2) Captan

(12-3) Folpet

(12-4) Iprodione

Hervorgehoben sind die in der folgenden Tabelle 11 angeführten Wirkstoffkombinationen K:

**Tabelle 11: Wirkstoffkombinationen K**

| Nr. | Carboxamid der Formel (I) | Dicarboximid |
|---|---|---|
| K-1 | (1-1) N-(3',4'-Dichlor-5-fluor-1,1'-biphenyl-2-yl)-3-(difluormethyl)-1-methyl-1H-pyrazol-4-carboxamid | (12-2) Captan |
| K-2 | (1-1) N-(3',4'-Dichlor-5-fluor-1,1'-biphenyl-2-yl)-3-(difluormethyl)-1-methyl-1H-pyrazol-4-carboxamid | (12-3) Folpet |
| K-3 | (1-1) N-(3',4'-Dichlor-5-fluor-1,1'-biphenyl-2-yl)-3-(difluormethyl)-1-methyl-1H-pyrazol-4-carboxamid | (12-4) Iprodione |
| K-4 | (1-7) N-(4'-Brom-1,1'-biphenyl-2-yl)-4-(difluormethyl)-2-methyl-1,3-thiazol-5-carboxamid | (12-2) Captan |
| K-5 | (1-7) N-(4'-Brom-1,1'-biphenyl-2-yl)-4-(difluormethyl)-2-methyl-1,3-thiazol-5-carboxamid | (12-3) Folpet |

(fortgesetzt)

| Nr. | Carboxamid der Formel (I) | Dicarboximid |
|---|---|---|
| K-6 | (1-7) N-(4'-Brom-1,1'-biphenyl-2-yl)-4-(difluormethyl)-2-methyl-1,3-thiazol-5-carboxamid | (12-4) Iprodione |
| K-7 | (1-8) 4-(Difluormethyl)-2-methyl-N-[4'-(trifluormethyl)-1,1'-biphenyl-2-yl]-1,3-thiazol-5-carboxamid | (12-2) Captan |
| K-8 | (1-8) 4-(Difluormethyl)-2-methyl-N-[4'-(trifluormethyl)-1,1'-biphenyl-2-yl]-1,3-thiazol-5-carboxamid | (12-3) Folpet |
| K-9 | (1-8) 4-(Difluormethyl)-2-methyl-N-[4'-(trifluormethyl)-1,1'-biphenyl-2-yl]-1,3-thiazol-5-carboxamid | (12-4) Iprodione |
| K-10 | (1-9) *N*-(4'-Chlor-3'-fluor-1,1'-biphenyl-2-yl)-4-(difluormethyl)-2-methyl-1,3-thiazol-5-carboxamid | (12-2) Captan |
| K-11 | (1-9) *N*-(4'-Chlor-3'-fluor-1,1'-biphenyl-2-yl)-4-(difluormethyl)-2-methyl-1,3-thiazol-5-carboxamid | (12-3) Folpet |
| K-12 | (1-9) *N*-(4'-Chlor-3'-fluor-1,1'-biphenyl-2-yl)-4-(difluormethyl)-2-methyl-1,3-thiazol-5-carboxamid | (12-4) Iprodione |

Die Wirkstoffkombinationen L enthalten neben einem Carboxamid der Formel (I) (Gruppe 1) auch ein Guanidin (Gruppe 13) ausgewählt aus

(13-1) Dodine

(13-2) Guazatine

(13-3) Iminoctadine triacetate

(13-4) Iminoctadine tris(albesilate)

Bevorzugt sind Wirkstoffkombinationen L, worin das Guanidin (Gruppe 13) aus der folgenden Liste ausgewählt ist:

(13-1) Dodine

(13-2) Guazatine

Hervorgehoben sind die in der folgenden Tabelle 12 angeführten Wirkstoffkombinationen L:

**Tabelle 12: Wirkstoffkombinationen L**

| Nr. | Carboxamid der Formel (I) | Guanidin |
|---|---|---|
| L-1 | (1-1) N-(3',4'-Dichlor-5-fluor-1,1'-biphenyl-2-yl)-3-(difluormethyl)-1-methyl-1H-pyrazol-4-carboxamid | (13-1) Dodine |
| L-2 | (1-1) N-(3',4'-Dichlor-5-fluor-1,1'-biphenyl-2-yl)-3-(difluormethyl)-1-methyl-1H-pyrazol-4-carboxamid | (13-2) Guazatine |
| L-3 | (1-7) N-(4'-Brom-1,1'-biphenyl-2-yl)-4-(difluormethyl)-2-methyl-1,3-thiazol-5-carboxamid | (13-1) Dodine |
| L-4 | (1-7) N-(4'-Brom-1,1'-biphenyl-2-yl)-4-(difluormethyl)-2-methyl-1,3-thiazol-5-carboxamid | (13-2) Guazatine |
| L-5 | (1-8) 4-(Difluormethyl)-2-methyl-N-[4'-(trifluormethyl)-1,1'-biphenyl-2-yl]-1,3-thiazol-5-carboxamid | (13-1) Dodine |

(fortgesetzt)

| Nr. | Carboxamid der Formel (I) | Guanidin |
|---|---|---|
| L-6 | (1-8) 4-(Difluormethyl)-2-methyl-N-[4'-(trifluormethyl)-1,1'-biphenyl-2-yl]-1,3-thiazol-5-carboxamid | (13-2) Guazatine (13-2) Guazatine |
| L-7 | (1-9) *N*-(4'-Chlor-3'-fluor-1,1'-biphenyl-2-yl)-4-(difluormethyl)-2-methyl-1,3-thiazol-5-carboxamid | (13-1) Dodine |
| L-8 | (1-9) *N*-(4'-Chlor-3'-fluor-1,1'-biphenyl-2-yl)-4-(difluormethyl)-2-methyl-1,3-thiazol-5-carboxamid | (13-2) Guazatine |

Die Wirkstoffkombinationen M enthalten neben einem Carboxamid der Formel (I) (Gruppe 1) auch ein Imidazol (Gruppe 14) ausgewählt aus

(14-1) Cyazofamid

(14-2) Prochloraz

(14-3) Triazoxide

(14-4) Pefurazoate

Bevorzugt sind Wirkstoffkombinationen M, worin das Imidazol (Gruppe 14) aus der folgenden Liste ausgewählt ist:

(14-2) Prochloraz

(14-3) Triazoxide

Hervorgehoben sind die in der folgenden Tabelle 13 angeführten Wirkstoffkombinationen M:

**Tabelle 13: Wirkstoffkombinationen M**

| Nr. | Carboxamid der Formel (I) | Imidazol |
|---|---|---|
| M-1 | (1-1) N-(3',4'-Dichlor-5-fluor-1,1'-biphenyl-2-yl)-3-(difluormethyl)-1-methyl-1H-pyrazol-4-carboxamid | (14-2) Prochloraz |
| M-2 | (1-1) N-(3',4'-Dichlor-5-fluor-1,1'-biphenyl-2-yl)-3-(difluormethyl)-1-methyl-1H-pyrazol-4-carboxamid | (14-3) Triazoxide |
| M-3 | (1-7) N-(4'-Brom-1,1'-biphenyl-2-yl)-4-(difluormethyl)-2-methyl-1,3-thiazol-5-carboxamid | (14-2) Prochloraz |
| M-4 | (1-7) N-(4'-Brom-1,1'-biphenyl-2-yl)-4-(difluormethyl)-2-methyl-1,3- thiazol-5-carboxamid | (14-3) Triazoxide |
| M-5 | (1-8) 4-(Difluormethyl)-2-methyl-N-[4'-(trifluormethyl)-1,1'-biphenyl-2-yl]-1,3-thiazol-5-carboxamid | (14-2) Prochloraz |
| M-6 | (1-8) 4-(Difluormethyl)-2-methyl-N-[4'-(trifluormethyl)-1,1'-biphenyl-2-yl]-1,3-thiazol-5-carboxamid | (14-3) Triazoxide |
| M-7 | (1-9) *N*-(4'-Chlor-3'-fluor-1,1'-biphenyl-2-yl)-4-(difluormethyl)-2-methyl-1,3-thiazol-5-carboxamid | (14-2) Prochloraz |
| M-8 | (1-9) *N*-(4'-Chlor-3'-fluor-1,1'-biphenyl-2-yl)-4-(difluormethyl)-2-methyl-1,3-thiazol-5-carboxamid | (14-3) Triazoxide |

Die Wirkstoffkombinationen N enthalten neben einem Carboxamid der Formel (I) (Gruppe 1) auch ein Morpholin (Gruppe 15) der Formel (X)

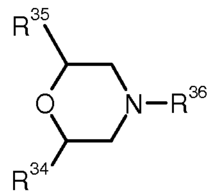

(X)

in welcher $R^{34}$, $R^{35}$ und $R^{36}$ die oben angegebenen Bedeutungen haben.

Bevorzugt sind Wirkstoffkombinationen N, worin das Morpholin (Gruppe 15) der Formel (X) aus der folgenden Liste ausgewählt ist:

(15-1) Aldimorph

(15-2) Tridemorph

(15-3) Dodemorph

(15-4) Fenpropimorph

(15-5) Dimethomorph

Besonders bevorzugt sind Wirkstoffkombinationen N, worin das Morpholin (Gruppe 15) der Formel (X) aus der folgenden Liste ausgewählt ist:

(15-4) Fenpropimorph

(15-5) Dimethomorph

Hervorgehoben sind die in der folgenden Tabelle 14 angeführten Wirkstoffkombinationen N:

**Tabelle 14: Wirkstoffkombinationen N**

| Nr. | Carboxamid der Formel (I) | Morpholin der Formel (X) |
|---|---|---|
| N-1 | (1-1) N-(3',4'-Dichlor-5-fluor-1,1'-biphenyl-2-yl)-3-(difluormethyl)-1-methyl-1H-pyrazol-4-carboxamid | (15-4) Fenpropimorph |
| N-2 | (1-7) N-(4'-Brom-1,1'-biphenyl-2-yl)-4-(difluormethyl)-2-methyl-1,3-thiazol-5-carboxamid | (15-4) Fenpropimorph |
| N-3 | (1-8) 4-(Difluormethyl)-2-methyl-N-[4'-(trifluormethyl)-1,1'-biphenyl-2-yl]-1,3-thiazol-5-carboxamid | (15-4) Fenpropimorph (15-4) Fenpropimorph |
| N-4 | (1-9) *N*-(4'-Chlor-3'-fluor-1,1'-biphenyl-2-yl)-4-(difluor-methyl)-2-methyl-1,3-thiazol-5-carboxamid | (15-4) Fenpropimorph |

Die Wirkstoffkombinationen O enthalten neben einem Carboxamid der Formel (I) (Gruppe 1) auch ein Pyrrol (Gruppe 16) der Formel (XI)

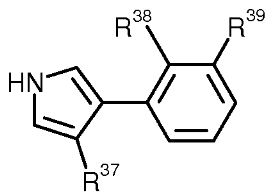

(XI)

in welcher $R^{37}$, $R^{38}$ und $R^{39}$ die oben angegebenen Bedeutungen haben.

Bevorzugt sind Wirkstoffkombinationen O, worin das Pyrrol (Gruppe 16) der Formel (XI) aus der folgenden Liste ausgewählt ist:

(16-1) Fenpiclonil

(16-2) Fludioxonil

(16-3) Pyrrolnitrine

Besonders bevorzugt sind Wirkstoffkombinationen O, worin das Pyrrol (Gruppe 16) der Formel (XI) aus der folgenden Liste ausgewählt ist:

(16-2) Fludioxonil

Hervorgehoben sind die in der folgenden Tabelle 15 angeführten Wirkstoffkombinationen O:

**Tabelle 15: Wirkstoffkombinationen O**

| Nr. | Carboxamid der Formel (I) | Pyrrol der Formel (XI) |
|---|---|---|
| O-1 | (1-1) N-(3',4'-Dichlor-5-fluor-1,1'-biphenyl-2-yl)-3-(difluormethyl)-1-methyl-1H-pyrazol-4-carboxamid | (16-2) Fludioxonil |
| O-2 | (1-7) N-(4'-Brom-1,1'-biphenyl-2-yl)-4-(difluormethyl)-2-methyl-1,3-thiazol-5-carboxamid | (16-2) Fludioxonil |
| O-3 | (1-8) 4-(Difluormethyl)-2-methyl-N-[4'-(trifluormethyl)-1,1'-biphenyl-2-yl]-1,3-thiazol-5-carboxamid | (16-2) Fludioxonil |
| O-4 | (1-9) N-(4'-Chlor-3'-fluor-1,1'-biphenyl-2-yl)-4-(difluormethyl)-2-methyl-1,3-thiazol-5-carboxamid | (16-2) Fludioxonil |

Die Wirkstoffkombinationen P enthalten neben einem Carboxamid der Formel (I) (Gruppe 1) auch ein Phosphonat (Gruppe 17) ausgewählt aus

(17-1) Fosetyl-Al
(17-2) Phosphonsäure

Hervorgehoben sind die in der folgenden Tabelle 16 angeführten Wirkstoffkombinationen P:

**Tabelle 16: Wirkstoffkombinationen P**

| Nr. | Carboxamid der Formel (I) | Phosphonat |
|---|---|---|
| P-1 | (1-1) N-(3',4'-Dichlor-5-fluor-1,1'-biphenyl-2-yl)-3-(difluormethyl)-1-methyl-1H-pyrazol-4-carboxamid | (17-1) Fosetyl-Al |
| P-2 | (1-7) N-(4'-Brom-1,1'-biphenyl-2-yl)-4-(difluormethyl)-2-methyl-1,3-thiazol-5-carboxamid | (17-1) Fosetyl-Al |
| P-3 | (1-8) 4-(Difluormethyl)-2-methyl-N-[4'-(trifluormethyl)-1,1'-biphenyl-2-yl]-1,3-thiazol-5-carboxamid | (17-1) Fosetyl-Al |
| P-4 | (1-9) N-(4'-Chlor-3'-fluor-1,1'-biphenyl-2-yl)-4-(difluormethyl)-2-methyl-1,3-thiazol-5-carboxamid | (17-1) Fosetyl-Al |

Die Wirkstoffkombinationen Q enthalten neben einem Carboxamid der Formel (I) (Gruppe 1) auch ein Fungizid (Gruppe 19) ausgewählt aus

(19-1) Acibenzolar-S-methyl

(19-2) Chlorothalonil

(19-3) Cymoxanil

(19-4) Edifenphos

(19-5) Famoxadone

(19-6) Fluazinam

(19-7) Kupferoxychlorid

(19-8) Kupferhydroxid

(19-9) Oxadixyl

(19-10) Spiroxamine

(19-11) Dithianon

(19-12) Metrafenone

(19-13) Fenamidone

(19-14) 2,3-Dibutyl-6-chlor-thieno[2,3-d]pyrimidin-4(3H)on

(19-15) Probenazole

(19-16) Isoprothiolane

(19-17) Kasugamycin

(19-18) Phthalide

(19-19) Ferimzone

(19-20) Tricyclazole

(19-21) N-({4-[(Cyclopropylamino)carbonyl]phenyl}sulfonyl)-2-methoxybenzamid

(19-22) 2-(4-Chlorphenyl)-N-{2-[3-methoxy-4-(prop-2-in-1-yloxy)phenyl]ethyl}-2-(prop-2-in-1-yloxy)acetamid

Bevorzugt sind Wirkstoffkombinationen Q, worin das Fungizid (Gruppe 19) aus der folgenden Liste ausgewählt ist:

(19-1) Acibenzolar-S-methyl

(19-2) Chlorothalonil

(19-3) Cymoxanil

(19-5) Famoxadone

(19-6) Fluazinam

(19-7) Kupferoxychlorid

(19-9) Oxadixyl

(19-10) Spiroxamine

(19-13) Fenamidone

(19-21) N-({4-[(Cyclopropylamino)carbonyl]phenyl}sulfonyl)-2-methoxybenzamid

(19-22) 2-(4-Chlorphenyl)-N-{2-[3-methoxy-4-(prop-2-in-1-yloxy)phenyl]ethyl}-2-(prop-2-in-1-yloxy)acetamid

Besonders bevorzugt sind Wirkstoffkombinationen Q, worin das Fungizid (Gruppe 19) aus der folgenden Liste ausgewählt ist:

(19-2) Chlorothalonil

(19-7) Kupferoxychlorid

(19-10) Spiroxamine

(19-21) N-({4-[(Cyclopropylamino)carbonyl]phenyl}sulfonyl)-2-methoxybenzamid (19-

22) 2-(4-Chlorphenyl)-N-{2-[3-methoxy-4-(prop-2-in-1-yloxy)phenyl]ethyl}-2-(prop-2-in-1-yloxy)acetamid

Hervorgehoben sind die in der folgenden Tabelle 17 angeführten Wirkstoffkombinationen Q:

**Tabelle 17: Wirkstoffkombinationen Q**

| Nr. | Carboxamid der Formel (I) | Fungizid |
|---|---|---|
| Q-1 | (1-1) N-(3',4'-Dichlor-5-fluor-1,1'-biphenyl-2-yl)-3-(difluormethyl)-1-methyl-1H-pyrazol-4-carboxamid | (19-2) Chlorothalonil |
| Q-2 | (1-1) N-(3',4'-Dichlor-5-fluor-1,1'-biphenyl-2-yl)-3-(difluormethyl)-1-methyl-1H-pyrazol-4-carboxamid | (19-7) Kupferoxychlorid |
| Q-3 | (1-1) N-(3',4'-Dichlor-5-fluor-1,1'-biphenyl-2-yl)-3-(difluormethyl)-1-methyl-1H-pyrazol-4-carboxamid | (19-10) Spiroxamine |
| Q-4 | (1-1) N-(3',4'-Dichlor-5-fluor-1,1'-biphenyl-2-yl)-3-(difluormethyl)-1-methyl-1H-pyrazol-4-carboxamid | (19-21) N-({4-[(Cyclopropylamino)-carbonyl] phenyl}sulfonyl)-2-methoxy-benzamid |
| Q-5 | (1-1) N-(3',4'-Dichlor-5-fluor-1,1'-biphenyl-2-yl)-3-(difluormethyl)-1-methyl-1H-pyrazol-4-carboxamid | (19-22) 2-(4-Chlorphenyl)-N-{2-[3- methoxy-4-(prop-2-in-1-yloxy)phenyl]-ethyl}-2-(prop-2-in-1-yloxy)acetamid |
| Q-6 | (1-7) N-(4'-Brom-1,1'-biphenyl-2-yl)-4-(difluor-methyl)-2-methyl-1,3-thiazol-5-carboxamid | (19-2) Chlorothalonil |
| Q-7 | (1-7) N-(4'-Brom-1,1'-biphenyl-2-yl)-4-(difluor-methyl)-2-methyl-1,3-thiazol-5-carboxamid | (19-7) Kupferoxychlorid |
| Q-8 | (1-7) N-(4'-Brom-1,1'-biphenyl-2-yl)-4-(difluor-methyl)-2-methyl-1,3-thiazol-5-carboxamid | (19-10) Spiroxamine |
| Q-9 | (1-7) N-(4'-Brom-1,1'-biphenyl-2-yl)-4-(difluor-methyl)-2-methyl-1,3-thiazol-5-carboxamid | (19-21) N-({4-[(Cyclopropylamino)-carbonyl] phenyl}sulfonyl)-2-methoxy-benzamid |
| Q-10 | (1-7) N-(4'-Brom-1,1'-biphenyl-2-yl)-4-(difluor methyl)-2-methyl-1,3-thiazol-5-carboxamid | (19-22) 2-(4-Chlorphenyl)-N-{2-[3-methoxy-4-(prop-2-in-1-yloxy)phenyl]-ethyl}-2-(prop-2-in-1-yloxy)acetamid |

(fortgesetzt)

| Nr. | Carboxamid der Formel (I) | Fungizid |
|---|---|---|
| Q-11 | (1-8) 4-(Difluormethyl)-2-methyl-N-[4'-(trifluormethyl)-1,1'-biphenyl-2-yl]-1,3-thiazol-5-carboxamid | (19-2) Chlorothalonil (19-2) Chlorothalonil |
| Q-12 | (1-8) 4-(Difluormethyl)-2-methyl-N-[4'-(trifluormethyl)-1,1'-biphenyl-2-yl]-1,3-thiazol-5-carboxamid | (19-7) Kupferoxychlorid |
| Q-13 | (1-8) 4-(Difluormethyl)-2-methyl-N-[4'-(trifluormethyl)-1,1'-biphenyl-2-yl]-1,3-thiazol-5-carboxamid | (19-10) Spiroxamine |
| Q-14 | (1-8) 4-(Difluormethyl)-2-methyl-N-[4'-(trifluorme thyl)-1,1'-biphenyl-2-yl]-1,3-thiazol-5-carboxamid | (19-21) N-({4-[(Cyclopropylamino)-carbonyl] phenyl}sulfonyl)-2-methoxy-benzamid |
| Q-15 | (1-8) 4-(Difluormethyl)-2-methyl-N-[4'-(trifluormethyl)-1,1'-biphenyl-2-yl]-1,3-thiazol-5-carboxamid | (19-22) 2-(4-Chlorphenyl)-N-{2-[3-methoxy-4-(prop-2-in-1-yloxy)phenyl]-ethyl}-2-(prop-2-in-1-yloxy)acetamid |
| Q-16 | (1-9) *N*-(4'-Chlor-3'-fluor-1,1'-biphenyl-2-yl)-4-(difluormethyl)-2-methyl-1,3-thiazol-5-carboxamid | (19-2) Chlorothalonil |
| Q-17 | (1-9) *N*-(4'-Chlor-3'-fluor-1,1'-biphenyl-2-yl)-4-(difluormethyl)-2-methyl-1,3-thiazol-5-carboxamid | (19-7) Kupferoxychlorid |
| Q-18 | (1-9) *N*-(4'-Chlor-3'-fluor-1,1'-biphenyl-2-yl)-4-(difluormethyl)-2-methyl-1,3-thiazol-5-carboxamid | (19-10) Spiroxamine |
| Q-19 | (1-9) *N*-(4'-Chlor-3'-fluor-1,1'-biphenyl-2-yl)-4-(di fluormethyl)-2-methyl-1,3-thiazol-5-carboxamid | (19-21) N-({4-[(Cyclopropylamino)-carbonyl] phenyl}sulfonyl)-2-methoxy-benzamid |
| Q-20 | (1-9) *N*-(4'-Chlor-3'-fluor-1,1'-biphenyl-2-yl)-4-(di fluormethyl)-2-methyl-1,3-thiazol-5-carboxamid | (19-22) 2-(4-Chlorphenyl)-N-{2-[3-methoxy-4-(prop-2-in-1-yloxy)phenyl]-ethyl}-2-(prop-2-in-1-yloxy)acetamid |

Die Wirkstoffkombinationen R enthalten neben einem Carboxamid der Formel (I) (Gruppe 1) auch ein (Thio) Harnstoff-Derivat (Gruppe 20) ausgewählt aus

(20-1) Pencycuron
(20-2) Thiophanate-methyl
(20-3) Thiophanate-ethyl

Bevorzugt sind Wirkstoffkombinationen R, worin das (Thio)Harnstoff-Derivat (Gruppe 20) aus der folgenden Liste ausgewählt ist:

(20-1) Pencycuron

(20-2) Thiophanate-methyl

Hervorgehoben sind die in der folgenden Tabelle 18 angeführten Wirkstoffkombinationen R:

**Tabelle 18: Wirkstoffkombinationen R**

| Nr. | Carboxamid der Formel (I) | (Thio)Harnstoff-Derivat |
|---|---|---|
| R-1 | (1-1) N-(3',4'-Dichlor-5-fluor-1,1'-biphenyl-2-yl)-3-(difluormethyl)-1-methyl-1H-pyrazol-4-carboxamid | (20-1) Pencycuron |
| R-2 | (1-7) N-(4'-Brom-1,1'-biphenyl-2-yl)-4-(difluormethyl)-2-methyl-1,3-thiazol-5-carboxamid | (20-1) Pencycuron |
| R-3 | (1-8) 4-(Difluormethyl)-2-methyl-N-[4'-(trifluormethyl)-1,1'-biphenyl-2-yl]-1,3-thiazol-5-carboxamid | (20-1) Pencycuron |

(fortgesetzt)

| Nr. | Carboxamid der Formel (I) | (Thio)Harnstoff-Derivat |
|---|---|---|
| R-4 | (1-9) *N*-(4'-Chlor-3'-fluor-1,1'-biphenyl-2-yl)-4-(difluor-methyl)-2-methyl-1,3-thiazol-5-carboxamid | (20-1) Pencycuron |

Die Wirkstoffkombinationen S enthalten neben einem Carboxamid der Formel (I) (Gruppe 1) auch ein Triazolopyrimidin (Gruppe 22) der Formel (XIV)

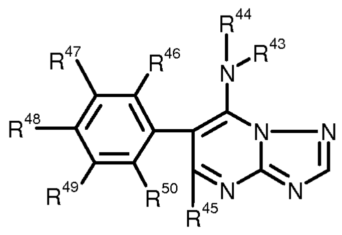

(XIV)

in welcher $R^{43}$, $R^{44}$, $R^{45}$, $R^{46}$, $R^{47}$, $R^{48}$, $R^{49}$ und $R^{50}$ die oben angegebenen Bedeutungen haben.

Bevorzugt sind Wirkstoffkombinationen S, worin das Triazolopyrimidin (Gruppe 22) der Formel (XIV) aus der folgenden Liste ausgewählt ist:

(22-1) 5-Chlor-*N*-[*(1S)*-2,2,2-trifluor-1-methylethyl]-6-(2,4,6-trifluorphenyl)[1,2,4]triazolo[1,5-a]-pyrimidin-7-amin

(22-2) 5-Chlor-*N*-[*(1R)*-1,2-dimethylpropyl]-6-(2,4,6-trifluorphenyl)[1,2,4]triazolo[1,5-a]pyrimidin-7-amin

(22-3) 5-Chlor-6-(2-chlor-6-fluorphenyl)-7-(4-methylpiperidin-1-yl)[1,2,4]triazolo[1,5-a]pyrimidin

(22-4) 5-Chlor-6-(2,4,6-trifluorphenyl)-7-(4-methylpiperidin-1-yl)[1,2,4]triazolo[1,5-a]pyrimidin

Besonders bevorzugt sind Wirkstoffkombinationen S, worin das Triazolopyrimidin (Gruppe 22) der Formel (XIV) aus der folgenden Liste ausgewählt ist:

(22-1) 5-Chlor-*N*-[*(1S)*-2,2,2-trifluor-1-methylethyl]-6-(2,4,6-trifluorphenyl)[1,2,4]triazolo[1,5-a]-pyrimidin-7-amin

(22-2) 5-Chlor-*N*-[*(1R)*-1,2-dimethylpropyl]-6-(2,4,6-trifluorphenyl)[1,2,4]triazolo[1,5-a]pyrimidin-7-amin

(22-4) 5-Chlor-6-(2,4,6-trifluorphenyl)-7-(4-methylpiperidin-1-yl)[1,2,4]triazolo[1,5-a]pyrimidin

Hervorgehoben sind die in der folgenden Tabelle 19 angeführten Wirkstoffkombinationen S:

**Tabelle 19: Wirkstoffkombinationen S**

| Nr. | Carboxamid der Formel (I) | Triazolopyrimidin der Formel (XIV) |
|---|---|---|
| S-1 | (1-1) N-(3',4'-Dichlor-5-fluor-1,1'-biphenyl-2-yl)-3-(difluormethyl)-1-methyl-1H-pyrazol-4-carboxamid | (22-1) 5-Chlor-*N*-[*(1S)*-2,2,2-trifluor-1-methylethyl]-6-(2,4,6-trifluorphenyl)-[1,2,4] triazolo[1,5-a]pyrimidin-7-amin |
| S-2 | (1-1) N-(3',4'-Dichlor-5-fluor-1,1'-biphenyl-2-yl)-3-(difluormethyl)-1-methyl-1H-pyrazol-4-carboxamid | (22-2) 5-Chlor-*N*-[*(1R)*-1,2-dimethylpropyl]-6-(2,4,6-trifluorphenyl)[1,2,4]triazolo[1,5-a]pyrimidin-7-amin |
| S-3 | (1-1) N-(3',4'-Dichlor-5-fluor-1,1'-biphenyl-2-yl)-3-(difluormethyl)-1-methyl-1H-pyrazol-4-carboxamid | (22-4) 5-Chlor-6-(2,4,6-trifluorphenyl)-7-(4-methylpiperidin-1-yl)[1,2,4]triazolo[1,5-a]pyrimidin |

(fortgesetzt)

| Nr. | Carboxamid der Formel (I) | Triazolopyrimidin der Formel (XIV) |
|---|---|---|
| S-4 | (1-7) N-(4'-Brom-1,1'-biphenyl-2-yl)-4-(difluormethyl)-2-methyl-1,3-thiazol-5-carboxamid | (22-1) 5-Chlor-*N*-[*(1S)*-2,2,2-trifluor-1-methylethyl]-6-(2,4,6-trifluorphenyl)-[1,2,4] triazolo[1,5-a]pyrimidin-7-amin |
| S-5 | (1-7) N-(4'-Brom-1,1'-biphenyl-2-yl)-4-(difluormethyl)-2-methyl-1,3-thiazol-5-carboxamid | (22-2) 5-Chlor-*N*-[*(1R)*-1,2-dimethylpropyl]-6-(2,4,6-trifluorphenyl)[1,2,4]triazolo[1,5-a]pyrimidin-7-amin |
| S-6 | (1-7) N-(4'-Brom-1,1'-biphenyl-2-yl)-4 (difluormethyl)-2-methyl-1,3-thiazol-5-carboxamid | (22-4) -5-Chlor-6-(2,4,6-trifluorphenyl)-7-(4-methylpiperidin-1-yl)[1,2,4]triazolo[1,5-a]pyrimidin |
| S-7 | (1-8) 4-(Difluormethyl)-2-methyl-N-[4'-(trifluormethyl)-1,1'-biphenyl-2-yl]-1,3-thiazol-5-carboxamid | (22-1) 5-Chlor-*N*-[*(1S)*-2,2,2-trifluor-1-methylethyl]-6-(2,4,6-trifluorphenyl)-[1,2,4] triazolo[1,5-a]pyrimidin-7-amin |
| S-8 | (1-8) 4-(Difluormethyl)-2-methyl-N-[4'-(trifluormethyl)-1,1'-biphenyl-2-yl]-1,3-thiazol-5-carboxamid | (22-2) 5-Chlor-*N*-[*(1R)*-1,2-dimethylpropyl]-6-(2,4,6-trifluorphenyl)[1,2,4]triazolo[1,5-a]pyrimidin-7-amin |
| S-9 | (1-8) 4-(Difluormethyl)-2-methyl-N-[4'-(trifluormethyl)-1,1'-biphenyl-2-yl]-1,3-thiazol-5-carboxamid | (22-4) 5-Chlor-6-(2,4,6-trifluorphenyl)-7-(4-methylpiperidin-1-yl)[1,2,4]triazolo[1,5-a]pyrimidin |
| S-10 | (1-9) *N*-(4'-Chlor-3'-fluor-1,1'-biphenyl-2-yl)-4-(difluormethyl)-2-methyl-1,3-thiazol-5-carboxamid | (22-1) 5-Chlor-*N*-[*(1S)*-2,2,2-trifluor-1-methylethyl]-6-(2,4,6-trifluorphenyl)-[1,2,4] triazolo[1,5-a]pyrimidin-7-amin |
| S-11 | (1-9) *N*-(4'-Chlor-3'-fluor-1,1'-biphenyl-2-yl)-4-(difluormethyl)-2-methyl-1,3-thiazol-5-carboxamid | (22-2) 5-Chlor-*N*-[*(1R)*-1,2-dimethylpropyl]-6-(2,4,6-trifluorphenyl)[1,2,4]triazolo[1,5-a]pyrimidin-7-amin |
| S-12 | (1-9) *N*-(4'-Chlor-3'-fluor-1,1'-biphenyl-2 yl)-4-(difluormethyl)-2-methyl-1,3-thiazol-5-carboxamid | (22-4) 5-Chlor-6-(2,4,6-trifluorphenyl)-7-(4-methylpiperidin-1-yl)[1,2,4]triazolo[1,5-a]pyrimidin |

Die Wirkstoffkombinationen T enthalten neben einem Carboxamid der Formel (I) (Gruppe 1) auch ein Iodochromon (Gruppe 23) der Formel (XV)

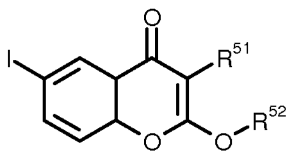

(XV)

in welcher $R^{51}$ und $R^{52}$ die oben angegebenen Bedeutungen haben.

Bevorzugt sind Wirkstoffkombinationen T, worin das Iodochromon (Gruppe 23) der Formel (XV) aus der folgenden Liste ausgewählt ist:

(23-1) 2-Butoxy-6-iod-3-propyl-benzopyran-4-on

(23-2) 2-Ethoxy-6-iod-3-propyl-benzopyran-4-on

(23-3) 6-Iod-2-propoxy-3-propyl-benzopyran-4-on

(23-4) 2-But-2-inyloxy-6-iod-3-propyl-benzopyran-4-on

(23-5) 6-Iod-2-(1-methyl-butoxy)-3-propyl-benzopyran-4-on

(23-6) 2-But-3-enyloxy-6-iod-benzopyran-4-on

(23-7) 3-Butyl-6-iod-2-isopropoxy-benzopyran-4-on

Besonders bevorzugt sind Wirkstoffkombinationen T, worin das Iodochromon (Gruppe 23) der Formel (XV) aus der folgenden Liste ausgewählt ist:

(23-1) 2-Butoxy-6-iod-3-propyl-benzopyran-4-on

(23-2) 2-Ethoxy-6-iod-3-propyl-benzopyran-4-on

Hervorgehoben sind die in der folgenden Tabelle 20 angeführten Wirkstoffkombinationen T:

**Tabelle 20: Wirkstoffkombinationen T**

| Nr. | Carboxamid der Formel (I) | Iodochromon der Formel (XV) |
|---|---|---|
| T-1 | (1-1) N-(3',4'-Dichlor-5-fluor-1,1'-biphenyl-2-yl)-3-(difluormethyl)-1-methyl-1H-pyrazol-4-carboxamid | (23-1) 2-Butoxy-6-iod-3-propyl-benzopyran-4-on |
| T-2 | (1-1) N-(3',4'-Dichlor-5-fluor-1,1'-biphenyl-2-yl)-3-(difluormethyl)-1-methyl-1H-pyrazol-4-carboxamid | (23-2) 2-Ethoxy-6-iod-3-propyl-benzopyran-4-on |
| T-3 | (1-7) N-(4'-Brom-1,1'-biphenyl-2-yl)-4-(difluormethyl)-2-methyl-1,3-thiazol-5-carboxamid | (23-1) 2-Butoxy-6-iod-3-propyl-benzopyran-4-on |
| T-4 | (1-7) N-(4'-Brom-1,1'-biphenyl-2-yl)-4-(difluormethyl)-2-methyl-1,3-thiazol-5-carboxamid | (23-2) 2-Ethoxy-6-iod-3-propyl-benzopyran-4-on |
| T-5 | (1-8) 4-(Difluormethyl)-2-methyl-N-[4'-(trifluormethyl)-1,1'-biphenyl-2-yl]-1,3-thiazol-5-carboxamid | (23-1) 2-Butoxy-6-iod-3-propyl-benzopyran-4-on |
| T-6 | (1-8) 4-(Difluormethyl)-2-methyl-N-[4'-(trifluormethyl)-1,1'-biphenyl-2-yl]-1,3-thiazol-5-carboxamid | (23-2) 2-Ethoxy-6-iod-3-propyl-benzopyran-4-on |
| T-7 | (1-9) N-(4'-Chlor-3'-fluor-1,1'-biphenyl-2-yl)-4-(difluor-methyl)-2-methyl-1,3-thiazol-5-carboxamid | (23-1) 2-Butoxy-6-iod-3-propyl-benzopyran-4-on |
| T-8 | (1-9) N-(4'-Chlor-3'-fluor-1,1'-biphenyl-2-yl)-4-(difluor-methyl)-2-methyl-1,3-thiazol-5-carboxamid | (23-2) 2-Ethoxy-6-iod-3-propyl-benzopyran-4-on |

Die erfindungsgemäßen Wirkstoffkombinationen enthalten neben einem Wirkstoff der Formel (I) mindestens einen Wirkstoff von den Verbindungen der Gruppen (2) bis (23). Sie können darüber hinaus auch weitere fungizid wirksame Zumischkomponenten enthalten.

Wenn die Wirkstoffe in den erfindungsgemäßen Wirkstoffkombinationen in bestimmten Gewichtsverhältnissen vorhanden sind, zeigt sich der synergistische Effekt besonders deutlich. Jedoch können die Gewichtsverhältnisse der Wirkstoffe in den Wirkstoffkombinationen in einem relativ großen Bereich variiert werden. Im Allgemeinen enthalten die erfindungsgemäßen Kombinationen Wirkstoffe der Formel (I) und einen Mischpartner aus einer der Gruppen (2) bis (23) in den in der nachfolgenden Tabelle 21 beispielhaft angegebenen Mischungsverhältnisse.

Die Mischungsverhältnisse basieren auf Gewichtsverhältnissen. Das Verhältnis ist zu verstehen als Wirkstoff der Formel (I) : Mischpartner

**Tabelle 21: Mischungsverhältnisse**

| Mischpartner | bevorzugtes Mischungsverhältnis | besonders bevorzugtes Mischungsverhältnis |
|---|---|---|
| Gruppe (2): Strobilurine | 50 : 1 bis 1 : 50 | 10 : 1 bis 1 : 20 |
| Gruppe (3): Triazole ohne (3-15) | 50 : 1 bis 1 : 50 | 20 : 1 bis 1 : 20 |
| (3-15): Prothioconazole | 50 : 1 bis 1 : 50 | 10 : 1 bis 1 : 20 |
| Gruppe (4): Sulfenamide | 1 : 1 bis 1 : 150 | 1 : 1 bis 1 : 100 |
| Gruppe (5): Valinamide | 50 : 1 bis 1 : 50 | 10 : 1 bis 1 : 20 |
| Gruppe (6): Carboxamide | 50 : 1 bis 1 : 50 | 20 : 1 bis 1 : 20 |
| Gruppe (7): Dithiocarbamate | 1 : 1 bis 1 : 150 | 1 : 1 bis 1 : 100 |
| Gruppe (8): Acylalanine | 10 : 1 bis 1 : 150 | 5 : 1 bis 1 : 100 |
| Gruppe (9): Anilino-pyrimidine | 5 : 1 bis 1 : 50 | 1 : 1 bis 1 : 20 |
| Gruppe (10): Benzimidazole | 10 : 1 bis 1 : 50 | 5 : 1 bis 1 : 20 |
| Gruppe (11): Carbamate ohne (11-1) | 1 : 1 bis 1 : 150 | 1 : 1 bis 1 : 100 |
| (11-1): Diethofencarb | 50 : 1 bis 1:50 | 10 : 1 bis 1:20 |
| Gruppe (12): (12-1)/(12-2)/(12-3) | 1 : 1 bis 1 : 150 | 1 : 5 bis 1 : 100 |
| Gruppe (12): (12-4)/(12-5)/(12-6) | 5 : 1 bis 1 : 50 | 1 : 1 bis 1 : 20 |
| Gruppe (13): Guanidine | 100: 1 bis 1 : 150 | 20 : 1 bis 1 : 100 |
| Gruppe (14): Imidazole | 50: 1 bis 1 : 50 | 10 : 1 bis 1 : 20 |
| Gruppe (15): Morpholine | 50 : 1 bis 1 : 50 | 10 : 1 bis 1 : 20 |
| Gruppe (16): Pyrrole | 50: 1 bis 1 : 50 | 10 : 1 bis 1 : 20 |
| Gruppe (17): Phosphonate | 10 : 1 bis 1 : 150 | 1 : 1 bis 1 : 100 |
| Gruppe (18): Phenylethanamide | 50 : 1 bis 1 : 50 | 10 : 1 bis 1 : 20 |
| (19-1): Acibenzolar-S-methyl | 50 : 1 bis 1 : 50 | 20 : 1 bis 1 : 20 |
| (19-2): Chlorothalonil | 1 : 1 bis 1 : 150 | 1 : 1 bis 1 : 100 |
| (19-3): Cymoxanil | 10 : 1 bis 1 : 50 | 5 : bis 1 : 20 |
| (19-4): Edifenphos | 10 : 1 bis 1 : 50 | 5 : bis 1 : 20 |
| (19-5): Famoxadone | 50 : 1 bis 1 : 50 | 10 : 1 bis 1 : 20 |
| (19-6): Fluazinam | 50 : 1 bis 1 : 50 | 10 : 1 bis 1 : 20 |
| (19-7): Kupferoxychlorid | 1 : 1 bis 1 : 150 | 1 : 5 bis 1 : 100 |
| (19-8): Kupferhydroxid | 1 : 1 bis 1 : 150 | 1 : 5 bis 1 : 100 |
| (19-9): Oxadixyl | 10 : 1 bis 1 : 150 | 5 : 1 bis 1 : 100 |
| (19-10): Spiroxamine | 50 : 1 bis 1 : 50 | 10 : 1 bis 1 : 20 |
| (19-11) Dithianon | 50 : 1 bis 1:50 | 10 : 1 bis 1 : 20 |
| (19-12) Metrafenone | 50 : 1 bis 1 : 50 | 10 : 1 bis 1 : 20 |
| (19-13) Fenamidone | 50 : 1 bis 1 : 50 | 10 : 1 bis 1 : 20 |
| (19-14): 2,3-Dibutyl-6-chlor-thieno-[2,3-d]pyrimidin-4(3H)on | 50 : 1 bis 1 : 50 | 10 : 1 bis 1 : 20 |
| (19-15): Probenazole | 10 : 1 bis 1 : 150 | 5 : 1 bis 1 : 100 |
| (19-16): Isoprothiolane | 10 : 1 bis 1 : 150 | 5:1 bis 1 : 100 |

(fortgesetzt)

| Mischpartner | bevorzugtes Mischungsverhältnis | besonders bevorzugtes Mischungsverhältnis |
|---|---|---|
| (19-17): Kasugamycin | 50 : 1 bis 1 : 50 | 10 : 1 bis 1 : 20 |
| (19-18): Phthalide | 10 : 1 bis 1:150 | 5:1 bis 1:100 |
| (19-19): Ferimzone | 50 : 1 bis 1:50 | 10 : 1 bis 1:20 |
| (19-20): Tricyclazole | 50 : 1 bis 1:50 | 10 : 1 bis 1:20 |
| (19-21): N-({4-[(Cyclopropylamino)-carbonyl] phenyl}sulfonyl)-2-methoxybenzamid | 10 : 1 bis 1 : 150 | 5:1 bis 1:100 |
| (19-22) 2-(4-Chlorphenyl)-N-{2-[3-meth-oxy-4-(prop-2-in-1-yloxy) phenyl]-ethyl}-2-(prop-2-in-1-yloxy)-acetamid | 50 : 1 bis 1 : 50 | 10 : 1 bis 1 : 20 |
| Gruppe (20): (Thio)Harnstoff-Derivate | 50 : 1 bis 1 : 50 | 10 : 1 bis 1 : 20 |
| Gruppe (21): Amide | 50 : 1 bis 1 : 50 | 10 : 1 bis 1 : 20 |
| Gruppe (22): Triazolopyrimidine | 50 : 1 bis 1 : 50 | 10 : 1 bis 1 : 20 |
| Gruppe (23): Iodochromone | 50 : 1 bis 1 : 50 | 10 : 1 bis 1 : 20 |

Das Mischungsverhältnis ist in jedem Fall so zu wählen, dass eine synergistische Mischung erhalten wird. Die Mischungsverhältnisse zwischen der Verbindung der Formel (I) und einer Verbindung aus einer der Gruppen (2) bis (23) kann auch zwischen den einzelnen Verbindungen einer Gruppe variieren. Die erfindungsgemäßen Wirkstoffkombinationen besitzen sehr gute fungizide Eigenschaften und lassen sich zur Bekämpfung von phytopathogenen Pilzen, wie Plasmodiophoromycetes, Oomycetes, Chytridiomycetes, Zygomycetes, Ascomycetes, Basidiomycetes, Deuteromycetes usw. einsetzen. Die erfindungsgemäßen Wirkstoffkombinationen eignen sich besonders gut zur Bekämpfung von Erysiphe graminis, Pyrenophora teres und Leptosphaeria nodorum.

Beispielhaft, aber nicht begrenzend, seien einige Erreger von pilzlichen Erkrankungen, die unter die oben aufgezählten Oberbegriffe fallen, genannt:

Pythium-Arten, wie z.B. *Pythium ultimum*; Phytophthora-Arten, wie z.B. *Phytophthora infestans*;
Pseudoperonospora-Arten, wie z.B. *Pseudoperonospora humuli* oder *Pseudoperonospora cubensis*;
Plasmopara-Arten, wie z.B. *Plasmopara viticola*; Bremia-Arten, wie z.B. *Bremia lactucae*;
Peronospora-Arten, wie z.B. *Peronospora pisi* oder *P. brassicae*; Erysiphe-Arten, wie z.B. *Erysiphe graminis*; Sphaerotheca-Arten, wie z.B. *Sphaerotheca fuliginea*; Podosphaera-Arten, wie z.B. *Podosphaera leucotricha*; Venturia-Arten, wie z.B. *Venturia inaequalis*; Pyrenophora-Arten, wie z.B. *Pyrenophora teres* oder *P. graminea* (Konidienform: Drechslera, Syn: Helminthosporium);
Cochliobolus-Arten, wie z.B. *Cochliobolus sativus* (Konidienform: Drechslera, Syn: Helminthosporium); Uromyces-Arten, wie z.B. *Uromyces appendiculatus*; Puccinia-Arten, wie z.B. *Puccinia recondita*; Sclerotinia-Arten, wie z.B. *Sclerotinia sclerotiorum*; Tilletia-Arten, wie z.B. *Tilletia caries*; Ustilago-Arten, wie z.B. *Ustilago nuda* oder *Ustilago avenae*; Pellicularia-Arten, wie z.B. *Pellicularia sasakii*; Pyricularia-Arten, wie z.B. *Pyricularia oryzae*; Fusarium-Arten, wie z.B. *Fusarium culmorum*; Botrytis-Arten, wie z.B. *Botrytis cinerea*; Septoria-Arten, wie z.B. *Septoria nodorum*; Leptosphaeria-Arten, wie z.B. *Leptosphaeria nodorum*; Cercospora-Arten, wie z.B. *Cercospora canescens*; Alternaria-Arten, wie z.B. *Alternaria brassicae*; Pseudocercosporella-Arten, wie z.B. *Pseudocercosporella herpotrichoides,* Rhizoctonia-Arten, wie z.B. *Rhizoctonia solani.*

Die gute Pflanzenverträglichkeit der Wirkstoffkombinationen in den zur Bekämpfung von Pflanzenkrankheiten notwendigen Konzentrationen erlaubt eine Behandlung der gesamten Pflanzen (oberirdische Pflanzenteile und Wurzeln), von Pflanz- und Saatgut, und des Bodens. Die erfindungsgemäßen Wirkstoffkombinationen können zur Blattapplikation oder auch als Beizmittel eingesetzt werden.

Die erfindungsgemäßen Wirkstoffkombinationen eignen sich auch zur Steigerung des Ernteertrages. Sie sind außerdem mindertoxisch und weisen eine gute Pflanzenverträglichkeit auf.

Erfindungsgemäß können alle Pflanzen und Pflanzenteile behandelt werden. Unter Pflanzen werden hierbei

alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen. Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die erfindungsgemäßen Wirkstoffkombinationen können in Abhängigkeit von ihren jeweiligen physikalischen und/oder chemischen Eigenschaften in die üblichen Formulierungen überführt werden, wie Lösungen, Emulsionen, Suspensionen, Pulver, Stäubemittel, Schäume, Pasten, lösliche Pulver, Granulate, Aerosole, Suspensions-Emulsions-Konzentrate, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe bzw. der Wirkstoffkombinationen mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier- und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Der Wirkstoffgehalt der aus den handelsüblichen Formulierungen bereiteten Anwendungsformen kann in weiten Bereichen variieren. Die Wirkstoffkonzentration der Anwendungsformen zum Bekämpfen tierischer Schädlingen wie Insekten und Akariden kann von 0,0000001 bis zu 95 Gew.-% Wirkstoff, vorzugsweise zwischen 0,0001 und 1 Gew.-% liegen. Die Anwendung geschieht in einer den Anwendungsformen angepassten üblichen Weise.

Die Formulierungen zur Bekämpfung unerwünschter phytopathogener Pilze enthalten im Allgemeinen zwischen 0,1 und 95 Gew.-% Wirkstoffe, vorzugsweise zwischen 0,5 und 90 %.

Die erfindungsgemäßen Wirkstoffkombinationen können als solche, in Form ihrer Formulierungen oder den daraus bereiteten Anwendungsformen, wie gebrauchsfertige Lösungen, emulgierbare Konzentrate, Emulsionen, Suspensionen, Spritzpulver, lösliche Pulver, Stäubemittel und Granulate, angewendet werden. Die Anwendung geschieht in üblicher Weise, z.B. durch Gießen (drenchen), Tröpfchenbewässerung" Verspritzen, Versprühen, Verstreuen, Verstäuben, Verschäumen, Bestreichen, Verstreichen, Trockenbeizen, Feuchtbeizen, Nassbeizen, Schlämmbeizen, Inkrustieren usw.

Die erfindungsgemäßen Wirkstoffkombinationen können in handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen.

Beim Einsatz der erfindungsgemäßen Wirkstoffkombinationen können die Aufwandmengen je nach Applikationsart innerhalb eines größeren Bereichs variiert werden. Bei der Behandlung von Pflanzenteilen liegen die Aufwandmengen an Wirkstoffkombination im allgemeinen zwischen 0,1 und 10 000 g/ha, vorzugsweise zwischen 10 und 1 000 g/ha. Bei der Saatgutbehandlung liegen die Aufwandmengen an Wirkstoffkombination im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoffkombination im allgemeinen zwischen 0,1 und 10 000 g/ha, vorzugsweise zwischen 1 und 5 000 g/ha.

Die Wirkstoffkombinationen können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln. Die gute fungizide Wirkung der erfindungsgemäßen Wirkstoffkombinationen geht aus den nachfolgenden Beispielen hervor. Während die einzelnen Wirkstoffe in der fungiziden Wirkung Schwächen aufweisen, zeigen die Kombinationen eine Wirkung, die über eine einfache Wirkungssummierung hinausgeht.

Ein synergistischer Effekt liegt bei Fungiziden immer dann vor, wenn die fungizide Wirkung der Wirkstoffkombinationen größer ist als die Summe der Wirkungen der einzeln applizierten Wirkstoffe.

Die zu erwartende fungizide Wirkung für eine gegebene Kombination zweier Wirkstoffe kann nach S.R. Colby ("Calculating Synergistic and Antagonistic Responses of Herbicide Combinations", Weeds 1967, 15, 20-22) wie folgt berechnet werden:

Wenn

X den *Wirkungsgrad* beim Einsatz des Wirkstoffes A in einer Aufwandmenge von *m g/ha* bedeutet,

Y den *Wirkungsgrad* beim Einsatz des Wirkstoffes B in einer Aufwandmenge *von n g/ha* bedeutet und

E den *Wirkungsgrad* beim Einsatz der Wirkstoffe A und B in Aufwandmengen von *m und n g/ha* bedeutet,

dann ist

$$E = X + Y - \frac{X \times Y}{100}$$

Dabei wird der Wirkungsgrad in % ermittelt. Es bedeutet 0 % ein Wirkungsgrad, der demjenigen der Kontrolle entspricht, während ein Wirkungsgrad von 100 % bedeutet, dass kein Befall beobachtet wird.

Ist die tatsächliche fungizide Wirkung größer als berechnet, so ist die Kombination in ihrer Wirkung überadditiv, d.h. es liegt ein synergistischer Effekt vor. In diesem Fall muss der tatsächlich beobachtete Wirkungsgrad größer sein als der aus der oben angeführten Formel errechnete Wert für den erwarteten Wirkungsgrad (E).

Die Erfindung wird durch die folgenden Beispiele veranschaulicht. Die Erfindung ist jedoch nicht auf die Beispiele limitiert.

**Anwendungsbeispiele**

In den nachfolgend aufgeführten Anwendungsbeispielen wurden jeweils Mischungen von folgenden Carboxamiden der allgemeinen Formel (I) (Gruppe 1) mit den jeweils angegebenen Mischungspartnern (Strukturformeln siehe oben) getestet.

Eingesetzte Carboxamide der Formel (I):

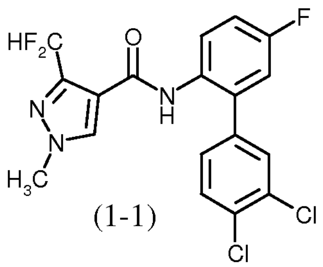

(1-1)

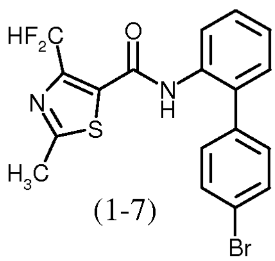

(1-7)

Beispiel A

**Pyrenophora teres-Test (Gerste) / kurativ**

| | |
|---|---|
| Lösungsmittel : | 50 Gewichtsteile N,N-Dimethylacetamid |
| Emulgator : | 1 Gewichtsteil Alkylarylpolyglykolether |

Zur Herstellung einer zweckmäßigen Wirkstoffzubereitung vermischt man 1 Gewichtsteil Wirkstoff oder Wirkstoffkombination mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer Konidiensuspension von *Pyrenophora teres* besprüht. Die Pflanzen verbleiben 48 Stunden bei 20°C und 100 % relativer Luftfeuchtigkeit in einer Inkubationskabine. Anschließend werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Aus der nachfolgenden Tabelle geht eindeutig hervor, dass die gefundene Wirkung der erfindungsgemäßen Wirkstoffkombination größer ist als die berechnete, d.h. dass ein synergistischer Effekt vorliegt.

Tabelle A

| **Pyrenophora teres-Test (Gerste) / kurativ** | | | |
|---|---|---|---|
| Wirkstoffe | Aufwandmenge an Wirkstoff in g/ha | Wirkungsgrad in % | |
| | | gef.* | ber.** |
| **(1-1)** | 25 | 43 | |
| (2-2) Fluoxastrobin | 25 | 0 | |
| (3-17) Tebuconazole | 25 | 29 | |
| **(1-1)** + (2-2) Fluoxastrobin (1: 1) | 25 + 25 | 71 | 43 |
| **(1-1)** + (3-17) Tebuconazole (1: 1) | 25 + 25 | 71 | 60 |
| * gef. = gefundene Wirkung<br>** ber. = nach der Colby-Formel berechnete Wirkung | | | |

Beispiel B

**Erysiphe-Test (Gerste) / protektiv**

| | |
|---|---|
| Lösungsmittel : | 50 Gewichtsteile N,N-Dimethylacetamid |
| Emulgator : | 1 Gewichtsteil Alkylarylpolyglykolether |

Zur Herstellung einer zweckmäßigen Wirkstoffzubereitung vermischt man 1 Gewichtsteil Wirkstoff oder Wirkstoffkombination mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelags werden die Pflanzen mit Sporen von *Erysiphe graminis f.sp. hordei* bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

6 Tage nach der Inokulation erfolgt die Auswertung. Dabei bedeutet 0 % ein Wirkungsgrad, der demjenigen

der Kontrolle entspricht, während ein Wirkungsgrad von 100 % bedeutet, dass kein Befall beobachtet wird.

Aus der nachfolgenden Tabelle geht eindeutig hervor, dass die gefundene Wirkung der erfindungsgemäßen Wirkstoffkombination größer ist als die berechnete, d.h. dass ein synergistischer Effekt vorliegt.

Tabelle B

| **Erysiphe-Test (Gerste) / protektiv** | | | |
|---|---|---|---|
| Wirkstoffe | Aufwandmenge an Wirkstoff in g/ha | Wirkungsgrad in % | |
| | | gef.* | ber.** |
| **(1-1)** | 12,5 | 0 | |
| (2-4) Trifloxystrobin | 12,5 | 78 | |
| (3-15) Prothioconazole | 12,5 | 67 | |
| **(1-1)** + (2-4) Trifloxystrobin (1: 1) | 12,5 + 12,5 | 94 | 78 |
| **(1-1)** + (3-15) Prothioconazole (1:1) | 12,5 + 12,5 | 89 | 67 |
| * gef. = gefundene Wirkung<br>** ber. = nach der Colby-Formel berechnete Wirkung | | | |

<u>Beispiel C</u>

**Puccinia-Test (Weizen) / kurativ**

| | |
|---|---|
| Lösungsmittel: | 50 Gewichtsteile N,N-Dimethylacetamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer Kondiensuspension von *Puccinia recondita* besprüht. Die Pflanzen verbleiben 48 Stunden bei 20°C und 100 % relativer Luftfeuchte in einer Inkubationskabine.

Anschließend werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Aus der nachfolgenden Tabelle geht eindeutig hervor, dass die gefundene Wirkung der erfindungsgemäßen Wirkstoffkombination größer ist als die berechnete, d.h. dass ein synergistischer Effekt vorliegt.

Tabelle C

| **Puccinia-Test (Weizen) / kurativ** | | | |
|---|---|---|---|
| Wirkstoffe | Aufwandmenge an Wirkstoff in g/ha | Wirkungsgrad in % | |
| | | gef.* | ber.** |
| **(1-1)** | 62,5 | 22 | |
| (19-10) Spiroxamine | 62,5 | 0 | |
| (6-14) | 62,5 | 44 | |
| (6-11) | 62,5 | 0 | |
| (2-11) Picoxystrobin | 62,5 | 78 | |
| **(1-1)** + (19-10) Spiroxamine (1:1) | 62,5 + 62,5 | 100 | 22 |

(fortgesetzt)

| Puccinia-Test (Weizen) / kurativ | | | |
|---|---|---|---|
| Wirkstoffe | Aufwandmenge an Wirkstoff in g/ha | Wirkungsgrad in % | |
| | | gef.* | ber.** |
| **(1-1)** + (6-14) (1:1) | 62,5 + 62,5 | 67 | 57 |
| **(1-1)** + (6-11) (1:1) | 62,5 + 62,5 | 44 | 22 |
| **(1-1)** + (2-11) Picoxystrobin (1:1) | 62,5 + 62,5 | 89 | 83 |
| * gef. = gefundene Wirkung<br>** ber. = nach der Colby-Formel berechnete Wirkung | | | |

Beispiel D

**Gibberella zeae-Test (Gerste) / kurativ**

Lösungsmittel: 50 Gewichtsteile N,N-Dimethylacetamid
Emulgator: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer Konidien-Suspension von Gibberella zeae besprüht. Die Pflanzen verbleiben 24 Stunden bei 22°C und 100 % relativer Luftfeuchtigkeit in einer Inkubationskabine. Anschließend besprüht man die Pflanzen mit der Wirkstoffzubereitung in der angegebenen Aufwandmenge. Nach Antrocknen des Spritzbelages verbleiben die Pflanzen in einem Gewächshaus unter lichtdurchlässigen Inkubationshauben bei einer Temperatur von ca. 22°C und einer relativen Luftfeuchtigkeit von ca. 100 %.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Aus der nachfolgenden Tabelle geht eindeutig hervor, dass die gefundene Wirkung der erfindungsgemäßen Wirkstoffkombination größer ist als die berechnete, d.h. dass ein synergistischer Effekt vorliegt.

Tabelle D

| Gibberella zeae-Test (Gerste) / kurativ | | | |
|---|---|---|---|
| Wirkstoffe | Aufwandmenge an Wirkstoff in g/ha | Wirkungsgrad in % | |
| | | gef.* | ber.** |
| **(1-1)** | 62,5 | 40 | |
| (2-12) Pyraclostrobin | 62,5 | 80 | |
| (3-12) Epoxyconazole | 62,5 | 0 | |
| **(1-1)** + (2-12) Pyraclostrobin (1:1) | 62,5 + 62,5 | 90 | 88 |
| **(1-1)** + (3-12) Epoxyconazole (1:1) | 62,5 + 62,5 | 60 | 40 |
| * gef. = gefundene Wirkung<br>** ber. = nach der Colby-Formel berechnete Wirkung | | | |

Beispiel E

**Sphaerotheca fuliginea -Test (Gurke) / protektiv**

Lösungsmittel: 24,5 Gewichtsteile Aceton
24,5 Gewichtsteile Dimethylacetamid
Emulgator: 1 Gewichtsteil Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von *Sphaerotheca fuliginea* inokuliert.

Die Pflanzen werden dann bei ca. 23°C und einer relativen Luftfeuchtigkeit von ca. 70% im Gewächshaus aufgestellt.

7 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

Aus der nachfolgenden Tabelle geht eindeutig hervor, dass die gefundene Wirkung der erfindungsgemäßen Wirkstoffkombination größer ist als die berechnete, d.h. dass ein synergistischer Effekt vorliegt.

Tabelle E

| **Sphaerotheca fuliginea-Test (Gurke) / protektiv** | | | |
|---|---|---|---|
| Wirkstoffe | Aufwandmenge an Wirkstoff in g/ha | Wirkungsgrad in % | |
| | | gef.* | ber.** |
| **(1-1)** | 4 | 30 | |
| | 2 | 36 | |
| | 1 | 16 | |
| | 0,5 | 0 | |
| **(1-7)** | 2 | 0 | |
| | 1 | 0 | |
| | 0,5 | 0 | |
| (2-1) Azoxystrobin | 0,5 | 20 | |
| (2-2) Fluoxastrobin | 1 | 0 | |
| (2-4) Trifloxystrobin | 2 | 10 | |
| (2-12) Pyraclostrobin | 2 | 0 | |
| (3-15) Prothioconazole | 1 | 43 | |
| (3-17) Tebuconazole | 1 | 10 | |
| (3-21) Bitertanol | 1 | 0 | |
| (4-2) Tolylfluanid | 20 | 0 | |
| (6-6) Fenhexamid | 20 | 0 | |
| (6-14) Penthiopyrad | 4 | 0 | |
| (7-1) Mancozeb | 20 | 0 | |
| (7-4) Propineb | 20 | 11 | |
| (9-3) Pyrimethanil | 20 | 0 | |
| (12-4) Iprodione | 20 | 0 | |
| (19-2) Chlorothalonil | 20 | 0 | |
| (19-10) Spiroxamine | 20 | 0 | |
| (22-1) | 2 | 11 | |

(fortgesetzt)

| Sphaerotheca fuliginea-Test (Gurke) / protektiv | | | |
|---|---|---|---|
| Wirkstoffe | Aufwandmenge an Wirkstoff in g/ha | Wirkungsgrad in % | |
| | | gef.* | ber.** |
| (22-2) | 1 | 22 | |
| **(1-1)** + (2-1) Azoxystrobin (1: 1) | 0,5 + 0,5 | 87 | 20 |
| **(1-7)** + (2-1) Azoxystrobin (1:1) | 0,5 + 0,5 | 63 | 20 |
| **(1-1)** + (2-2) Fluoxastrobin (1: 1) | 1 + 1 | 95 | 16 |
| **(1-7)** + (2-2) Fluoxastrobin (1: 1) | 1 + 1 | 92 | 0 |
| **(1-1)** + (2-4) Trifloxystrobin (1: 1) | 2 + 2 | 57 | 42 |
| **(1-7)** + (2-4) Trifloxystrobin (1:1) | 2 + 2 | 93 | 10 |
| **(1-1)** + (2-12) Pyraclostrobin (1:1) | 2 + 2 | 53 | 36 |
| **(1-1)** + (3-15) Prothioconazole (1:1) | 1 + 1 | 70 | 52 |
| **(1-1)** + (3-17) Tebuconazole (1: 1) | 1 + 1 | 90 | 24 |
| **(1-1)** + (3-21) Bitertanol (1:1) | 1 + 1 | 50 | 16 |
| **(1-1)** + (4-2) Tolylfluanid (1:10) | 2 + 20 | 98 | 36 |
| **(1-1)** + (6-6) Fenhexamid (1:10) | 2 + 20 | 85 | 36 |
| **(1-1)** + (6-14) Penthiopyrad (1: 1) | 4 + 4 | 82 | 30 |
| **(1-1)** + (7-1) Mancozeb (1:10) | 2 + 20 | 93 | 36 |
| **(1-1)** + (7-4) Propineb (1:10) | 2 + 20 | 65 | 43 |
| **(1-1)** + (9-3) Pyrimethanil (1:10) | 2 + 20 | 96 | 36 |
| **(1-1)** + (12-4) Iprodione (1:10) | 2 + 20 | 74 | 36 |
| **(1-1)** + (19-2) Chlorothalonil (1:10) | 2 + 20 | 91 | 36 |
| **(1-1)** + (19-10) Spiroxamine (1:10) | 2 + 20 | 100 | 36 |
| **(1-1)** + (22-1) (1:1) | 2+2 | 67 | 43 |
| **(1-1)** + (22-2) (1:1) | 1 + 1 | 94 | 34 |
| * gef. = gefundene Wirkung<br>** ber. = nach der Colby-Formel berechnete Wirkung | | | |

Beispiel F

**Alternaria solani - Test (Tomate) / protektiv**

| | |
|---|---|
| Lösungsmittel: | 24,5 Gewichtsteile Aceton |
| | 24,5 Gewichtsteile Dimethylacetamid |
| Emulgator: | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von *Alternaria solani* inokuliert.

Die Pflanzen werden dann in einer Inkubationskabine bei ca. 20°C und 100% relativer Luftfeuchtigkeit aufgestellt.

3 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

Aus der nachfolgenden Tabelle geht eindeutig hervor, dass die gefundene Wirkung der erfindungsgemäßen Wirkstoffkombination größer ist als die berechnete, d.h. dass ein synergistischer Effekt vorliegt.

Tabelle F

| **Alternaria solani - Test (Tomate) / protektiv** | | | |
|---|---|---|---|
| Wirkstoffe | Aufwandmenge an Wirkstoff in g/ha | Wirkungsgrad in % | |
| | | gef.* | ber.** |
| **(1-1)** | 1<br>0,5 | 61<br>42 | |
| **(1-7)** | 1<br>0,5 | 63<br>28 | |
| (2-3) | 0,5 | 22 | |
| (3-3) Propiconazole | 0,5 | 3 | |
| (5-3) Benthiavalicarb | 1 | 5 | |
| (8-4) Metalaxyl-M | 0,5 | 7 | |
| (8-5) Benalaxyl-M | 0,5 | 14 | |
| **(1-7)** + (2-3) (1:1) | 0,5 + 0,5 | 67 | 44 |
| **(1-7)** + (3-3) Propiconazole (1: 1) | 0,5 + 0,5 | 56 | 30 |
| **(1-1)** + (5-3) Benthiavalicarb (1:1) | 1 + 1 | 77 | 63 |
| **(1-1)** + (8-4) Metalaxyl-M (1:1) | 0,5 + 0,5 | 62 | 46 |
| **(1-1)** + (8-5) Benalaxyl-M (1:1) | 0,5 + 0,5 | 67 | 50 |
| * gef. = gefundene Wirkung<br>** ber. = nach der Colby-Formel berechnete Wirkung | | | |

Beispiel G

**Phytophthora infestans - Test (Tomate) / protektiv**

| | |
|---|---|
| Lösungsmittel: | 24,5 Gewichtsteile Aceton<br>24,5 Gewichtsteile Dimethylacetamid |
| Emulgator: | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von *Phytophthora infestans* inokuliert. Die Pflanzen werden dann in einer Inkubationskabine bei ca. 20°C und 100% relativer Luftfeuchtigkeit aufgestellt.

3 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

Aus der nachfolgenden Tabelle geht eindeutig hervor, dass die gefundene Wirkung der erfindungsgemäßen Wirkstoffkombination größer ist als die berechnete, d.h. dass ein synergistischer Effekt vorliegt.

Tabelle G

| Phytophthora infestans - Test (Tomate) / protektiv | | | |
|---|---|---|---|
| Wirkstoffe | Aufwandmenge an Wirkstoff in g/ha | Wirkungsgrad in % gef.* | ber.** |
| **(1-1)** | 10<br>5<br>1<br>0,5 | 0<br>0<br>0<br>0 | |
| (4-2) Tolylfluanid | 10 | 0 | |
| (5-1) Iprovalicarb | 10<br>5 | 64<br>61 | |
| (5-3) Benthiavalicarb | 0,5 | 56 | |
| (19-13) Fenamidone | 0,5 | 41 | |
| **(1-1)** + (4-2) Tolylfluanid (1:10) | 1 + 10 | 51 | 0 |
| **(1-1)** + (5-1) Iprovalicarb (1:1) | 10 + 10<br>5+5 | 88<br>77 | 64<br>61 |
| **(1-1)** + (5-3) Benthiavalicarb (1:1) | 0,5 + 0,5 | 73 | 56 |
| **(1-1)** + (19-13) Fenamidone (1:1) | 0,5 + 0,5 | 51 | 41 |
| * gef. = gefundene Wirkung<br>** ber. = nach der Colby-Formel berechnete Wirkung | | | |

Beispiel H

**Botrytis cinerea - Test (Bohne) / protektiv**

| | |
|---|---|
| Lösungsmittel: | 24,5 Gewichtsteile Aceton |
| | 24,5 Gewichtsteile Dimethylacetamid |
| Emulgator: | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit *Botrytis cinerea* bewachsene Agarstückchen aufgelegt. Die inokulierten Pflanzen werden in einer abgedunkelten Kammer bei ca. 20°C und 100% relativer Luftfeuchtigkeit aufgestellt.

2 Tage nach der Inokulation wird die Größe der Befallsflecken auf den Blättern ausgewertet. Dabei bedeutet 0% ein Wirkungsgrad, der demjenigen der Kontrolle entspricht, während ein Wirkungsgrad von 100% bedeutet, dass kein Befall beobachtet wird.

Aus der nachfolgenden Tabelle geht eindeutig hervor, dass die gefundene Wirkung der erfindungsgemäßen Wirkstoffkombination größer ist als die berechnete, d.h. dass ein synergistischer Effekt vorliegt.

Tabelle H

| Botrytis cinerea - Test (Bohne) / protektiv | | | |
|---|---|---|---|
| Wirkstoffe | Aufwandmenge an Wirkstoff in g/ha | Wirkungsgrad in % | |
| | | gef.* | ber.** |
| **(1-1)** | 5 | 54 | |
| (9-3) Pyrimethanil | 5 | 4 | |
| (12-4) Iprodione | 5 | 13 | |
| **(1-1)** + (9-3) Pyrimethanil (1:1) | 5+5 | 92 | 56 |
| **(1-1)** + (12-4) Iprodione (1:1) | 5+5 | 100 | 60 |
| * gef. = gefundene Wirkung<br>** ber. = nach der Colby-Formel berechnete Wirkung | | | |

Beispiel I

**Alternaria mali -Test (in vitro) / Mikrotiterplatten**

Der Microtest wird in Mikrotiterplatten mit Potato-Dextrose Broth (PDB) als flüssigem Versuchsmedium durchgeführt. Die Anwendung der Wirkstoffe erfolgt als technisches a.i., gelöst in Aceton.

Zur Inokulation wird eine Sporensuspension von *Alternaria mali* verwendet. Nach 5 Tagen Inkubation bei Dunkelheit und unter Schütteln (10 Hz) wird die Lichtdurchlässigkeit in jeder gefüllten Kavität der Mikrotiterplatten mit Hilfe eines Spectrophotometers ermittelt.

Dabei bedeutet 0 % ein Wirkungsgrad, der dem Wachstum in den Kontrollen entspricht, während ein Wirkungsgrad von 100 % bedeutet, dass kein Pilzwachstum beobachtet wird.

Aus der nachfolgenden Tabelle geht eindeutig hervor, dass die gefundene Wirkung der erfindungsgemäßen Wirkstoffkombination größer ist als die berechnete, d.h. dass ein synergistischer Effekt vorliegt.

Tabelle I

| Alternaria mali -Test (in vitro) / Mikrotiterplatten | | | |
|---|---|---|---|
| Wirkstoffe | Aufwandmenge an Wirkstoff in ppm | Wirkungsgrad in % | |
| | | gef.* | ber.** |
| **(1-1)** | 0,03<br>0,003 | 51<br>25 | |
| (10-3) Carbendazim | 0,03 | 15 | |
| (19-3) Fenamidone | 0,003 | 2 | |
| (20-1) Pencycuron | 0,003 | 11 | |
| **(1-1)** + (10-3) Carbendazim (1:1) | 0,03+0,03 | 79 | 59 |
| **(1-1)** + (19-3) Fenamidone (1:1) | 0,003+0,003 | 35 | 27 |
| **(1-1)** + (20-1) Pencycuron (1:1) | 0,003+0,003 | 67 | 33 |
| * gef. = gefundene Wirkung<br>** ber. = nach der Colby-Formel berechnete Wirkung | | | |

Beispiel J

**Rhizoctonia solani-Test (in vitro) / Mikrotiterplatten**

Der Microtest wird in Mikrotiterplatten mit Potato-Dextrose Broth (PDB) als flüssigem Versuchsmedium durchgeführt. Die Anwendung der Wirkstoffe erfolgt als technisches a.i., gelöst in Aceton.

Zur Inokulation wird eine Myzelsuspension von *Rhizoctonia solani* verwendet. Nach 5 Tagen Inkubation bei

Dunkelheit und unter Schütteln (10 Hz) wird die Lichtdurchlässigkeit in jeder gefüllten Kavität der Mikrotiterplatten mit Hilfe eines Spectrophotometers ermittelt.

Dabei bedeutet 0 % ein Wirkungsgrad, der dem Wachstum in den Kontrollen entspricht, während ein Wirkungsgrad von 100 % bedeutet, dass kein Pilzwachstum beobachtet wird.

Aus der nachfolgenden Tabelle geht eindeutig hervor, dass die gefundene Wirkung der erfindungsgemäßen Wirkstoffkombination größer ist als die berechnete, d.h. dass ein synergistischer Effekt vorliegt.

Tabelle J

| **Rhizoctonia solani-Test (in vitro) / Mikrotiterplatten** | | | |
|---|---|---|---|
| Wirkstoffe | Aufwandmenge an Wirkstoff in ppm | Wirkungsgrad in % | |
| | | gef.* | ber.** |
| **(1-1)** | 0,3<br>0,1 | 80<br>40 | |
| (17-1) Fosetyl-Al | 0,3 | 24 | |
| (11-2) Propamocarb | 0,1 | 25 | |
| **(1-1)** + (17-1) Fosetyl-Al (1:1) | 0,3+0,3 | 98 | 85 |
| **(1-1)** + (11-2) Propamocarb (1:1) | 0,1+0,1 | 88 | 55 |
| * gef. = gefundene Wirkung<br>** ber. = nach der Colby-Formel berechnete Wirkung | | | |

Beispiel K

**Septoria tritici-Test (in vitro) / Mikrotiterplatten**

Der Microtest wird in Mikrotiterplatten mit Potato-Dextrose Broth (PDB) als flüssigem Versuchsmedium durchgeführt. Die Anwendung der Wirkstoffe erfolgt als technisches a.i., gelöst in Aceton.

Zur Inokulation wird eine Sporensuspension von *Septoria tritici* verwendet. Nach 7 Tagen Inkubation bei Dunkelheit und unter Schütteln (10 Hz) wird die Lichtdurchlässigkeit in jeder gefüllten Kavität der Mikrotiterplatten mit Hilfe eines Spectrophotometers ermittelt.

Dabei bedeutet 0 % ein Wirkungsgrad, der dem Wachstum in den Kontrollen entspricht, während ein Wirkungsgrad von 100 % bedeutet, dass kein Pilzwachstum beobachtet wird.

Aus der nachfolgenden Tabelle geht eindeutig hervor, dass die gefundene Wirkung der erfindungsgemäßen Wirkstoffkombination größer ist als die berechnete, d.h. dass ein synergistischer Effekt vorliegt.

Tabelle K

| **Septoria tritici-Test (in vitro) / Mikrotiterplatten** | | | |
|---|---|---|---|
| Wirkstoffe | Aufwandmenge an Wirkstoff in ppm | Wirkungsgrad in % | |
| | | gef.* | ber.** |
| **(1-1)** | 0,01 | 15 | |
| (14-3) Triazoxide | 0,01 | 29 | |
| **(1-1)** + (14-3) Triazoxide (1:1) | 0,01+0,01 | 69 | 40 |
| * gef. = gefundene Wirkung<br>** ber. = nach der Colby-Formel berechnete Wirkung | | | |

Beispiel L

**Sphaerotheca fuliginea - Test (Gherkin) / protektiv**

Zur Herstellung einer zweckmäßigen Wirkstoffzubereitung wird die zu testende Substanz in einer Mischung aus Aceton/Tween/Wasser homogenisiert. Die Suspension wird mit Wasser auf die gewünschte Konzentration verdünnt.

Gherkin Pflanzen (Vert petit de Paris Varietät) werden in Startercups auf 50/50 Torfboden-Puzzolanerde-Substrat ausgesät und bei 20°C/23°C angezogen. Im 2-Blatt-Stadium werden die Pflanzen mit der Wirkstoffzubereitung in der angegebenen Aufwandmenge besprüht.

Zur Prüfung auf protektive Wirksamkeit werden die Pflanzen nach 24 h mit einer wässrigen Sporensuspension von *Sphaerotheca fuliginea* (100000 Sporen/ml) besprüht. Die Pflanzen verbleiben anschließen bei 20°C/25°C und 60/70 % relativer Luftfeuchtigkeit.

21 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Aus der nachfolgenden Tabelle geht eindeutig hervor, dass die gefundene Wirkung der erfindungsgemäßen Wirkstoffkombination größer ist als die berechnete, d.h. dass ein synergistischer Effekt vorliegt.

Tabelle L

| **Sphaerotheca fuliginea - Test (Gherkin) / protectiv** | | | |
|---|---|---|---|
| Wirkstoffe | Aufwandmenge an Wirkstoff in ppm | Wirkungsgrad in % | |
| | | gef.* | ber.** |
| **(1-1)** | 8 | 60 | |
| (6-2) Boscalid | 8 | 50 | |
| **(1-1)** + (6-2) Boscalid (1:1) | 8+8 | 98 | 80 |
| * gef. = gefundene Wirkung<br>** ber. = nach der Colby-Formel berechnete Wirkung | | | |

[1] Synergistische fungizide Wirkstoffkombinationen enthaltend ein Carboxamid der allgemeinen Formel (I) (Gruppe 1)

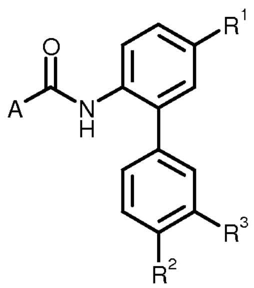

(I)

in welcher

$R^1$ für Wasserstoff oder Fluor steht,

$R^2$ für Halogen, $C_1$-$C_3$-Alkyl, $C_1$-$C_3$-Halogenalkyl mit 1 bis 7 Fluor-, Chlor- und/ oder Bromatomen, $C_1$-$C_3$-Alkoxy, $C_1$-$C_3$-Halogenalkoxy mit 1 bis 7 Fluor-, Chlor- und/ oder Bromatomen oder für -C($R^4$)=N-O$R^5$ steht,

$R^3$ für Wasserstoff, Halogen, $C_1$-$C_3$-Alkyl oder $C_1$-$C_3$-Halogenalkyl mit 1 bis 7 Fluor-, Chlor- und/ oder Bromatomen steht,

$R^4$ für Wasserstoff oder Methyl steht,

$R^5$ für $C_1$-$C_5$-Alkyl, $C_1$-$C_5$-Alkenyl oder $C_1$-$C_5$-Alkinyl steht,

A für einen der folgenden Reste A1 bis A7 steht:

A1 A2 A3 A4 A5 A6 A7

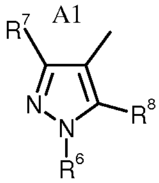 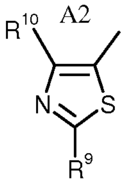 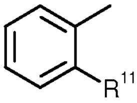 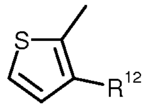 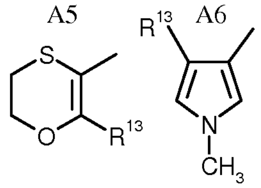 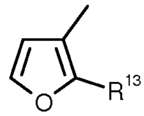

$R^6$ für $C_1$-$C_3$-Alkyl steht,

$R^7$ für Wasserstoff, Halogen, $C_1$-$C_3$-Alkyl oder $C_1$-$C_3$-Halogenalkyl mit 1 bis 7 Fluor-, Chlor- und/ oder Bromatomen steht,

$R^8$ für Wasserstoff, Halogen oder $C_1$-$C_3$-Alkyl steht,

$R^9$ für Wasserstoff, Halogen, $C_1$-$C_3$-Alkyl, Amino, Mono- oder Di($C_1$-$C_3$-alkyl) amino steht,

$R^{10}$ für Wasserstoff, Halogen, $C_1$-$C_3$-Alkyl oder $C_1$-$C_3$-Halogenalkyl mit 1 bis 7 Fluor-, Chlor- und/ oder Bromatomen steht,

$R^{11}$ für Halogen, $C_1$-$C_3$-Alkyl oder $C_1$-$C_3$-Halogenalkyl mit 1 bis 7 Fluor-, Chlor- und/ oder Bromatomen steht,

$R^{12}$ für Halogen, $C_1$-$C_3$-Alkyl oder $C_1$-$C_3$-Halogenalkyl mit 1 bis 7 Fluor-, Chlor- und/ oder Bromatomen steht,

$R^{13}$ für Wasserstoff, Halogen, $C_1$-$C_3$-Alkyl oder $C_1$-$C_3$-Halogenalkyl mit 1 bis 7 Fluor-, Chlor- und/ oder Bromatomen steht,

und mindestens einen Wirkstoff, der aus den folgenden Gruppen (2) bis (23) ausgewählt ist:

Gruppe (2) Strobilurine der allgemeinen Formel (II)

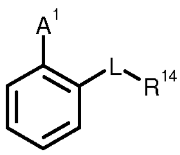 (II)

in welcher

$A^1$ für eine der Gruppen

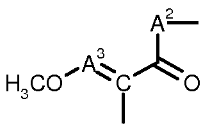 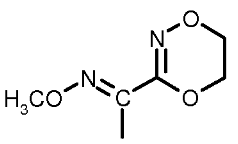 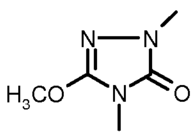 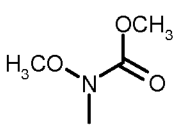

steht,

$A^2$ für NH oder O steht,

$A^3$ für N oder CH steht,

L für eine der Gruppen

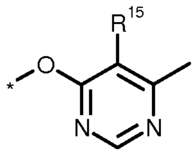 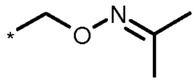 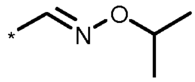 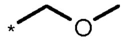 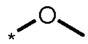

steht, wobei die Bindung, die mit einem Stern (*) markiert ist an den Phenylring gebunden ist,

$R^{14}$ für jeweils gegebenenfalls einfach oder zweifach, gleich oder verschieden durch Chlor, Cyano, Methyl oder Trifluormethyl substituiertes Phenyl, Phenoxy oder Pyridinyl, oder für 1-(4-Chlorphenyl)-pyrazol-3-yl oder für 1,2-Propandion-bis(O- methyloxim)-1-yl steht,

$R^{15}$ für Wasserstoff oder Fluor steht;

Gruppe (3) Triazole der allgemeinen Formel (III)

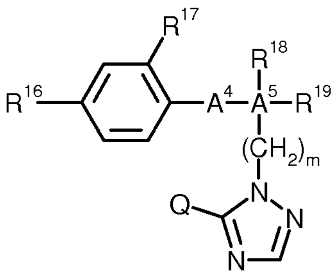

(III)

in welcher

Q für Wasserstoff oder SH steht,

m für 0 oder 1 steht,

$R^{16}$ für Wasserstoff, Fluor, Chlor, Phenyl oder 4-Chlor-phenoxy steht,

$R^{17}$ für Wasserstoff oder Chlor steht,

$A^4$ für eine direkte Bindung, $-CH_2-$, $-(CH_2)_2-$ oder -O- steht,

$A^4$ außerdem für $*-CH_2-CHR^{20}-$ oder $*-CH=CR^{20}-$ steht, wobei die mit * markierte Bindung mit dem Phenylring verknüpft ist, und

$R^{18}$ und $R^{20}$ dann zusammen für $-CH_2-CH_2-CH[CH(CH_3)_2]-$ oder $-CH_2-CH_2-C(CH_3)_2-$ stehen,

$A^5$ für C oder Si (Silizium) steht,

$A^4$ außerdem für $-N(R^{20})-$ steht und $A^5$ außerdem zusammen mit $R^{18}$ und $R^{19}$ für die Gruppe $C=N-R^{21}$ steht, wobei $R^{20}$ und $R^{21}$ dann zusammen für die Gruppe

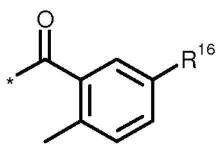

stehen, wobei die mit * markierte Bindung mit $R^{20}$ verbunden ist,

$R^{18}$ für Wasserstoff, Hydroxy oder Cyano steht,

$R^{19}$ für 1-Cyclopropylethyl, 1-Chlorcyclopropyl, $C_1$-$C_4$-Alkyl, $C_1$-$C_6$-Hydroxyalkyl, $C_1$- $C_4$-Alkylcarbonyl, $C_1$-$C_2$-Halogenalkoxy-$C_1$-$C_2$-alkyl, Trimethylsilyl-$C_1$-$C_2$-alkyl, Monofluorphenyl, oder Phenyl steht,

$R^{18}$ und $R^{19}$ außerdem zusammen für -O-$CH_2$-CH($R^{21}$)-O-, -O-$CH_2$-CH($R^{21}$)-$CH_2$-, oder -O- CH(2-Chlorphenyl)- stehen,

$R^{21}$ für Wasserstoff, $C_1$-$C_4$-Alkyl oder Brom steht;

Gruppe (4) Sulfenamide der allgemeinen Formel (IV)

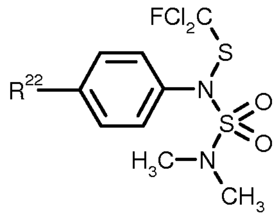

(IV)

in welcher $R^{22}$ für Wasserstoff oder Methyl steht;

Gruppe (5) Valinamide ausgewählt aus

(5-1) Iprovalicarb

(5-2) *N*[1]-[2-(4-{[3-(4-chlorophenyl)-2-propynyl]oxy}-3-methoxyphenyl)ethyl]-*N*[2]-(methylsulfonyl)-D-valinamid;

(5-3) Benthiavalicarb

Gruppe (6) Carboxamide der allgemeinen Formel (V)

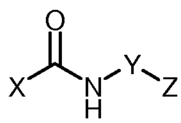

(V)

in welcher

X für 2-Chlor-3-pyridinyl, für 1-Methylpyrazol-4-yl, welches in 3-Position durch Me- thyl oder Trifluormethyl und in 5-Position durch Wasserstoff oder Chlor substituiert ist, für 4-Ethyl-2-ethylamino-1,3-thiazol-5-yl, für 1-Methyl-cyclohexyl, für 2,2-Di- chlor-1-ethyl-3-methyl-cyclopropyl, für 2-Fluor-2-propyl, oder für Phenyl steht, wel- ches einfach bis dreifach, gleich oder verschieden durch Chlor oder Methyl substitu- iert ist, steht,

X außerdem für 3,4-Dichlor-isothiazol-5-yl, 5,6-Dihydro-2-methyl-1,4-oxathiin-3-yl, 4- Methyl-1,2,3-thiadiazol-5-yl, 4,5-Dimethyl-2-trimethylsilyl-thiophen-3-yl, 1-Methyl- pyrrol-3-yl, welches in 4-Position durch Methyl oder Trifluormethyl und in 5-Posi- tion durch Wasserstoff oder Chlor substituiert ist, steht,

Y für eine direkte Bindung, gegebenenfalls durch Chlor, Cyano oder Oxo substituiertes $C_1$-$C_6$-Alkandiyl (Alkylen) oder Thiophendiyl steht,

Y außerdem für $C_2$-$C_6$-Alkendiyl (Alkenylen) steht,

Z für Wasserstoff oder die Gruppe

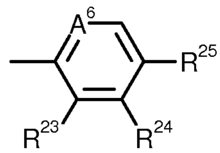

steht,

Z außerdem für $C_1$-$C_6$-Alkyl steht,

$A^6$ für CH oder N steht,

$R^{23}$ für Wasserstoff, Chlor, durch gegebenenfalls einfach oder zweifach, gleich oder ver- schieden durch Chlor oder Di($C_1$-$C_3$-alkyl)aminocarbonyl substituiertes Phenyl steht,

$R^{23}$ außerdem für Cyano oder $C_1$-$C_6$-Alkyl steht,

$R^{24}$ für Wasserstoff oder Chlor steht,

$R^{25}$ für Wasserstoff, Chlor, Hydroxy, Methyl oder Trifluormethyl steht,

$R^{25}$ außerdem für Di($C_1$-$C_3$-alkyl)aminocarbonyl steht,

$R^{23}$ und $R^{24}$ außerdem gemeinsam für *-$CH(CH_3)$-$CH_2$-$C(CH_3)_2$- oder *-$CH(CH_3)$-O-$C(CH_3)_2$- steht, wobei die mit * markierte Bindung mit $R^{23}$ verknüpft ist;

Gruppe (7) Dithiocarbamate ausgewählt aus

(7-1) Mancozeb

(7-2) Maneb

(7-3) Metiram

(7-4) Propineb

(7-5) Thiram

(7-6) Zineb

(7-7) Ziram

Gruppe (8) Acylalanine der allgemeinen Formel (VI)

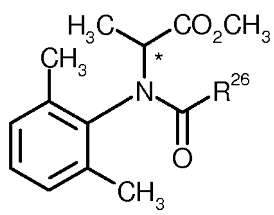

(VI)

in welcher

* ein Kohlenstoffatom in der R- oder der S-Konfiguration, bevorzugt in der S- Konfiguration, kennzeichnet,
$R^{26}$ für Benzyl, Furyl oder Methoxymethyl steht;

Gruppe (9): Anilino-pyrimidine der allgemeinen Formel (VII)

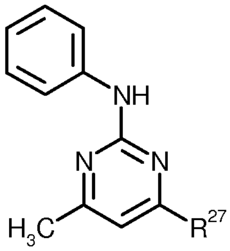

(VII)

in welcher

$R^{27}$ für Methyl, Cyclopropyl oder 1-Propinyl steht;

Gruppe (10): Benzimidazole der allgemeinen Formel (VIII)

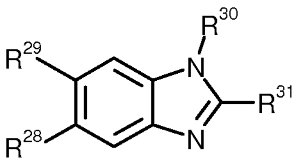

(VIII)

in welcher

$R^{28}$ und $R^{29}$ jeweils für Wasserstoff oder zusammen für -O-$CF_2$-O- stehen,

$R^{30}$ für Wasserstoff, $C_1$-$C_4$-Alkylaminocarbonyl oder für 3,5-Dimethylisoxazol-4-ylsulfo- nyl steht,

$R^{31}$ für Chlor, Methoxycarbonylamino, Chlorphenyl, Furyl oder Thiazolyl steht;

Gruppe (11): Carbamate der allgemeinen Formel (IX)

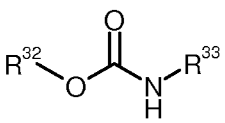

(IX)

in welcher

$R^{32}$ für n- oder iso-Propyl steht,

$R^{33}$ für Di($C_1$-$C_2$-alkyl)amino-$C_2$-$C_4$-alkyl oder Diethoxyphenyl steht,

wobei auch Salz dieser Verbindungen eingeschlossen sind;

Gruppe (12): Dicarboximide ausgewählt aus

(12-1) Captafol

(12-2) Captan

(12-3) Folpet

(12-4) Iprodione

(12-5) Procymidone

(12-6) Vinclozolin

Gruppe (13): Guanidine ausgewählt aus

(13-1) Dodine

(13-2) Guazatine

(13-3) Iminoctadine triacetate

(13-4) Iminoctadine tris(albesilate)

Gruppe (14): Imidazole ausgewählt aus

(14-1) Cyazofamid

(14-2) Prochloraz

(14-3) Triazoxide

(14-4) Pefurazoate

Gruppe (15): Morpholine der allgemeinen Formel (X)

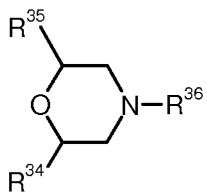

(X)

in welcher

$R^{34}$ und $R^{35}$ unabhängig voneinander für Wasserstoff oder Methyl stehen,

$R^{36}$ für $C_1$-$C_{14}$-Alkyl (bevorzugt $C_{12}$-$C_{14}$-Alkyl), $C_5$-$C_{12}$-Cycloalkyl (bevorzugt $C_{10}$-$C_{12}$- Cycloalkyl), Phenyl-$C_1$-$C_4$-alkyl, welches im Phenylteil durch Halogen oder $C_1$-$C_4$- Alkyl substituiert sein kann, oder für Acrylyl, welches durch Chlorphenyl und Dimethoxyphenyl substituiert ist, steht;

Gruppe (16): Pyrrole der allgemeinen Formel (XI)

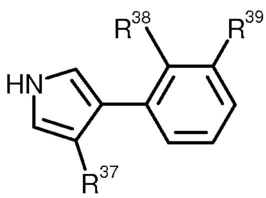

(XI)

in welcher

$R^{37}$ für Chlor oder Cyano steht,

$R^{38}$ für Chlor oder Nitro steht,

$R^{39}$ für Chlor steht

$R^{38}$ und $R^{39}$ außerdem gemeinsam für $-O-CF_2-O-$ stehen;

Gruppe (17): Phosphonate ausgewählt aus

(17-1) Fosetyl-Al

(17-2) Phosphonsäure;

Gruppe (18): Phenylethanamide der allgemeinen Formel (XII)

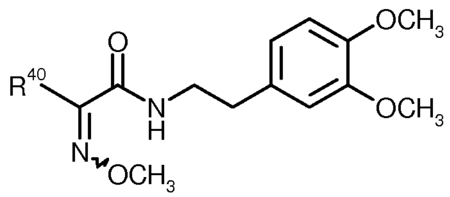

(XII)

in welcher

$R^{40}$ für unsubstituiertes oder durch Fluor, Chlor, Brom, Methyl oder Ethyl substituiertes Phenyl, 2-Naphthyl, 1,2,3,4-Tetrahydronaphthyl oder Indanyl steht;

Gruppe (19): Fungizide ausgewählt aus

(19-1) Acibenzolar-S-methyl
(19-2) Chlorothalonil
(19-3) Cymoxanil
(19-4) Edifenphos
(19-5) Famoxadone
(19-6) Fluazinam
(19-7) Kupferoxychlorid
(19-8) Kupferhydroxid
(19-9) Oxadixyl
(19-10) Spiroxamine
(19-11) Dithianon
(19-12) Metrafenone
(19-13) Fenamidone
(19-14) 2,3-Dibutyl-6-chlor-thieno[2,3-d]pyrimidin-4(3H)on
(19-15) Probenazole
(19-16) Isoprothiolane
(19-17) Kasugamycin
(19-18) Phthalide
(19-19) Ferimzone
(19-20) Tricyclazole
(19-21) N-({4-[(Cyclopropylamino)carbonyl]phenyl}sulfonyl)-2-methoxybenzamid
(19-22) 2-(4-Chlorphenyl)-N-{2-[3-methoxy-4-(prop-2-in-1-yloxy)phenyl]ethyl}-2-(prop-2-in-1-yloxy)acetamid

Gruppe (20): (Thio)Harnstoff-Derivate ausgewählt aus

(20-1) Pencycuron

(20-2) Thiophanate-methyl

(20-3) Thiophanate-ethyl

Gruppe (21): Amide der allgemeinen Formel (XIII)

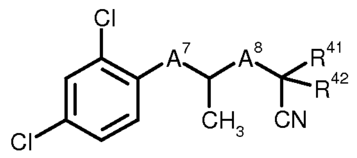

(XIII)

in welcher

$A^7$ für eine direkte Bindung oder -O- steht,

$A^8$ für -C(=O)NH- oder -NHC(=O)- steht,

$R^{41}$ für Wasserstoff oder $C_1$-$C_4$-Alkyl steht,

$R^{42}$ für $C_1$-$C_6$-Alkyl steht;

Gruppe (22): Triazolopyrimidine der allgemeinen Formel (XIV)

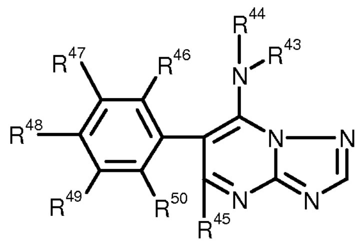

(XIV)

in welcher

$R^{43}$ für $C_1$-$C_6$-Alkyl oder $C_2$-$C_6$-Alkenyl steht,

$R^{44}$ für $C_1$-$C_6$-Alkyl steht,

$R^{43}$ und $R^{44}$ außerdem gemeinsam für $C_4$-$C_5$-Alkandiyl (Alkylen) stehen, welches einfach oder zweifach durch $C_1$-$C_6$-Alkyl substituiert ist,

$R^{45}$ für Brom oder Chlor steht,

$R^{46}$ und $R^{50}$ unabhängig voneinander für Wasserstoff, Fluor, Chlor oder Methyl stehen,

$R^{47}$ und $R^{49}$ unabhängig voneinander für Wasserstoff oder Fluor stehen,

$R^{48}$ für Wasserstoff, Fluor oder Methyl steht,

Gruppe (23): Iodochromone der allgemeinen Formel (XV)

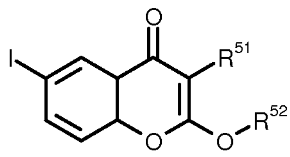

(XV)

in welcher

$R^{51}$ für $C_1$-$C_6$-Alkyl steht,

$R^{52}$ für $C_1$-$C_6$-Alkyl, $C_2$-$C_6$-Alkenyl oder $C_2$-$C_6$-Alkinyl steht.

[2] Wirkstoffkombinationen wie in Absatz [1] beschrieben enthaltend ein Carboxamid der allgemeinen Formel (I) gemäß Absatz [1] (Gruppe 1), in welcher

$R^1$ für Wasserstoff oder Fluor steht,

$R^2$ für Fluor, Chlor, Brom, Iod, Methyl, Trifluormethyl, Trifluormethoxy oder für -C($R^4$)=N-O$R^5$ steht,

$R^3$ für Wasserstoff, Fluor, Chlor, Brom, Methyl oder Trifluormethyl steht,

$R^4$ für Wasserstoff oder Methyl steht,

$R^5$ für $C_1$-$C_5$-Alkyl steht,

A für einen der folgenden Reste A1 bis A7 steht:

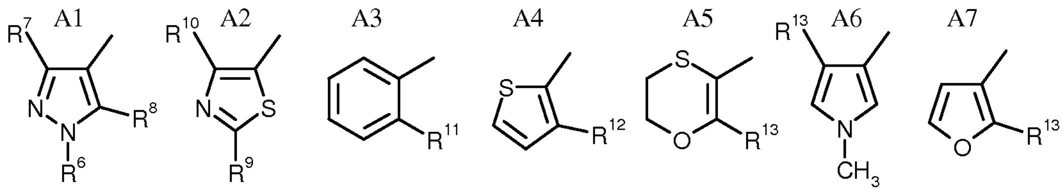

A1 A2 A3 A4 A5 A6 A7

$R^6$ für Methyl steht,

$R^7$ für Iod, Methyl, Difluormethyl oder Trifluormethyl steht,

$R^8$ für Wasserstoff, Fluor, Chlor oder Methyl steht,

$R^9$ für Wasserstoff, Chlor, Methyl, Amino oder Dimethylamino steht,

$R^{10}$ für Methyl, Difluormethyl oder Trifluormethyl steht,

$R^{11}$ für Chlor, Brom, Iod, Methyl, Difluormethyl oder Trifluormethyl steht,

$R^{12}$ für Brom oder Methyl steht,

$R^{13}$ für Methyl oder Trifluormethyl steht.

[3] Wirkstoffkombinationen wie in Absatz [1] beschrieben, wobei die Wirkstoffe der Gruppen (2) bis (23) aus der folgenden Liste ausgewählt sind:

(2-1) Azoxystrobin

(2-2) Fluoxastrobin

(2-3) (2*E*)-2-(2-{[6-(3-Chlor-2-methylphenoxy)-5-fluor-4-pyrimidinyl]oxy}phenyl)-2-(methoxyimino)-*N*-methylethanamid

(2-4) Trifloxystrobin

(2-5) (2*E*)-2-(Methoxyimino)-*N*-methyl-2-(2-{[({(1*E*)-1[3-(trifluormethyl)phenyl]ethyliden}amino)oxy]methyl}phenyl)ethanamid

(2-6) (2*E*)-2-(Methoxyimino)-*N*-methyl-2-{2-[(*E*)-({1-[3-(trifluormethyl)phenyl]-ethoxy}imino)methyl]phenyl}ethanamid

(2-7) Orysastrobin

(2-8) 5-Methoxy-2-methyl-4-(2-{[({(1*E*)-1-[3-(trifluormethyl)phenyl]ethyliden}amino)-oxy]methyl}phenyl)-2,4-dihydro-3*H*-1,2,4-triazol-3-on

(2-9) Kresoxim-methyl

(2-10) Dimoxystrobin

(2-11) Picoxystrobin

(2-12) Pyraclostrobin

(2-13) Metominostrobin

(3-1) Azaconazole

(3-2) Etaconazole

(3-3) Propiconazole

(3-4) Difenoconazole

(3-5) Bromuconazole

(3-6) Cyproconazole

(3-7) Hexaconazole

(3-8) Penconazole

(3-9) Myclobutanil

(3-10) Tetraconazole

(3-11) Flutriafol

(3-12) Epoxiconazole

(3-13) Flusilazole

(3-14) Simeconazole

(3-15) Prothioconazole

(3-16) Fenbuconazole

(3-17) Tebuconazole

(3-18) Ipconazole

(3-19) Metconazole

(3-20) Triticonazole

(3-21) Bitertanol

(3-22) Triadimenol

(3-23) Triadimefon

(3-24) Fluquinconazole

(3-25) Quinconazole

(4-1) Dichlofluanid

(4-2) Tolylfluanid

(5-1) Iprovalicarb

(5-3) Benthiavalicarb

(6-1) 2-Chloro-N-(1,1,3-trimethyl-indan-4-yl)-nicotinamid

(6-2) Boscalid

(6-3) Furametpyr

(6-4) 1-Methyl-3-trifluormethyl-1H-pyrazol-4-carbonsäure-(3-p-tolyl-thiophen-2-yl)-amid

(6-5) Ethaboxam

(6-6) Fenhexamid

(6-7) Carpropamid

(6-8) 2-Chlor-4-(2-fluor-2-methyl-propionylamino)-N,N-dimethyl-benzamid

(6-9) Picobenzamid

(6-10) Zoxamide

(6-11) 3,4-Dichlor-N-(2-cyanophenyl)isothiazol-5-carboxamid

(6-12) Carboxin

(6-13) Tiadinil

(6-14) Penthiopyrad

(6-15) Silthiofam

(6-16) *N*-[2-(1,3-Dimethylbutyl)phenyl]-1-methyl-4-(trifluormethyl)-1*H*-pyrrol-3-carboxamid

(7-1) Mancozeb

(7-2) Maneb

(7-3) Metiram

(7-4) Propineb

(7-5) Thiram

(7-6) Zineb

(7-7) Ziram

(8-1) Benalaxyl

(8-2) Furalaxyl

(8-3) Metalaxyl

(8-4) Metalaxyl-M

(8-5) Benalaxyl-M

(9-1) Cyprodinil

(9-2) Mepanipyrim

(9-3) Pyrimethanil

(10-1) 6-Chlor-5-[(3,5-dimethylisoxazol-4-yl)sulfonyl]-2,2-difluor-5H-[1,3]dioxolo[4,5-f]-benzimidazol

(10-2) Benomyl

(10-3) Carbendazim

(10-4) Chlorfenazole

(10-5) Fuberidazole

(10-6) Thiabendazole

(11-1) Diethofencarb

(11-2) Propamocarb

(11-3) Propamocarb-hydrochloride

(11-4) Propamocarb-Fosetyl

(12-1) Captafol

(12-2) Captan

(12-3) Folpet

(12-4) Iprodione

(12-5) Procymidone

(12-6) Vinclozolin

(13-1) Dodine

(13-2) Guazatine

(13-3) Iminoctadine triacetate

(14-1) Cyazofamid

(14-2) Prochloraz

(14-3) Triazoxide

(14-4) Pefurazoate

(15-1) Aldimorph

(15-2) Tridemorph

(15-3) Dodemorph

(15-4) Fenpropimorph

(15-5) Dimethomorph

(16-1) Fenpiclonil

(16-2) Fludioxonil

(16-3) Pyrrolnitrine

(17-1) Fosetyl-Al

(17-2) Phosphonic acid

(18-1) 2-(2,3-Dihydro-1H-inden-5-yl)-N-[2-(3,4-dimethoxyphenyl)ethyl]-2-(methoxyimino)acetamid

(18-2) N-[2-(3,4-Dimethoxyphenyl)ethyl]-2-(methoxyimino)-2-(5,6,7,8-tetrahydronaphthalen-2-yl)acetamid

(18-3) 2-(4-Chlorphenyl)-N-[2-(3,4-dimethoxyphenyl)ethyl]-2-(methoxyimino)acetamid

(18-4) 2-(4-Bromphenyl)-N-[2-(3,4-dimethoxyphenyl)ethyl]-2-(methoxyimino)acetamid

(18-5) 2-(4-Methylphenyl)-N-[2-(3,4-dimethoxyphenyl)ethyl]-2-(methoxyimino)acetamid

(18-6) 2-(4-Ethylphenyl)-N-[2-(3,4-dimethoxyphenyl)ethyl]-2-(methoxyimino)acetamid

(19-1) Acibenzolar-S-methyl

(19-2) Chlorothalonil

(19-3) Cymoxanil

(19-4) Edifenphos

(19-5) Famoxadone

(19-6) Fluazinam

(19-7) Kupferoxychlorid

(19-9) Oxadixyl

(19-10) Spiroxamine

(19-11) Dithianon

(19-12) Metrafenone

(19-13) Fenamidone

(19-14) 2,3-Dibutyl-6-chlor-thieno[2,3-d]pyrimidin-4(3H)on

(19-15) Probenazole

(19-16) Isoprothiolane

(19-17) Kasugamycin

(19-18) Phthalide

(19-19) Ferimzone

(19-20) Tricyclazole

(19-21) N-({4-[(Cyclopropylamino)carbonyl]phenyl}sulfonyl)-2-methoxybenzamid

(19-22) 2-(4-Chlorphenyl)-N-{2-[3-methoxy-4-(prop-2-in-1-yloxy)phenyl]ethyl}-2-(prop-2-in-1-yloxy)acetamid

(20-1) Pencycuron

(20-2) Thiophanate-methyl

(20-3) Thiophanate-ethyl

(21-1) Fenoxanil

(21-2) Diclocymet

(22-1) 5-Chlor-*N*-[*(1S)*-2,2,2-trifluor-1-methylethyl]-6-(2,4,6-trifluorphenyl)[1,2,4]triazolo-[1,5-a]pyrimidin-7-amin

(22-2) 5-Chlor-*N*-[*(1R)*-1,2-dimethylpropyl]-6-(2,4,6-trifluorphenyl)[1,2,4]triazolo[1,5-a]-pyrimidin-7-amin

(22-3) 5-Chlor-6-(2-chlor-6-fluorphenyl)-7-(4-methylpiperidin-1-yl)[1,2,4]triazolo[1,5-a]-pyrimidin

(22-4) 5-Chlor-6-(2,4,6-trifluorphenyl)-7-(4-methylpiperidin-1-yl)[1,2,4]triazolo[1,5-a]pyrimidin

(23-1) 2-Butoxy-6-iod-3-propyl-benzopyran-4-on

(23-2) 2-Ethoxy-6-iod-3-propyl-benzopyran-4-on

(23-3) 6-Iod-2-propoxy-3-propyl-benzopyran-4-on

(23-4) 2-But-2-inyloxy-6-iod-3-propyl-benzopyran-4-on

(23-5) 6-Iod-2-(1-methyl-butoxy)-3-propyl-benzopyran-4-on

(23-6) 2-But-3-enyloxy-6-iod-benzopyran-4-on

(23-7) 3-Butyl-6-iod-2-isopropoxy-benzopyran-4-on

[4] Wirkstoffkombinationen wie in Absatz [1] beschrieben, enthaltend das Carboxamid (1-1) *N*-(3',4'-Dichlor-5-fluor-1,1'-biphenyl-2-yl)-3-(difluormethyl)-1-methyl-1*H*-pyrazol-4-carboxamid (Gruppe 1) und mindestens einen Wirkstoff, der aus den folgenden Gruppen (2) bis (23) gemäß Absatz [1] ausgewählt ist.

[5] Wirkstoffkombinationen wie in Absatz [1] beschrieben, enthaltend das Carboxamid (1-1) *N*-(3',4'-Dichlor-5-fluor-1,1'-biphenyl-2-yl)-3-(difluormethyl)-1-methyl-1*H*-pyrazol-4-carboxamid (Gruppe 1) und mindestens einen Wirkstoff, der aus den folgenden Gruppen (2) bis (23) gemäß Absatz [3] ausgewählt ist.

[6] Wirkstoffkombinationen wie in Absatz [1] beschrieben, enthaltend das Carboxamid (1-7) *N*-(4'-Brom-1,1'-biphenyl-2-yl)-4-(difluormethyl)-2-methyl-1,3-thiazol-5-carboxamid (Gruppe 1) und mindestens einen Wirkstoff, der aus den folgenden Gruppen (2) bis (23) gemäß Absatz [1] ausgewählt ist.

[7] Wirkstoffkombinationen wie in Absatz [1] beschrieben, enthaltend das Carboxamid (1-7) *N*-(4'-Brom-1,1'-biphenyl-2-yl)-4-(difluormethyl)-2-methyl-1,3-thiazol-5-carboxamid (Gruppe 1) und mindestens einen Wirkstoff, der aus den folgenden Gruppen (2) bis (23) gemäß Absatz [3] ausgewählt ist.

[8] Wirkstoffkombinationen wie in Absatz [1] beschrieben, enthaltend das Carboxamid (1-8) 4-(Difluormethyl)-2-methyl-N-[4'-(trifluormethyl)-1,1'-biphenyl-2-yl] -1,3-thiazol-5-carboxamid (Gruppe 1) und mindestens einen Wirkstoff, der aus den folgenden Gruppen (2) bis (23) gemäß Absatz [1] ausgewählt ist.

[9] Wirkstoffkombinationen wie in Absatz [1] beschrieben, enthaltend das Carboxamid (1-8) 4-(Difluormethyl)-2-methyl-*N*-[4'-(trifluormethyl)-1,1'-biphenyl-2-yl]-1,3-thiazol-5-carboxamid (Gruppe 1) und mindestens einen Wirkstoff, der aus den folgenden Gruppen (2) bis (23) gemäß Absatz [3] ausgewählt ist.

[10] Wirkstoffkombinationen wie in Absatz [1] beschrieben, enthaltend das Carboxamid (1-9) *N*-(4'-Chlor-3'-fluor-1,1'-biphenyl-2-yl)-4-(difluormethyl)-2-methyl-1,3-thiazol-5-carboxamid (Gruppe 1) und mindestens einen Wirkstoff, der aus den folgenden Gruppen (2) bis (23) gemäß Anspruch 1 ausgewählt ist.

[11] Wirkstoffkombinationen wie in Absatz [1] beschrieben, enthaltend das Carboxamid (1-9) *N*-(4'-Chlor-3'-fluor-1,1'-biphenyl-2-yl)-4-(difluormethyl)-2-methyl-1,3-thiazol-5-carboxamid (Gruppe 1) und mindestens einen Wirkstoff, der aus den folgenden Gruppen (2) bis (23) gemäß Absatz [3] ausgewählt ist.

[12] Verwendung von Wirkstoffkombinationen wie in Absatz [1] beschrieben, zur Bekämpfung von unerwünschten phytopathogenen Pilzen.

[13] Verfahren zum Bekämpfen von unerwünschten phytopathogenen Pilzen, **dadurch gekennzeichnet, dass** man Wirkstoffkombinationen gemäß Absatz [1] auf die unerwünschten phytopathogenen Pilze und/oder deren Lebensraum ausbringt.

[14] Verfahren zum Herstellen von fungiziden Mitteln, **dadurch gekennzeichnet, dass** man Wirkstoffkombinationen gemäß Absatz [1] mit Streckmitteln und/ oder oberflächenaktiven Stoffen vermischt.

## Patentansprüche

**1.** Synergistische fungizide Wirkstoffkombinationen enthaltend das Carboxamid (1-1) *N*-(3',4'-Dichlor-5-fluor-1,1'-biphenyl-2-yl)-3-(difluormethyl)-1-methyl-1H-pyrazol-4-carboxamid (Gruppe 1) und Fenpropimorph (15-4).

2. Verwendung von Wirkstoffkombinationen gemäß Anspruch 1 zum Bekämpfung von unerwünschten phytopathogenen Pilzen.

3. Verfahren zum Bekämpfen von unerwünschten phytopathogenen Pilzen, **dadurch gekennzeichnet, dass** man Wirkstoffkombinationen gemäß Anspruch 1 auf die unerwünschten phytopathogenen Pilze und/oder deren Lebensraum ausbringt.

4. Verfahren zum Herstellen von fungiziden Mitteln, **dadurch gekennzeichnet, dass** man Wirkstoffkombinationen gemäß Anspruch 1 mit Streckmitteln und/ oder oberflächenaktiven Stoffen vermischt.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10215292 A
- WO 0208197 A
- WO 0014701 A
- EP 0040345 A
- DE 2201063 A
- DE 2324010 A
- EP 0382375 A
- EP 0515901 A
- WO 9706171 A
- DE 19646407 A1
- EP 712396 B
- WO 03070705 A
- WO 03066609 A
- WO 03066610 A
- DE 19602095 A
- DE 19646407 A
- EP 0712396 B
- EP 0460575 A
- EP 0569384 A
- EP 0596254 A
- DE 19539324 A
- WO 9823155 A
- EP 0253213 A
- EP 0398692 A
- EP 0278595 A
- DE 4423612 A
- DE 2551560 A
- EP 0112284 A
- EP 0258161 A
- DE 3406993 A
- DE 3042303 A
- DE 2735872 A
- EP 0145294 A
- EP 0234242 A
- EP 0015756 A
- EP 0196038 A
- EP 0068813 A
- EP 0537957 A
- WO 9616048 A
- DE 3721786 A
- EP 0329397 A
- EP 0378953 A
- EP 0183458 A
- DE 1193498 A
- DE 4026966 A
- WO 9604252 A
- EP 0256503 A
- DE 19531813 A
- EP 0315502 A
- EP 0737682 A
- EP 0639574 A
- EP 0339418 A
- EP 0341475 A
- EP 0600629 A
- WO 9942447 A
- EP 0604019 A
- WO 9924413 A
- US 3249499 A
- US 6616054 B
- WO 9618631 A
- WO 0238542 A
- DE 1234704 A
- US 2504404 A
- DE 1076434 A
- GB 935981 A
- US 1972961 A
- DE 1081446 A
- US 2588428 A
- DE 2903612 A
- DE 2513732 A
- DE 2515091 A
- WO 9601559 A
- EP 0310550 A
- EP 0270111 A
- DD 151404
- US 3631176 A
- US 3010968 A
- DE 1209799 A
- US 3206468 A
- EP 0078663 A
- US 3513241 A
- US 3178447 A
- US 2553770 A
- DE 2149923 A
- DE 2012656 A
- DE 2207576 A
- GB 1103989 A
- GB 1114155 A
- EP 0155509 A
- EP 0298196 A
- DE 2429523 A
- DE 2802488 A
- EP 0248086 A
- DD 140041
- GB 988630 A
- DE 2543279 A
- DE 2656747 A
- EP 0219756 A

- EP 0236272 A
- EP 0206999 A
- JP 6525876 B
- DE 2456627 A
- WO 9623793 A
- EP 0313512 A
- US 3290353 A
- DE 2312956 A
- DE 1493736 A
- EP 0393911 A
- EP 0031257 A
- DE 3030026 A
- DE 3735555 A
- JP 4429464 A
- EP 0897904 A
- EP 0629616 A
- WO 9914202 A
- US 3629428 A
- US 3856814 A
- GB 1094567 A
- JP 57055844 A
- EP 0019450 A
- DE 2250077 A
- WO 0187822 A
- DE 2732257 A
- DE 1806123 A
- EP 0262393 A
- JP 7206608 A
- US 5986135 A
- WO 0238565 A
- US 5593996 A
- DE 10124208 A
- WO 03014103 A

**In der Beschreibung aufgeführte Nicht-Patentliteratur**

- **S.R. Colby.** Calculating Synergistic and Antagonistic Responses of Herbicide Combinations. *Weeds,* 1967, vol. 15, 20-22